(19) **Europäisches Patentamt / European Patent Office / Office européen des brevets**

(11) **EP 1 862 797 A1**

(12) **EUROPEAN PATENT APPLICATION**
published in accordance with Art. 158(3) EPC

(43) Date of publication:
**05.12.2007 Bulletin 2007/49**

(51) Int Cl.:
***G01N 33/48*** (2006.01)

(21) Application number: **06715368.4**

(22) Date of filing: **07.03.2006**

(86) International application number:
**PCT/JP2006/304398**

(87) International publication number:
**WO 2006/098192 (21.09.2006 Gazette 2006/38)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **16.03.2005 JP 2005076064**

(71) Applicant: **Ajinomoto Co., Inc.**
**Tokyo 104-8315 (JP)**

(72) Inventors:
• **NOGUCHI, Yasushi,**
**c/o Ajinomoto Co.,Inc.**
**Kawasaki-shi, Kanagawa, 2108681 (JP)**

• **SAKAI, Ryosei,**
**c/o Ajinomoto Co.,Inc.**
**Kawasaki-shi, Kanagawa, 2108681 (JP)**
• **SUGIMOTO, Tetsuya,**
**c/o Ajinomoto Co., Inc.**
**Kawasaki-shi, Kanagawa, 2108681 (JP)**

(74) Representative: **Johnson, Richard Alan et al**
**Mewburn Ellis LLP**
**York House**
**23 Kingsway**
**London WC2B 6HP (GB)**

(54) **BIOCONDITION EVALUATING DEVICE, BIOCONDITION EVALUATING METHOD, BIOCONDITION EVALUATING SYSTEM, BIOCONDITION EVALUATING PROGRAM, EVALUATION FUNCTION GENERATING DEVICE, EVALUATION FUNCTION GENERATING METHOD, EVALUATION FUNCTION GENERATING PROGRAM, AND RECORDING MEDIUM**

(57)     Provided are a biological state-evaluating apparatus allowing accurate evaluation of the biological state of an evaluation subject by using a verified evaluation function, a biological state-evaluating method, a biological state-evaluating system, a biological state-evaluating program, a recording medium, and the like. The biological state-evaluating system according to the present invention includes a biological state-evaluating apparatus 100 that evaluates biological state and information communication terminal apparatuses 200 that provide the metabolite concentration data to be evaluated, communicatively connected thereto via a network 300. The biological state-evaluating apparatus 100 generates an evaluation function having the metabolite concentration as the variable, based on previously-obtained biological state information including metabolite concentration data concerning metabolite concentration and biological state indicator data concerning the indicator showing the biological state, and evaluates the biological state to be evaluated, based on the generated evaluation function and the previously acquired metabolite concentration data to be evaluated.

FIG.1

**Description**

TECHNICAL FIELD

The present invention relates to a biological state-evaluating apparatus, a biological state-evaluating method, a biological state-evaluating system, a biological state-evaluating program and a recording medium that generate an evaluation function having a metabolite concentration as the variable, based on biological state information including metabolite concentration data concerning metabolite concentration and biological state indicator data concerning the indicator showing the biological state, and evaluates the biological state to be evaluated, based on the generated evaluation function and the metabolite concentration data to be evaluated.

The present invention also relates to a biological state-evaluating apparatus that evaluates the biological state to be evaluated, based on a previously stored evaluation function having a metabolite concentration as the variable and metabolite concentration data to be evaluated.

The present invention also relates to an evaluation function-generating apparatus, an evaluation function-generating method, an evaluation function-generating program and recording medium that generate an evaluation function having a metabolite concentration as the variable, based on biological state information including metabolite concentration data concerning metabolite concentration and biological state indicator data concerning the indicator showing the biological state.

In the present specification, the "biological state" is a concept including a healthy state (healthiness) and various disease states. The "biological state indicator data" is a concept including measured values of an individual and diagnosis result data concerning the biological state of the subject. The "data" and the "information" are concepts including those of digitalized ones and conceptual ones (e.g., healthy or with disease, severity, and kind).

In the present specification, the "candidate evaluation function" and "evaluation function" are functions having a metabolite concentration as the variable, and specifies the difference in biological state quantitatively (e.g., "healthiness and disease states", "a plurality of different disease states", and " progress of a particular disease").

BACKGROUND ART

Along with progress in diagnostic technologies by using bioinformatics, in addition to the unit such as single-nucleotide polymorphism analysis and gene expression profiling, protein expression profiling and more recently metabolite profiling have been used more frequently gradually. Among them, the importance of the metabolite profiling has been more emphasized, and its practical realization is longed for.

In the metabolite profiling, a metabolite concentration in the body, which reflects a dynamic equilibrium controlled by various metabolic factors, is considered to be a useful data source that indicates the metabolic activity collectively in a particular biological state. Thus, change in biological state may lead to significant change in a metabolite concentration of the body, and thus, a metabolite concentration in the body is considered to be involved in defining various biological states. Accordingly, diagnosis by using metabolite profiling, differently from genetic diagnosis, advantageously reflects the biological state including not only metabolic factors but also environmental factors during the test, and thus, the potential of the metabolite profiling is extremely important. In particular, the amino acid concentration in the biological sample (including blood) of a patient with various disease, such as liver disease, abnormality in various genetic metabolism, diabetes, hypertension, cancer, muscular dysbolism, renal disease, various nervous disease, or hormone abnormality, is reported to show a characteristic fluctuation, compared to that of a healthy subject, and thus, profiling of the concentration of various amino acids, which are metabolites in the body, is a favorable example showing significance of the metabolite profiling. Development of the methods of quantitatively determining the concentration of metabolites in biological sample is under rapid progress, and amino acid analyzers, which determine the concentration of various amino acids (clinically 21 to 41 kinds of amino acids) in a biological sample such as blood sample, are already established clinically.

However, because there may be some fluctuation in concentration of a plurality of metabolites to be measured along with change in biological state in metabolite profiling, it is important to evaluate (predict) the biological state, based on multivariate data concerning the metabolite concentration, in diagnosis by using metabolite profiling and it is necessary to generate a mathematical model properly reflecting the difference in biological state (evaluation function described above). If it is possible to generate a mathematical model showing the relationship between the fluctuation in the metabolite concentration data collected from known subjects and the corresponding biological state, it becomes possible to evaluate a biological state, theoretically based on the mathematical model, from the metabolite concentration data concerning biological state of an unknown subject.

Methods so far known for evaluation and prediction of biological state include, for example, Fischer ratio and the methods described in Patent Documents 1 and 2. The Fischer ratio is an indicator having the concentration of aromatic amino acids increasing during liver cirrhosis as the denominator and the concentration of branched-chain amino acids decreasing as the numerator: "(Ile+Leu+Val) ÷(Phe+Tyr) ", and it is possible to predict the condition of liver cirrhosis, based on the amino acid concentration data of the subject. Alternatively, Patent Document 1 discloses a technique of predicting biological state by using a prediction indicator (corresponding to an evaluation function described above) generated by neural network (nonlinear analysis). Specifically, when the disease state of patients with heart disease or dental amalgam syndrome and healthy people and the blood analysis data of the patients and the healthy people are input into computer, the computer generates a prediction indicator, based on the input data, by neural network, optimizes the prediction indicator for more differentiation of the data by training the neural network, and predicts the biological state by using the optimized prediction indicator. It was thus possible to predict the condition, for example, of heart disease or dental amalgam syndrome, based on the blood analysis data of subjects.

Alternatively, Patent Document 2 discloses a technique of simulating the biological state of an individual, based on indicator data concerning biological state, blood metabolite concentration data (e.g., amino acid concentration data), and blood metabolite concentration data of the individual to be simulated (e.g., amino acid concentration data). Specifically, the biological state of an individual to be simulated is simulated, by generating a correlation formula (corresponding to an evaluation function above) showing the relationship between indicator data concerning biological state obtained from individuals and blood concentration data of the metabolites obtained from respective individuals and substituting the measured blood concentration data of each metabolite of the individual to be simulated in the generated correlation formula. It is thus possible to simulate, for example, health state, progress of disease, treatment state of disease, future risk of disease, medicinal effectiveness, and side-effect of medicine effectively, based on the blood metabolite concentration of an individual.

Patent Document 1: U.S. Patent No. 5687716
Patent Document 2: WO 2004/052191

DISCLOSURE OF THE INVENTION

PROBLEMS TO BE SOLVED BY THE INVENTION

However, because the validity of the prediction indicator or the correlation formula (corresponding to the evaluation function described above) in evaluating biological state was not verified in the traditional methods, the methods had a problem that they could not always allow evaluation of the biological state of evaluation subject with sufficient accuracy.

An object of the present invention, which was made to solve the problems above, is to provide a biological state-evaluating apparatus, a biological state-evaluating method, a biological state-evaluating system, and a biological state-evaluating program and recording medium allowing accurate evaluation of the biological state of an evaluation subject by using a verified evaluation function. Another object of the present invention is to provide a biological state-evaluating apparatus allowing evaluation of the biological state to be evaluated only by inputting metabolite concentration data to be evaluated.

Yet another object of the present invention is to provide an evaluation function-generating apparatus, an evaluation function-generating method, an evaluation function-generating program and a recording medium that generate an evaluation function optimal for evaluating the biological state of evaluation subject by verification of the evaluation function.

MEANS FOR SOLVING PROBLEMS

To solve the above problems and achieve the above objects, a biological state-evaluating apparatus, a biological state-evaluating method, or a biological state-evaluating program according to one aspect of the present invention includes an evaluation function-generating unit (evaluation function-generating step) that generates an evaluation function having the metabolite concentration as the variable, based on previously-obtained biological state information including metabolite concentration data concerning metabolite concentration and biological state indicator data concerning the indicator showing the biological state, and a biological state-evaluating unit (biological state-evaluating step) that evaluates the biological state to be evaluated based on the evaluation function generated by the evaluation function-generating unit (evaluation function-generating step) and the previously acquired metabolite concentration data to be evaluated, the evaluation function-generating unit (evaluation function-generating step) further including candidate evaluation function-generating unit (candidate evaluation function-generating step) that generates a candidate evaluation function that is a candidate of the evaluation function from the biological state information according to a particular function-generating method, a candidate evaluation function-verifying unit (candidate evaluation function-verifying step) that verifies the candidate evaluation function generated by the candidate evaluation function-generating unit (candidate evaluation function-generating step) according to a particular verification method, and a variable-selecting unit (variable-selecting step) that selects the combination of the metabolite concentration data contained in the biological state information to be used in preparing the candidate evaluation function, by selecting a variable of the candidate evaluation function from the verification results obtained by the candidate evaluation function-verifying unit (candidate evaluation function-verifying step) according to a particular variable selection method, wherein the evaluation function-generating unit generates the evaluation function by selecting a candidate evaluation function to be used as the evaluation function among the candidate evaluation functions based on the verification results obtained by repeated execution of the candidate evaluation function-generating unit (candidate evaluation function-generating step), the candidate evaluation function-verifying unit (candidate evaluation function-verifying step) and the variable-selecting unit (variable-selecting step).

Another aspect of the present invention is the biological state-evaluating apparatus, the biological state-evaluating method, or the biological state-evaluating program, wherein the candidate evaluation functions are generated from the biological state information, by using a plurality of different function-generating methods by the candidate evaluation function-generating unit (candidate evaluation function-generating step).

Still another aspect of the present invention is the biological state-evaluating apparatus, the biological state-evaluating method, or the biological state-evaluating program, wherein the function-generating method is multivariate analysis.

Still another aspect of the present invention is the biological state-evaluating apparatus, the biological state-evaluating method, or the biological state-evaluating program, wherein at least one of the discrimination rate, sensitivity, specificity, and information criterion of the candidate evaluation functions is verified according to at least one of bootstrap method, holdout method, and leave-one-out method, by the candidate evaluation function-verifying unit (candidate evaluation function-verifying step).

Still another aspect of the present invention is the biological state-evaluating apparatus, the biological state-evaluating method, or the biological state-evaluating program, wherein the variable of the candidate evaluation function is selected from the verification results according to at least one of stepwise method, best path method, local search method, and genetic algorithm, by the variable-selecting unit (variable-selecting step).

Still another aspect of the present invention is the biological state-evaluating apparatus, the biological state-evaluating method, or the biological state-evaluating program, wherein the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample.

Still another aspect of the present invention is the biological state-evaluating apparatus, the biological state-evaluating method, or the biological state-evaluating program, wherein the metabolite concentration data to be evaluated are of a patient with ulcerative colitis or Crohn's disease.

The present invention also relates to a biological state-evaluating system, and the biological state-evaluating system according to still another aspect of the present invention includes a biological state-evaluating apparatus that evaluates biological state and information communication terminal apparatuses that provide the metabolite concentration data to be evaluated communicatively connected thereto via a network, the information communication terminal apparatus including a sending unit that sends the metabolite concentration data to the biological state-evaluating apparatus and a receiving unit that receives the evaluation results corresponding to the metabolite concentration data sent from the sending unit from the biological state-evaluating apparatus, the biological state-evaluating apparatus including an evaluation function-generating unit that generates an evaluation function having the metabolite concentration as the variable, based on previously-obtained biological state information including metabolite concentration data concerning metabolite concentration and biological state indicator data concerning the indicator showing the biological state, a biological state-evaluating unit that evaluates the biological state to be evaluated, based on the evaluation function generated by the evaluation function-generating unit and the previously acquired metabolite concentration data to be evaluated, and an evaluation result-sending unit that sends the evaluation results obtained by the biological state-evaluating unit to the information communication terminal apparatus, wherein the evaluation function-generating unit further includes a candidate evaluation function-generating unit that generates a candidate evaluation function that is a candidate of the evaluation function from the biological state information according to a particular function-generating method, a candidate evaluation function-verifying unit that verifies the candidate evaluation function prepared by the candidate evaluation function-generating unit according to a particular verification method, and a variable-selecting unit that selects the combination of the metabolite concentration data contained in the biological state information to be used in preparing the candidate evaluation function by selecting a variable of the candidate evaluation function from the verification results by the candidate evaluation function verification unit according to a particular variable selection method, and the evaluation function-generating unit generates the evaluation function by selecting a candidate evaluation function to be used as the evaluation function among the candidate evaluation functions based on the verification results accumulated by repeated execution of the candidate evaluation function-generating unit, the candidate evaluation function-verifying unit and the variable-selecting unit.

Still another aspect of the present invention is the biological state-evaluating system, wherein the candidate evaluation function-generating unit generates the candidate evaluation functions from the biological state information

by using a plurality of different function-generating methods.

Still another aspect of the present invention is the biological state-evaluating system, wherein the function-generating method is multivariate analysis.

Still another aspect of the present invention is the biological state-evaluating system, wherein the candidate evaluation function-verifying unit verifies at least one of the discrimination rate, sensitivity, specificity, and information criterion of the candidate evaluation functions according to at least one of bootstrap method, holdout method, and leave-one-out method.

Still another aspect of the present invention is the biological state-evaluating system, wherein the variable-selecting unit selects the variable of the candidate evaluation function from the verification results according to at least one of stepwise method, best path method, local search method, and genetic algorithm.

Still another aspect of the present invention is the biological state-evaluating system, wherein the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample.

Still another aspect of the present invention is the biological state-evaluating system, wherein the metabolite concentration data to be evaluated are of a patient with ulcerative colitis or Crohn's disease.

The present invention also relates to a recording medium, and the recording medium according to still another aspect of the present invention carries the biological state-evaluating program described above.

The present invention also relates to a biological state-evaluating apparatus, and the biological state-evaluating apparatus according to still another aspect of the present invention includes an evaluation function-storing unit that stores an evaluation function having the metabolite concentration as the variable and a biological state-evaluating unit that evaluates the biological state to be evaluated, based on the evaluation function stored in the evaluation function-storing unit and the previously acquired metabolite concentration data to be evaluated.

Still another aspect of the present invention is the biological state-evaluating apparatus, wherein the evaluation function-storing unit stores at least one of the evaluation functions represented by the following Formulae 1 to 4; and the biological state-evaluating unit evaluates the biological state, based on at least one of the stored evaluation functions and the metabolite concentration data.

[Formula 1]

$$a_1x_1 + a_2x_2 + \cdots + a_nx_n \quad \cdots (\text{formula1})$$

[Formula 2]

$$\frac{1}{1 + \exp(b_{n+1} + b_1x_1 + b_2x_2 + \cdots + b_nx_n)} \quad \cdots (\text{formula2})$$

[Formula 3]

$$c_1x_1 + c_2x_2 + \cdots + c_nx_n + \Theta(\vec{x}) \quad \cdots (\text{formula3})$$

[Formula 4]

$$\left[ K \mid \left(\bar{x} - \overrightarrow{x_K}\right) X_K \left(\bar{x} - \overrightarrow{x_K}\right)^t \\ = \min\left\{\left(\bar{x} - \overrightarrow{x_1}\right) X_1 \left(\bar{x} - \overrightarrow{x_1}\right)^t, \left(\bar{x} - \overrightarrow{x_2}\right) X_2 \left(\bar{x} - \overrightarrow{x_2}\right)^t, \cdots, \left(\bar{x} - \overrightarrow{x_j}\right) X_j \left(\bar{x} - \overrightarrow{x_j}\right)^t\right\} \right]$$

$\cdots$ (formula4)

[Formula 5]

$$\Theta = \left[\left\{\gamma(\bar{c} \cdot \bar{x}) + c_0\right\}^p, \quad \exp\left(-\gamma \times |\bar{c} - \bar{x}|^2\right), \quad \tanh\left\{\gamma(\bar{c} \cdot \bar{x}) + c_0\right\}\right]$$
$$\bar{x} = \left(x_1, x_2, \cdots, x_n\right) \qquad \bar{c} = \left(c_1, c_2, \cdots, c_n\right) \qquad \gamma, c_0 : \text{cons} \tan t$$

$\cdots$ (formula5)

(in Formula 1, each of $a_1$ to $a_n$ is a real number, satisfying the formula: "$a_1 + a_2 + ... + a_n = 1$"; in Formula 2, each of $b_1$ to $b_{n+1}$ is a real number, satisfying the formula "$|b_i| < 1$" (i=1 to n) ; in Formula 3, each of $c_1$ to $c_n$ is a real number; $\Theta$ is defined by Formula 5; and in Formula 4, j is an integer).

Still another aspect of the present invention is the biological state-evaluating apparatus, wherein the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample.

Still another aspect of the present invention is the biological state-evaluating apparatus, wherein the metabolite concentration data to be evaluated are of a patient with any of ulcerative colitis, Crohn's disease, asthma, or rheumatism.

The present invention also relates to an evaluation function-generating apparatus, an evaluation function-generating method, and an evaluation function-generating program. The evaluation function-generating apparatus, the evaluation function-generating method, or the evaluation function-generating program that makes computer execute an evaluation function-generating method according to still another aspect of the invention, generates an evaluation function having the metabolite concentration as the variable, based on previously-obtained biological state information including metabolite concentration data concerning metabolite concentration and biological state indicator data concerning the indicator showing the biological state. The evaluation function-generating apparatus, the evaluation function-generating method, or the evaluation function-generating program includes a candidate evaluation function-generating unit (candidate evaluation function-generating step) that generates a candidate evaluation function that is a candidate of the evaluation function from the biological state information according to a particular function-generating method, a candidate evaluation function-verifying unit (candidate evaluation function-verifying step) that verifies the candidate evaluation function generated by the candidate evaluation function-generating unit (candidate evaluation function-generating step) according to a particular verification method, and a variable-selecting unit (variable-selecting step) that selects the combination of the metabolite concentration data contained in the biological state information to be used in preparing the candidate evaluation function by selecting a variable of the candidate evaluation function from the verification results by the candidate evaluation function verification unit (candidate evaluation function-verifying step) according to a particular variable selection method, wherein the evaluation function is generated by selecting a candidate evaluation function to be used as the evaluation function among the candidate evaluation functions based on the verification results accumulated by repeated execution of the candidate evaluation function-generating unit (candidate evaluation function-generating step), the candidate evaluation function-verifying unit (candidate evaluation function-verifying step) and the variable-selecting unit(variable-selecting step).

Still another aspect of the present invention is the evaluation function-generating apparatus, the evaluation function-generating method, or the evaluation function-generating program, wherein the candidate evaluation functions are generated from the biological state information, by using a plurality of different function-generating methods by the candidate evaluation function-generating unit (candidate evaluation function-generating step).

Still another aspect of the present invention is the evaluation function-generating apparatus, the evaluation function-generating method, or the evaluation function-generating program, wherein the function-generating method is multivariate analysis.

Still another aspect of the present invention is the evaluation function-generating apparatus, the evaluation function-generating method, or the evaluation function-generating program, wherein at least one of the discrimination rate, sensitivity, specificity, and information criterion of the candidate evaluation functions is verified according to at least one of bootstrap method, holdout method, and leave-one-out method, by the candidate evaluation function-verifying unit (candidate evaluation function-verifying step).

Still another aspect of the present invention is the evaluation function-generating apparatus, the evaluation function-generating method, or the evaluation function-generating program, wherein the variable of the candidate evaluation function is selected from the verification results according to at least one of stepwise method, best path method, local search method, and genetic algorithm, by the variable-selecting unit (variable-selecting step).

Still another aspect of the present invention is the evaluation function-generating apparatus, the evaluation function-generating method, or the evaluation function-generating program, wherein the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample.

The present invention also relates to a recording medium, and the recording medium according to still another aspect of the present invention carries the evaluation function-generating program.

EFFECTS OF THE INVENTION

The biological state-evaluating apparatus, the biological state-evaluating method, and the biological state-evaluating program according to the present invention (1) generate an evaluation function having the metabolite concentration as the variable (evaluation index (the same shall apply hereinafter)), based on previously-obtained biological state information including metabolite concentration data concerning metabolite concentration and biological state indicator data concerning the indicator showing the biological state, and (2) evaluate the biological state to be evaluated, based on the generated evaluation function and the previously acquired metabolite concentration data to be evaluated. In preparation of the evaluation function (1), they (1-1) generate a candidate evaluation function that is a candidate of evaluation function (candidate evaluation index (the same shall apply hereinafter)) from the biological state information according to a particular function-generating method, (1-2) verify the prepared candidate evaluation function according to a particular verification method, (1-3) select the combination of the metabolite concentration data contained in the biological state information to be used in preparing the candidate evaluation function by selecting a variable of the candidate evaluation function from the verification results according to a particular variable selection method, and generate an evaluation function, based on the verification results accumulated by repeated execution of (1-1), (1-2) and (1-3), by selecting a candidate evaluation function to be used as the evaluation function among the candidate evaluation functions. Thus, it is possible advantageously to evaluate the biological state to be evaluated accurately by using a verified evaluation function.

In particular according to the present invention, it is possible to evaluate (monitor) quantitatively the degree (progress of disease) of chronic diseases (such as life-style diseases). Thus, it becomes possible to diagnose various diseases promptly by using the present invention.

According to the present invention, it is also possible to evaluate (monitor) favorable and adverse effects of drug administration quantitatively. Thus, it becomes possible to carry out new drug development efficiently and, as a result, to reduce the development cost, by using the present invention.

According to the present invention, it is also possible to determine whether a patient is with an acute disease (e.g., viral disease or cancer) or whether the patient is healthy or unhealthy. Thus, it is possible to evaluate particular diseases qualitatively.

The biological state-evaluating apparatus, the biological state-evaluating method, and the biological state-evaluating program according to the present invention generate the candidate evaluation functions from the biological state information, by using a plurality of different function-generating methods in combination. Thus, the formats of respective candidate evaluation functions prepared are different from each other, according to the function-generating methods. Advantageously, it is possible to generate an evaluation function appropriate for evaluating the biological state. Although various diagnoses including not only two-group discrimination between healthy and unhealthy, but also other diagnoses from various viewpoints (e.g., multigroup classification such as similar disease identification and progress prediction of progressive disease) are demanded in clinical diagnosis, such an evaluation function has been generated, based on only one predetermined algorithm. However, according to the present invention, because the candidate evaluation functions are generated by using a plurality of different function-generating methods in combination, it is possible to generate an appropriate evaluation function suitable for diagnostic condition finally.

In the biological state-evaluating apparatus, the biological state-evaluating method, and the biological state-evaluating program according to the present invention, the function-generating method is related to multivariate analysis. Advantageously, it is possible to generate a candidate evaluation function, based on an existing function-generating method.

The biological state-evaluating apparatus, the biological state-evaluating method, and the biological state-evaluating program according to the present invention verify at least one of the discrimination rate, sensitivity, specificity, and information criterion of the candidate evaluation functions according to at least one of bootstrap method, holdout method, and leave-one-out method. Advantageously, it is possible to generate a candidate evaluation function higher in predictability or reliability.

The biological state-evaluating apparatus, the biological state-evaluating method, and the biological state-evaluating program according to the present invention select the variable of the candidate evaluation function from the verification results, according to at least one of stepwise method, best path method, local search method, and genetic algorithm. Advantageously, it is possible to select the variable of the candidate evaluation function properly.

In the biological state-evaluating apparatus, the biological state-evaluating method, and the biological state-evaluating program according to the present invention, the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample. Thus, it is possible to generate an evaluation function higher in evaluation accuracy, by using metabolite concentration data concerning metabolite concentration higher in measurement accuracy. For example, it is possible to generate an evaluation function higher in reliability, by using the favorable physical properties of amino acids such as high measurement accuracy and measurement variance smaller than the variance due to individual difference.

In the present specification, the "amino acid analogues" include carnitine, dihydroxyquinoline, quinoline acid, methylimidazole acetic acid, and the like.

The "amino or imino group-containing compounds" in the present specification include creatine, creatinine, amines (such as putrescine, spermidine, and spermine), taurine, hypotaurine, N-acetylglutamic acid, N-acetylaspartylglutamic acid, anserine, carnosine, acetylanserine, acetylcarnosine, purines (such as adenine and guanine xanthine), pyrimidines (such as uracil, orotic acid, and thymine), catecholamines (such as adrenaline, dopamine, and noradrenaline), melanin, and the like.

In the biological state-evaluating apparatus, the biological state-evaluating method, and the biological state-evaluating program according to the present invention, the metabolite concentration data to be evaluated is that of the patients with ulcerative colitis or Crohn's disease. Thus, it is possible to evaluate the disease state accurately, based on the metabolite concentration data that can be obtained from the patients easily, even for diseases giving greater physical and mental burdens to the patients in diagnosis of disease state. For example, in the case of ulcerative colitis normally diagnosed using endoscope, it is possible advantageously to evaluate the ulcerative colitis accurately without an endoscope and consequently to reduce the burden to the patient effectively. Evaluation of disease state of inflammatory bowel diseases (IBDs) was performed by a diagnostic method such as intestinal endoscope or biopsy or with a score such as CDAI (Crohn's disease activeness indicator), IOIBD, or DutchAI. However, the diagnostic method employing an intestinal endoscope or biopsy demanded a professional physician and exerted greater burden on the patient. Alternatively, the score such as CDAI or IOIBD, which includes items subjective to the patient such as the state of stomachache and feces, often could not express the disease state accurately. Evaluation of asthma disease state has been made by combination of oral examination, for example concerning the state of stridor, hematological test, lung function test, and X-ray test. Alternatively, evaluation of rheumatoid disease state has been made by combination of oral examination for example concerning joint edema, rheumatic reaction test, and X-ray test. Unfavorably, these methods contained patient-subjective factors and the disease state did not agree well with the test result. In the present invention, it is possible to evaluate the disease state of IBD, asthma and rheumatism accurately, based on the data concerning metabolite concentration in the body that is extremely objective and can be determined easily.

The biological state-evaluating system according to the present invention includes a biological state-evaluating apparatus which evaluates biological state and information communication terminal apparatuses which provide the metabolite concentration data to be evaluated that are communicatively connected to each other via a network. The information communication terminal apparatus sends metabolite concentration data to the biological state-evaluating apparatus, and receives the evaluation results corresponding to the sent metabolite concentration data from the biological state-evaluating apparatus. The biological state-evaluating apparatus (1) generates an evaluation function having the metabolite concentration as the variable, based on previously-obtained biological state information including metabolite concentration data concerning metabolite concentration and biological state indicator data concerning the indicator showing the biological state, (2) evaluates the biological state to be evaluated, based on the generated evaluation function and the previously acquired metabolite concentration data to be evaluated, and (3) sends the evaluation results to the information communication terminal apparatus. In generating the evaluation function (1), the biological state-evaluating apparatus (1-1) generates a candidate evaluation function that is a candidate of evaluation function from the biological state information according to a particular function-generating method, (1-2) verifies the prepared candidate evaluation function according to a particular verification method verification, (1-3) selects the combination of the metabolite concentration data contained in the biological state information to be used in preparing the candidate evaluation function,

by selecting a variable of the candidate evaluation function from the verification results according to a particular variable selection method, and generates an evaluation function, based on the verification results accumulated by repeated execution of (1-1), (1-2) and (1-3), by selecting a candidate evaluation function to be used as the evaluation function among the candidate evaluation functions. Thus, it is possible advantageously to evaluate the biological state to be evaluated accurately by using a verified evaluation function.

In particular according to the present invention, it is possible to evaluate (monitor) quantitatively the degree (progress of disease) of chronic diseases (such as life-style diseases). Thus, it becomes possible to diagnose various diseases promptly by using the present invention.

According to the present invention, it is also possible to evaluate (monitor) favorable and adverse effects of drug administration quantitative. Thus, it becomes possible to carry out new drug development efficiently and, as a result, to reduce the development cost, by using the present invention.

According to the present invention, it is also possible to determine whether a patient is with an acute disease (e.g., viral disease or cancer) or whether the patient is healthy or unhealthy. Thus, it is possible to evaluate particular diseases qualitatively.

The biological state-evaluating system according to the present invention generates the candidate evaluation functions from the biological state information, by using a plurality of different function-generating methods in combination. Thus, the formats of respective candidate evaluation functions prepared are different from each other, according to the function-generating methods. Advantageously, it is possible to generate an evaluation function appropriate for evaluating the biological state.

Although various diagnoses including not only two-group discrimination between healthy and unhealthy, but also other diagnoses from various viewpoints (e.g., multigroup classification such as similar disease identification and progress prediction of progressive disease) are demanded in clinical diagnosis, such an evaluation function has been generated, based on only a predetermined algorithm. However, according to the present invention, because the candidate evaluation functions are generated by using a plurality of different function-generating methods in combination, it is possible to generate an appropriate evaluation function suitable for diagnostic condition finally.

In the biological state-evaluating system according to the present invention, the function-generating method is multivariate analysis. It is thus possible to generate a candidate evaluation function by using an existing function-generating method.

The biological state-evaluating system according to the present invention verifies at least one of the discrimination rate, sensitivity, specificity, and information criterion of the candidate evaluation functions, according to at least one of bootstrap method, holdout method, and leave-one-out method. Advantageously, it is possible to generate a candidate evaluation function higher in predictability or reliability.

The biological state-evaluating system according to the present invention selects the variable of the candidate evaluation function from the verification results according to at least one of stepwise method, best path method, local search method, and genetic algorithm. Advantageously, it is possible to select the variable of the candidate evaluation function properly.

In the biological state-evaluating system according to the present invention, the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample. Thus, it is possible to generate an evaluation function higher in evaluation accuracy, by using metabolite concentration data concerning metabolite concentration higher in measurement accuracy. It is possible to generate an evaluation function higher in reliability, by using the favorable physical properties of amino acids such as high measurement accuracy and measurement variance smaller than the variance due to individual difference.

In the biological state-evaluating system according to the present invention, the metabolite concentration data to be evaluated is that of the patients with ulcerative colitis or Crohn's disease. Thus, it is possible to evaluate the disease state accurately, based on the metabolite concentration data that can be obtained from the patients easily, even for diseases giving greater physical and mental burdens to the patients in diagnosis of disease state. For example, in the case of ulcerative colitis normally diagnosed using endoscope, it is possible advantageously to evaluate the ulcerative colitis accurately without an endoscope and consequently to reduce the burden to the patient effectively. Evaluation of disease state of inflammatory bowel diseases (IBDs) was performed by a diagnostic method such as intestinal endoscope or biopsy or with a score such as CDAI (Crohn's disease activeness indicator), IOIBD, or DutchAI. However, the diagnostic method employing an intestinal endoscope or biopsy demanded a professional physician and exerted greater burden on the patient. Alternatively, the score such as CDAI or IOIBD, which includes items subjective to the patient such as the state of stomachache and feces, often could not express the disease state accurately. Evaluation of asthma disease state has been made by combination of oral examination, for example concerning the state of stridor, hematological test, lung function test, and X-ray test. Alternatively, evaluation of rheumatoid disease state has been made by combination of oral examination for example concerning joint edema, rheumatic reaction test, and X-ray test. Unfavorably, these methods contained patient-subjective factors and the disease state did not agree well with the test result. In the present invention, it is possible to evaluate the disease state of IBD, asthma and rheumatism accurately, based on the data concerning metabolite concentration in the body that is extremely objective and can be determined easily.

The biological state-evaluating apparatus according to the present invention stores the evaluation function having the metabolite concentration as the variable and evaluates the biological state to be evaluated, based on the stored evaluation function and previously acquired metabolite concentration data to be evaluated. Thus, it is possible to evaluate the biological state to be evaluated, only with input of the metabolite concentration data to be evaluated.

The biological state-evaluating apparatus according to the present invention stores at least one of the evaluation functions represented by Formulae 1 to 4 and evaluates the biological state, based on at least one of the stored evaluation functions and metabolite concentration data. Thus, it is possible to evaluate the biological state to be evaluated accurately only by inputting metabolite concentration data to be evaluated. The coefficients and the constants in respective Formulae are previously determined according to the data concerning the disease to be evaluated.

In the biological state-evaluating apparatus according to the present invention, the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample. Thus, it is possible to evaluate the biological state to be evaluated accurately, by using metabolite concentration data concerning metabolite concentration higher in measurement accuracy. For example, it is possible to evaluate the biological state to be evaluated accurately by using the favorable physical properties of amino acids such as high measurement accuracy and measurement variance smaller than the variance due to individual difference.

In the biological state-evaluating apparatus according to the present invention, the metabolite concentration data to be evaluated are of a patient with ulcerative colitis, Crohn's disease, asthma, or rheumatism. Thus, it is possible to evaluate the disease state accurately, based on the metabolite concentration data that can be obtained from the patients easily, even for diseases giving greater physical and mental burdens to the patients in diagnosis of disease state. For example, in the case of ulcerative colitis normally diagnosed using endoscope, it is possible advantageously to evaluate the ulcerative colitis accurately without an endoscope and consequently to reduce the burden to the patient effectively. Evaluation of disease state of inflammatory bowel diseases (IBDs) was performed by a diagnostic method such as intestinal endoscope or biopsy or with a score such as CDAI (Crohn's disease activeness indicator), IOIBD, or DutchAI. However, the diagnostic method employing an intestinal endoscope or biopsy demanded a professional physician and exerted greater burden on the patient. Alternatively, the score such as CDAI or IOIBD, which includes items subjective to the patient such as the state of stomachache and feces, often could not express the disease state accurately. Evaluation of asthma disease state has been made by combination of oral examination, for example concerning the state of stridor, hematological test, lung function test, and X-ray test. Alternatively, evaluation of rheumatoid disease state has been made by combination of oral examination for example concerning joint edema, rheumatic reaction test, and X-ray test. Unfavorably, these methods contained patient-subjective factors and the disease state did not agree well with the test result. In the present invention, it is possible to evaluate the disease state of IBD, asthma and rheumatism accurately, based on the data concerning metabolite concentration in the body that is extremely objective and can be determined easily.

The evaluation function-generating apparatus, the evaluation function-generating method and the evaluation function-generating program according to the present invention generate an evaluation function having the metabolite concentration as the variable, based on previously-obtained biological state information including metabolite concentration data concerning metabolite concentration and biological state indicator data concerning the indicator showing the biological state. Specifically, they (1) generate a candidate evaluation function that is a candidate of evaluation function from the biological state information according to a particular function-generating method, (2) verify the prepared candidate evaluation function according to a particular verification method, (3) select the combination of the metabolite concentration data contained in the biological state information to be used in preparing the candidate evaluation function by selecting a variable of the candidate evaluation function from the verification results according to a particular variable selection method, and generate an evaluation function, based on the verification results accumulated by repeated execution of (1), (2) and (3) by selecting a candidate evaluation function to be used as the evaluation function among the candidate evaluation functions. Thus, it is possible to generate an evaluation function optimal for evaluation of the biological state to be evaluated by verifying the evaluation function.

The evaluation function-generating apparatus, the evaluation function-generating method and the evaluation function-generating program according to the present invention generate the candidate evaluation functions from the biological state information, by using a plurality of different function-generating methods in combination. Thus, the formats of respective candidate evaluation functions prepared are different from each other, according to the function-generating methods. Advantageously, it is possible to generate an evaluation function appropriate for evaluating the biological state. Although various diagnoses including not only two-group discrimination between healthy and unhealthy, but also other diagnoses from various viewpoints (e.g., multigroup classification such as similar disease identification and progress prediction of progressive disease) are demanded in clinical diagnosis, such an evaluation function has been generated, based on only one predetermined algorithm. However, according to the present invention, because the candidate evaluation functions are generated by using a plurality of different function-generating methods in combination, it is possible to generate an appropriate evaluation function suitable for diagnostic condition finally.

In the evaluation function-generating apparatus, the evaluation function-generating method and the evaluation function-generating program according to the present invention, the function-generating method is multivariate analysis. It is thus possible to generate a candidate evaluation function by using an existing function-generating method.

The evaluation function-generating apparatus, the evaluation function-generating method and the evaluation function-generating program according to the present invention verify at least one of the discrimination rate, sensitivity, specificity, and information criterion of the candidate evaluation function, according to at least one of bootstrap method, holdout method, and leave-one-out method. Advantageously, it is possible to generate a candidate evaluation function higher in predictability or reliability.

The evaluation function-generating apparatus, the evaluation function-generating method and the evaluation function-generating program according to the present invention select the variable of the candidate evaluation function from the verification results according to at least one of stepwise method, best path method, local search method, and genetic algorithm. Advantageously, it is possible to select the variable of the candidate evaluation function properly.

In the evaluation function-generating apparatus, the evaluation function-generating method and the evaluation function-generating program according to the present invention, the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample. Thus, it is possible to generate an evaluation function higher in evaluation accuracy, by using metabolite concentration data concerning metabolite concentration higher in measurement accuracy. For example, it is possible to generate an evaluation function higher in reliability, by using the favorable physical properties of amino acids such as high measurement accuracy and measurement variance smaller than the variance due to individual difference.

In addition, the recording medium according to the present invention can make computer execute a biological state-evaluating program and an evaluation function-generating program, by making computer read and execute the biological state-evaluating program and the evaluation function-generating program recorded on the recording medium, and thus, it is possible to obtain an effect similar to that obtained by the biological state-evaluating program or the evaluation function-generating program.

BRIEF DESCRIPTION OF THE DRAWINGS

FIG. 1 is a principal configurational diagram showing the basic principle of the invention;
FIG. 2 is a diagram showing an example of the entire configuration of the present system;
FIG. 3 is a diagram showing another entire configuration of the present system;
FIG. 4 is a block diagram showing an example of the configuration of the biological state-evaluating apparatus 100 in the present system;
FIG. 5 is a chart showing an example of the information stored in the user information file 106a;
FIG. 6 is a chart showing an example of the information stored in the biological state information file 106b;
FIG. 7 is a chart showing an example of the information stored in the designated biological state information file 106c;
FIG. 8 is a chart showing an example of the information stored in the candidate evaluation function file 106d1;
FIG. 9 is a chart showing an example of the information stored in the verification result file 106d2;
FIG. 10 is a chart showing an example of the information stored in the selected biological state information file 106d3;
FIG. 11 is a chart showing an example of the information stored in the evaluation function file 106d4;
FIG. 12 is a chart showing an example of the information stored in the evaluation result file 106e;
FIG. 13 is a block diagram showing an example of the configuration of the evaluation function-generating part 102i;
FIG. 14 is a block diagram showing an example of the configuration of the client apparatus 200 in the present system;
FIG. 15 is a block diagram showing an example of the configuration of the database apparatus 400 in the present system;
FIG. 16 is a flowchart showing an example of the biological state evaluation service processing performed by using the present system;
FIG. 17 is a flowchart showing an example of the biological state evaluation processing performed in the biological state-evaluating apparatus 100;
FIG. 18 is a flowchart showing an example of the candidate evaluation function-generating processing performed in the candidate evaluation function-generating part 102i1;

FIG. 19 is a table showing the relationship among the scores of the disease states respectively of healthy people and ulcerative colitis patients, as determined by logistic regression analysis, support vector machine, discriminant analysis and MAP method, and the disease states predicted from the scores;

FIG. 20 is a table showing the relationship among the scores of newly added subjects as determined according to respective models generated, the disease states predicted from the scores, and the diagnosis results (disease states) determined by a doctor;

FIG. 21 is a table showing the relationship among the disease states of healthy people and Crohn's disease patients, the scores as determined by logistic regression analysis, support vector machine, discriminant analysis and MAP method, and the disease states predicted from the scores;

FIG. 22 is a table showing the relationship among the scores of newly added subjects, as determined according to respective models generated, the disease states predicted from the scores, and the diagnosis results (disease states) determined by a doctor;

FIG. 23 is a table showing the relationship among the diseases states of healthy rats and diabetic rats, the scores as determined by logistic regression analysis, support vector machine, discriminant analysis and MAP method, and the disease states predicted from the scores;

FIG. 24 is a table showing the relationship among the diseases states of healthy rats and diabetic rats, the scores as determined by logistic regression analysis, support vector machine, discriminant analysis and MAP method, and the disease states predicted from the scores;

FIG. 25 is a table showing the relationship between the scores of diabetic rat after insulin administration according to the respective models generated and the disease states predicted from the scores;

FIG. 26 is a graph showing the scores, as determined by logistic regression analysis, of healthy rats, diabetic rats, and insulin-administered/treated diabetic rats;

FIG. 27 is a graph showing the scores as determined by support vector machine of healthy rats, diabetic rats, and insulin-administered/treated diabetic rats;

FIG. 28 is a graph showing the scores as determined by discriminant analysis of healthy rats, diabetic rats, and insulin-administered/treated diabetic rats;

FIG. 29 is a graph showing the scores as determined by MAP method of healthy rats, diabetic rats, and insulin-administered/treated diabetic rats;

FIG. 30 is a principal configurational diagram showing the basic principle of the present invention;

FIG. 31 is a table showing the relationship among the disease states of healthy people and ulcerative colitis patients, the scores as determined by the evaluation functions 1 to 3, and the disease states predicted from the scores;

FIG. 32 is a table showing the relationship among the disease states of healthy people and Crohn's disease patients, the scores as determined by the evaluation functions 1 to 3, and the disease states predicted from the scores;

FIG. 33 is a table showing the data used in training model in the support vector machine.

FIG. 34 is a table showing the relationship among the disease states of healthy mice and asthma model mice, blood amino acid (Lys, Arg and Asn) concentration, the scores obtained by the prepared training model, and the disease states predicted from the scores; and

FIG. 35 is a table showing the relationship among the disease states of healthy mice and rheumatoid mice, the scores determined by evaluation functions 1 to 3, and the disease states predicted from the scores.

EXPLANATION OF REFERENCES

100 Biological state-evaluating apparatus
102 Controlling device
102a Instruction-analyzing part
102b Browsing processing part
102c Authentication-processing part
102d Electronic mail-generating part
102e Web page-generating part
102f Sending part
102g Biological state information-acquiring part
102h Biological state information-designating part
102i Evaluation function-generating part
102i1 Candidate evaluation function-generating part
102i2 Candidate evaluation function-verifying part
102i3 Variable-selecting part

102j Biological state-evaluating part
102k Result outputting part
104 Communication interface
106 Memory device
106a User information file
106b Biological state information file
106c Designated biological state information file
106d Evaluation function-related information database
106d1 Candidate evaluation function file
106d2 Verification result file
106d3 Selected biological state information file
106d4 Evaluation function file
106e Evaluation result file
108 Input/output interface
112 Input device
114 Output device
200 Client apparatus (information communication terminal apparatus)
300 Network
400 Database apparatus

BEST MODE(S) FOR CARRYING OUT THE INVENTION

Hereinafter, favorable embodiments of the biological state-evaluating apparatus, the biological state-evaluating method, the biological state-evaluating system, the biological state-evaluating program, the evaluation function-generating apparatus, the evaluation function-generating method, the evaluation function-generating program and the recording medium according to the present invention will be described in detail with reference to drawings. The present invention is not limited by these embodiments.

[1. Summary of the present invention]

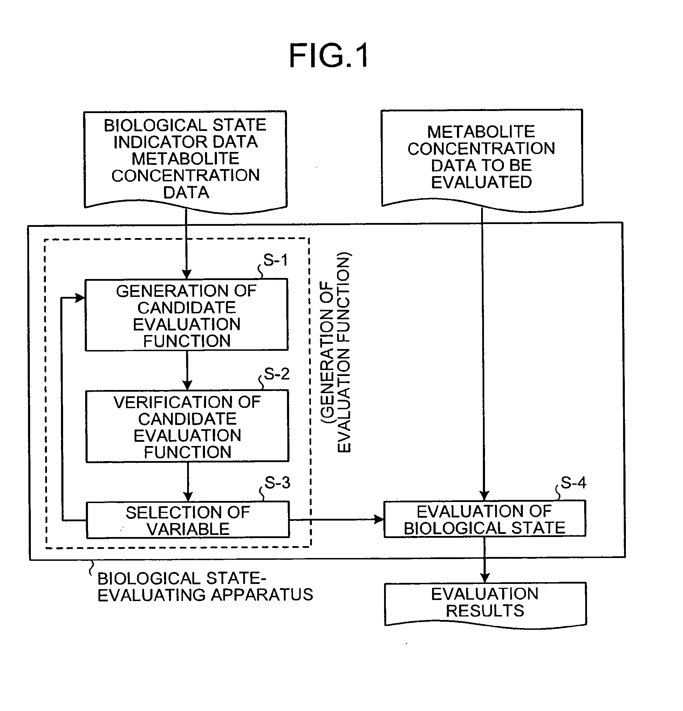

Here, the summary of the present invention will be described with reference to FIG. 1. FIG. 1 is a principal configurational diagram showing the basic principle of the present invention.
First, biological state information including metabolite concentration data concerning metabolite concentration and biological state indicator data concerning the indicator showing the biological state is obtained; and a candidate evaluation function having metabolite concentration as the variable that is a candidate of evaluation function, (e.g., $y = a_1x_1 + a_2x_2 + ... + a_nx_n$, y: biological state indicator data, $x_i$: metabolite concentration data, $a_i$: constant, i = 1,2, ..., n) is generated from the obtained biological state information according to a particular function-generating method (step S-1). Data containing defective and outliers may be removed from the obtained biological state information (data filtering, data editing).
In step S-1, the candidate evaluation functions may be formed from the biological state information by using a plurality of different function-generating methods (including those for multivariate analysis such as principal component analysis, discriminant analysis, support vector machine, multi regression analysis, logistic regression analysis, k-means method, cluster analysis, and decision tree). Specifically, a plurality of groups of candidate evaluation functions may be formed simultaneously and concurrently, with biological state information which is multivariate data including metabolite concentration data and biological state indicator data obtained by analyzing the biological samples from a plurality of healthy peoples and diseased patients by using a plurality of different algorithms. For example, two different candidate evaluation functions may be formed by performing discriminant analysis and logistic regression analysis simultaneously with different algorithms. Alternatively, a candidate evaluation function may be formed by converting biological state information with the candidate evaluation function prepared by performing principal component analysis and performing discriminant analysis of the converted biological state information. In this way, it is possible to form an appropriate evaluation function suitable for diagnostic condition finally.
The candidate evaluation function prepared by performing principal component analysis is a linear expression of variables maximizing the variance of all metabolite concentration data. The candidate evaluation function prepared by discriminant analysis is a high-powered expression of variables (including exponential and logarithmic expressions) minimizing the ratio of the sum of the variances in respective groups to the variance of all metabolite concentration data. The candidate evaluation function prepared by using support vector machine is a high-powered expression of variables (including kernel function) maximizing the boundary between groups. The candidate evaluation function prepared by multiple regression analysis is a high-powered expression of variables minimizing the sum of the distances from all metabolite concentration data. The candidate evaluation function prepared by logistic regression analysis is a fraction expression having, as a component, the natural logarithm of a number having a linear expression of a plurality of variables maximizing the likelihood as the index. The k-means method is a method of searching k pieces of neighboring metabolite concentration data in various groups designating the group containing the greatest number of the neighboring points as its data-belonging group, and selecting a variable that makes the group to which input metabolite concentration data belong agrees well with the data-belonging group. The cluster analysis is a method of clustering the points closest in entire metabolite concentration data. The decision tree is a method of ordering variables and predicting the group of metabolite concentration data from the pattern possibly held by the higher-ordered variable.

Then, the candidate evaluation function prepared in step S-1 is verified (mutually verified) by a particular verification method (step S-2). Here in step S-2, at least one of the discrimination rate, sensitivity, specificity, and information criterion of the candidate evaluation function may be verified by at least one of the bootstrap, holdout, and leave-one-out methods. In this way, it is possible to prepare a candidate evaluation function higher in predictability or reliability, by taking the biological state information and the diagnostic condition into consideration. The verification of candidate evaluation function is performed to each candidate evaluation function prepared.

The discrimination rate is the rate of the data wherein the biological state evaluated according to the present invention is correct, in all input data. The sensitivity is the rate of the biological states judged correct according to the present invention in the biological states declared unhealthy in the input data. The specificity is the rate of the biological states judged correct according to the present invention in the biological states described healthy in the input data. The information criterion is the sum of the number of the variables in the candidate evaluation function prepared and the difference in number between the biological states evaluated according to the present invention and those described in input data.

The predictability is the average of the discrimination rate, sensitivity, or specificity obtained by repeating verification of the candidate evaluation function. Alternatively, the reliability is the variance of the discrimination rate, sensitivity, or specificity obtained by repeating verification of the candidate evaluation function.

Subsequently, a combination of the metabolite concentration data contained in the biological state information to be used in preparing the candidate evaluation function is selected by selecting a variable of the candidate evaluation function from the verification result in step S-2 according to a particular variable selection method (step S-3).

Here in step S-3, the variable of the candidate evaluation function may be selected from the verification results according to at least one of stepwise method, best path method, local search method, and genetic algorithm. The best path method is a method of selecting a variable, by optimizing the evaluation index of the candidate evaluation function, while eliminating the variables contained in the candidate evaluation function one by one. The selection of variable is performed to each candidate evaluation function prepared. In this way, it is possible to select the variable of the candidate evaluation function properly.

The step S-1 is executed once again by using the biological state information including the metabolite concentration data selected in step S-3.

An evaluation function is prepared by selecting a candidate evaluation function to be used as the evaluation function from the candidate evaluation functions, based on the verification results obtained by executing the steps S-1, S-2 and S-3 repeatedly. In selecting the candidate evaluation function, for example, the optimal candidate evaluation function may be selected from the candidate evaluation functions prepared by the same function-generating method or from all candidate evaluation functions.

Thus in the present invention, it is possible to prepare an evaluation function optimal for evaluation of biological state, because the processing for preparation of candidate evaluation function, verification of candidate evaluation function, and variable selection is executed integrally in a series of processings.

Subsequently, the biological state to be evaluated is evaluated, based on the generated evaluation function and the previously acquired metabolite concentration data to be evaluated (step S-4). Specifically, an indicator of the biological state of evaluation subject is calculated by applying the metabolite concentration data of evaluation subject to the generated evaluation function. More specifically, the biological state indicator data of the evaluation subject is determined.

According to the present invention, it is possible to prepare an evaluation function, based on the biological state indicator data obtained from healthy people and patients (e.g., health state, disease name, and severity) and the metabolite concentration data thereof (e.g., blood amino acid concentration and blood biochemical concentration) and to evaluate the biological state of an evaluation subject from the metabolite concentration data of the evaluation subject, by using the evaluation function generated. According to the present invention, it is possible to prepare an evaluation function for evaluation of the biological state to be evaluated as the optimal index and evaluate (predict) various phenomenon defining the biological state of the evaluation subject. As a result, it is possible to evaluate biological state accurately according to the present invention. According to the present invention, because the information input is biological state information and the metabolite concentration data of evaluation subjects for use in preparation of evaluation function, even the user who does not have professional knowledge, for example statistical knowledge, can use the present invention easily, and, for that reason, the present invention is highly advantageous.

In the present invention, data concerning the "concentration of an amino acid, an amino acid analogue, or an amino or imino group-containing compound in a biological sample" or data concerning the "concentration of a peptide, a protein, a sugar, a lipid, a vitamin, a mineral or the metabolite thereof in a biological sample" may be suitably used as the metabolite concentration data.

In addition, the present invention may be applied suitably to evaluation of the disease state of the patients with ulcerative colitis or Crohn's disease. In addition to ulcerative colitis and Crohn's disease, the present invention may also be applied to other diseases in which the concentration of metabolites (in particular, amino acids) varies according to fluctuation of the biological state. Examples thereof include malignant tumors (lung cancer, esophageal cancer, stomach cancer, colon cancer, hepatoma, pancreatic cancer, gallbladder cancer-cholangiocarcinoma, prostate cancer, breast cancer, uterine cancer, ovarian cancer, hematopoietic tumor, hypophysis cancer, and thyroid cancer), Basedow's disease, hyperlipemia, diabetes, collagen diseases (rheumatoid arthritis, nettle rash, and systemic erythematosus), osteoporosis, arteriosclerosis obliterans (ASO), angina pectoris, myocardial infarction, cardiomyopathy, heart failure, arrhythmia, cerebrovascular diseases (cerebral infarction and cerebral aneurysm), chronic liver diseases (chronic hepatitis B/C and liver cirrhosis), acute hepatitis, fatty liver, cholelithiasis, cholecystitis, jaundice, edema, hypertension, glomerulonephritis, pyelonephritis, tubular diseases (Fanconi's syndrome and renal tubular acidosis), renal amyloidosis, toxic renal damage, gestosis, nephrosclerosis, renal failure, porphyria, methemoglobinemia, leukemia, pituitary gland diseases (lobus anterior and lobus posterior hypopituitarism, and syndrome of inappropriate antidiuretic hormone secretion), thyroidal diseases (hypothyroidism, hyperthyroidism, diffuse goiter, and thyroiditis), hyperinsulinism, hyperglucagonemia, adrenocortical diseases (hyperadrenocorticalism, hypoadrenocorticism, hyperaldosteronism, and adrenogenital syndrome), hysteromyoma, endometriosis, urinary calculus, nephrotic syndrome, anaphylactic syndrome (drug eruption), keloid, atopic dermatitis, pollinosis, asthma, tuberculosis, interstitial pneumonia-plumonary fibrosis, plumonary emphysema (COPD), cataract, glaucoma, febrile convulsion, epilepsy, periodontal disease, amyotrophic lateral sclerosis (ALS), inflammatory bowel diseases (ulcerative colitis and Crohn's disease), immunodeficiency disease, acquired immunodeficiency syndrome (AIDS), infectious disease, gout, hyperuricemia, phenylketonuria, tyrosinuria, alcaptonuria, homocystinuria, maple syrup urine disease, renal amino acid uria, Niemann-Pick disease, Gaucher's disease, Tay-Sachs disease, mitochondrial encephalomyopathy, glycogenosis, galactosemia, Lesch-Nyhan syndrome, Wilson's disease, muscular dystrophy, hemophilia, duodenal ulcer, gastric ulcer, gastrisis, gastric polyp, gastric adenoma, Alzheimer's disease, Parkinson's disease, polio, and the like.

[2. System configuration]

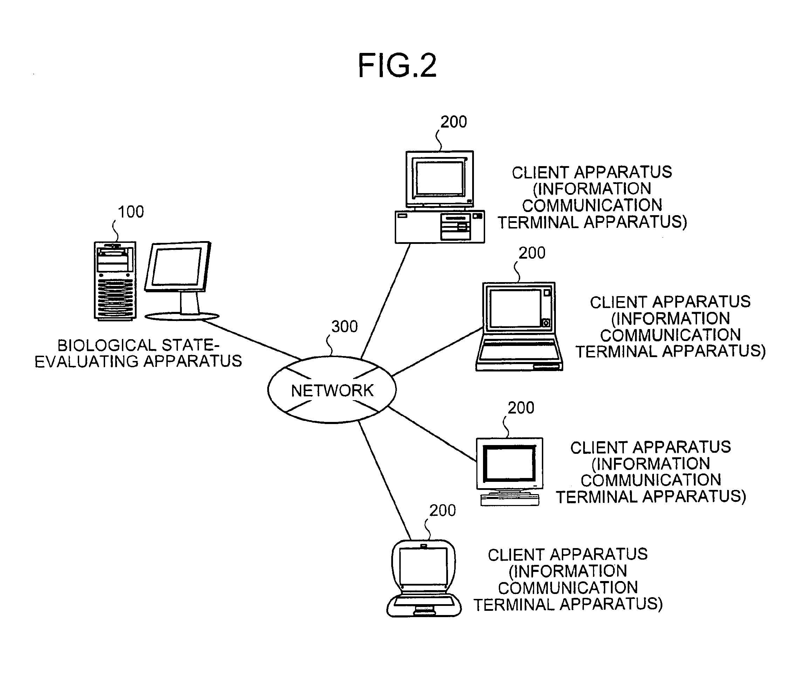

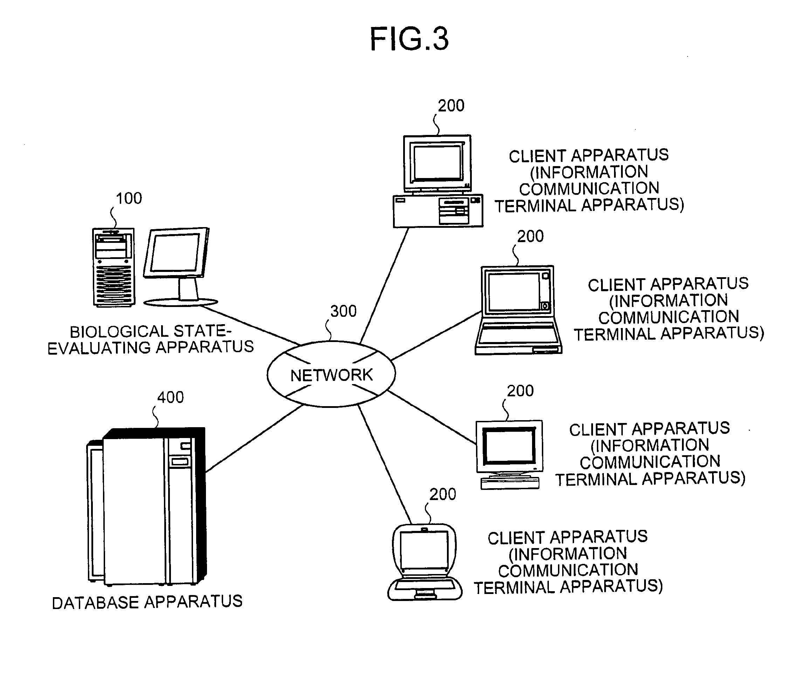

Hereinafter, the configuration of the biological state-evaluating system to which the present invention is applied (hereinafter, referred to as present system) will be described with reference to FIGS. 2 to 15. First, the entire configuration of the present system will be described with reference to FIGS. 2 and 3.

FIG. 2 is a diagram showing an example of the entire configuration of the present system. FIG. 3 is a diagram showing another example of the entire configuration of the present system.

As shown in FIG. 2, the present system includes a biological state-evaluating apparatus 100 which evaluates biological state and client apparatuses 200 (information-communicating terminal apparatuses) which provide the metabolite concentration data to be evaluated that are connected to each other communicatively via a network 300. As shown in FIG. 3, the present system may have, in addition to the biological state-evaluating apparatus 100 and the client apparatuses 200, a database apparatus 400 storing, for example, the biological state information to be used in preparing an evaluation function in the biological state-evaluating apparatus 100 and the evaluation function prepared in the biological state-evaluating apparatus 100, that are connected to each other communicatively via the network 300. Thus, the information on biological state is transmitted via the network 300 from the biological state-evaluating apparatus 100 to the client apparatuses 200 and the database apparatus 400, or from the client apparatuses 200 and the database apparatus 400 to the biological state-evaluating apparatus 100. The "information on biological state" is information on the measured values of particular items of the biological state of organisms including human. Examples of the information on biological state include the disease state information described below. The information on biological state is generated in the biological state-evaluating apparatus 100, client apparatus 200, and other apparatuses (e.g., various measuring apparatuses) and stored mainly in the database apparatus 400.

[2-1. System configuration of biological state-evaluating apparatus 100]

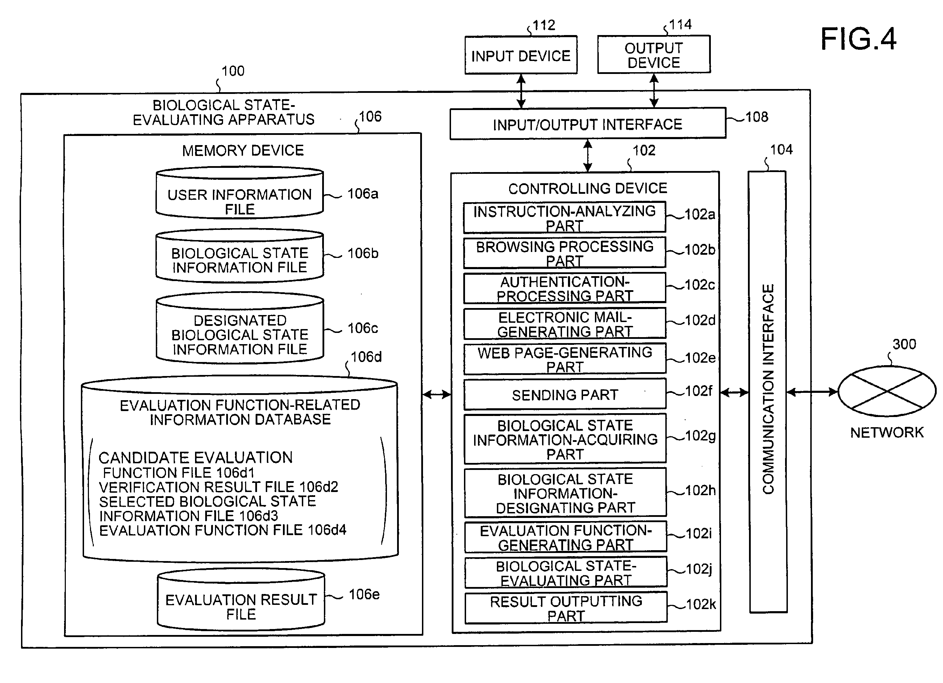

FIG. 4 is a block diagram showing an example of the configuration of the biological state-evaluating apparatus 100 in the present system, and only the region in the configuration relevant to the present invention is shown conceptually. The biological state-evaluating apparatus 100 includes a controlling device 102, such as CPU, which integrally controls the biological state-evaluating apparatus 100, a communication interface 104 which connects the biological state-evaluating apparatus 100 communicatively via the network 300 and also via a communication apparatus such as router or a wired or wireless communication line such as private line, a memory device 106 which stores various databases, tables and files, and an input/output interface 108 connected to an input device 112 and an output device 114, and these parts are connected to each other communicatively via any communication channel. The biological state-evaluating apparatus 100 may be present with various analyzers (e.g., amino acid analyzer, etc.) in the same housing. Typical shape of the configuration of the biological state-evaluating apparatus 100 is not limited to that shown in the figure, and all or part of it may be disintegrated or integrated functionally or physically at any rate, for example, according to various loads. For example, part of the evaluation process may be performed via a CGI (Common Gateway Interface).

The memory device 106 is a storage means, and examples thereof include memory apparatuses such as RAM and ROM, hard disk drives such as hard disk, flexible disk, optical disk, and the like. The memory device 106 also stores computer programs giving instructions to CPU for various processing together with OS (Operating System). As shown in the figure, the memory device 106 stores a user information file 106a, a biological state information file 106b, a designated biological state information file 106c, an evaluation function-related information database 106d, and an evaluation result file 106e.

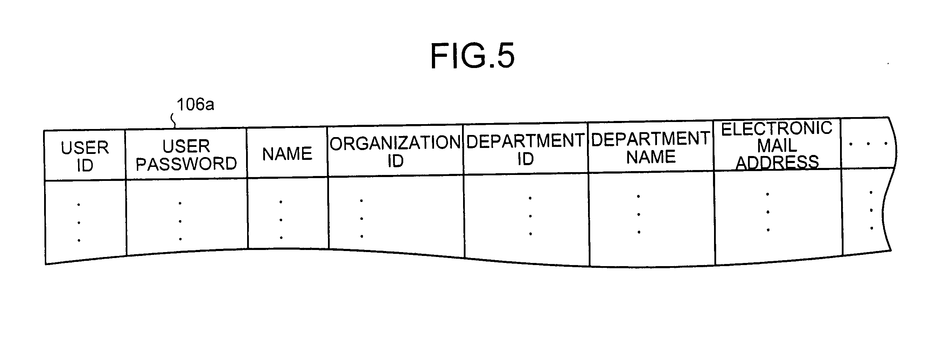

The user information file 106a stores information about users (user information). FIG. 5 is a chart showing an example of the information stored in the user information file 106a. As shown in FIG. 5, the information stored in the user information file 106a contains user ID identifying the user uniquely, user password for authentication of the user, user name, organization ID uniquely identifying the organization of the user, department ID uniquely identifying the department of the user organization, department name, and electronic mail address of the user that are correlated to each other.

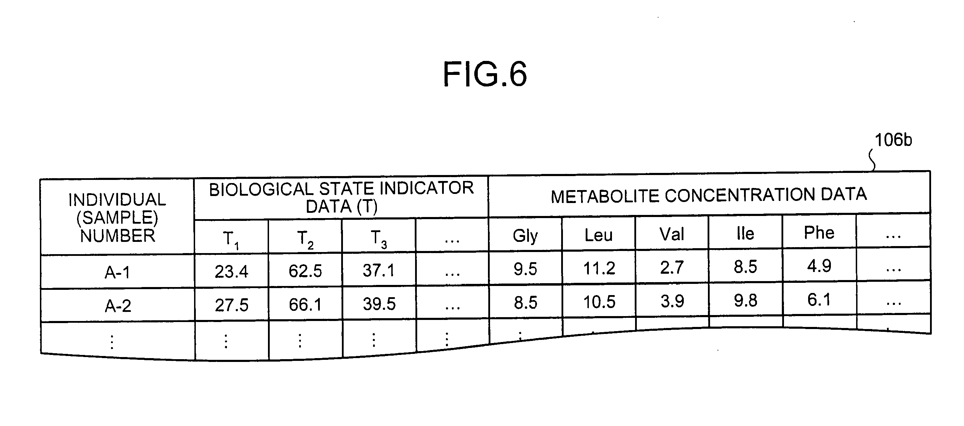

Back in FIG. 4, the biological state information file 106b stores the biological state information including biological state indicator data and metabolite concentration data. FIG. 6 is a chart showing an example of the information stored in the biological state information file 106b. As shown in FIG. 6, the information stored in the biological state information file 106b include individual (sample) number, biological state indicator data corresponding to the biological state indicator (T), and metabolite concentration data concerning the concentration of the metabolite (e.g., amino acid in FIG. 6) that are correlated to each other. In FIG. 6, the biological state indicator data and the metabolite concentration data are assumed to be numerical values, i.e., on continuous scale, but the biological state indicator data and the metabolite concentration data may be expressed on nominal scale or ordinal scale. In the case of nominal or ordinal scale, any number may be allocated to each state for analysis. The biological state indicator data is a single known state indicator, or a marker, of biological state (e.g., severity of cancer, liver cirrhosis, dementia, or obesity), and numerical data including blood concentration of a particular metabolite, enzyme activity, gene expression amount, dementia index (HDSR) and others may also be used as the biological state indicator data. Data on the concentration of amino acid, amino acid analogue, carbohydrate, lipid, nucleotide, or the like in biological sample, or combination of such data with other biological information (e.g., sex difference, age, smoking, digitalized electrocardiogram waveform, enzyme concentration, and gene expression quantity) may be used as the metabolite concentration data.

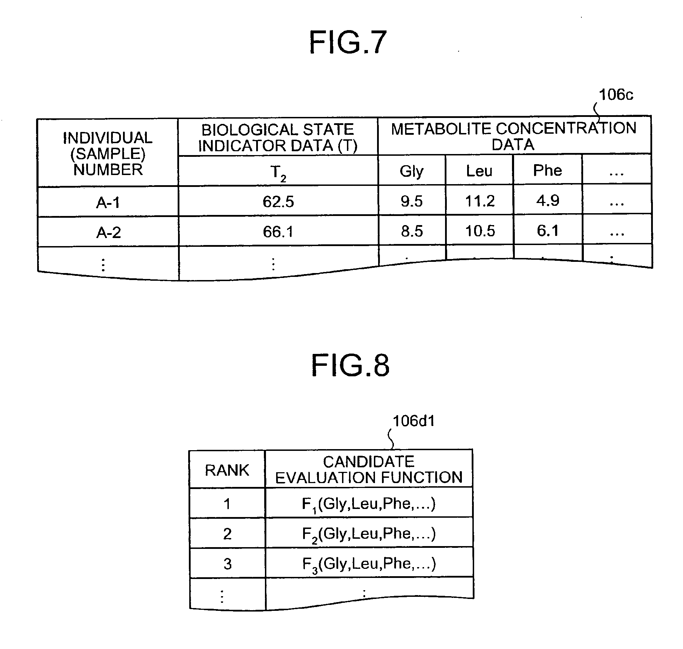

Back in FIG. 4, the designated biological state information file 106c stores the biological state information of which the biological state indicator data and the metabolite concentration data are designated in the biological state information-designating part 102h described below. The evaluation function-generating part 102i described below generates an evaluation function, based on the designated biological state information. FIG. 7 is a chart showing an example of the information stored in the designated biological state information file 106c. As shown in FIG. 7, the information stored in the designated biological state information file 106c includes individual (sample) number, biological state indicator data corresponding to the designated biological state indicator (T), and metabolite concentration data on the concentration of a designated metabolite (e.g., amino acid in FIG. 7) that are correlated to each other.

Back in FIG. 4, the evaluation function-related information database 106d stores: a candidate evaluation function file 106d1 storing the candidate evaluation function prepared in the candidate evaluation function-generating part 102i1 contained in the evaluation function-generating part 102i described below; a verification result file 106d2 storing the verification results in the candidate evaluation function-verifying part 102i2 contained in the evaluation function-generating part 102i described below; a selected biological state information file 106d3 storing the biological state information containing the combination of metabolite concentration data selected in the variable-selecting part 102i3 contained in the evaluation function-generating part 102i described below; and an evaluation function file 106d4 storing the evaluation function generated in the evaluation function-generating part 102i described below.

The candidate evaluation function file 106d1 stores the candidate evaluation function generated in the candidate evaluation function-generating part 102i1 described below. FIG. 8 is a chart showing an example of the information stored in the candidate evaluation function file 106d1. As shown in FIG. 8, the information stored in the candidate evaluation function file 106d1 includes rank, and candidate evaluation function (e.g., $F_1$(Gly, Leu, Phe, ...), $F_2$(Gly, Leu, Phe, ...), or $F_3$(Gly, Leu, Phe, ...) in FIG. 8) that are correlated to each other.

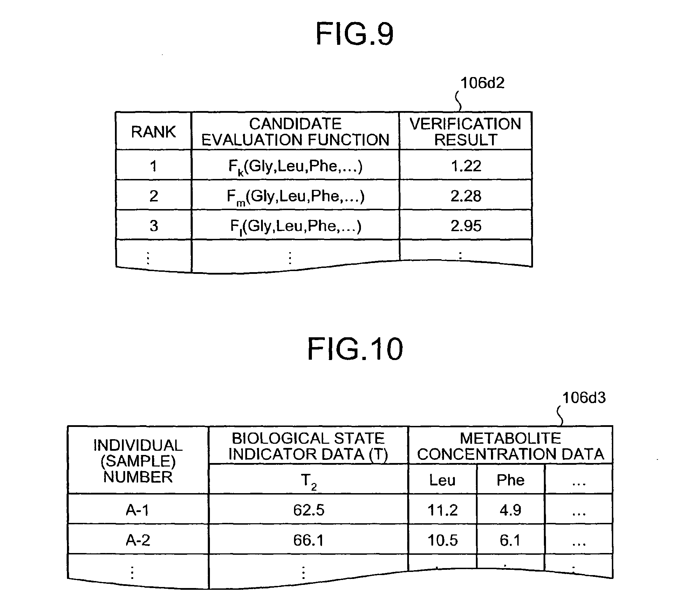

The verification result file 106d2 stores the verification results verified in the candidate evaluation function-verifying part 102i2 described below. FIG. 9 is a chart showing an example of the information stored in the verification result file 106d2. As shown in FIG. 9, the information stored in the verification result file 106d2 includes rank, candidate evaluation function (e.g., $F_k$(Gly, Leu, Phe, ...), $F_m$(Gly, Leu, Phe, ...), $F_1$(Gly, Leu, Phe, ...) in FIG. 9), and the results of each verification of candidate evaluation function (e.g., evaluation value) that are correlated to each other.

The selected biological state information file 106d3 stores the biological state information including the combination of metabolite concentration data corresponding to the variable selected in the variable-selecting part 102i3 described below. FIG. 10 is a chart showing an example of the information stored in the selected biological state information file 106d3. As shown in FIG. 10, the information stored in the selected biological state information file 106d3 includes individual (sample) number, the biological state indicator data corresponding to the biological state indicator (T) designated in the biological state information-designating part 102h described below, and the metabolite concentration data concerning the concentration of the metabolite selected in the variable-selecting part 102i3 described below (e.g., amino acid in FIG. 10) that are correlated to each other.

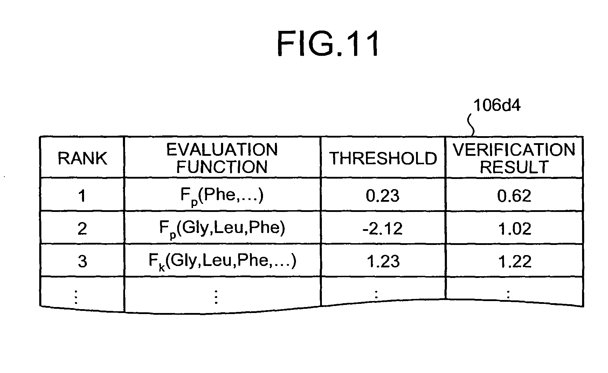

The evaluation function file 106d4 stores the evaluation function generated in the evaluation function-generating part 102i described below. FIG. 11 is a chart showing an example of the information stored in the evaluation function file 106d4. As shown in FIG. 11, the information stored in the evaluation function file 106d4 includes rank, evaluation function (e.g., Fp(Phe, ...), Fp(Gly, Leu, Phe), $F_k$(Gly, Leu, Phe, ...) in FIG. 11), a particular threshold corresponding to each function-generating method, and verification results of each evaluation function (e.g., evaluation value) that are correlated to each other.

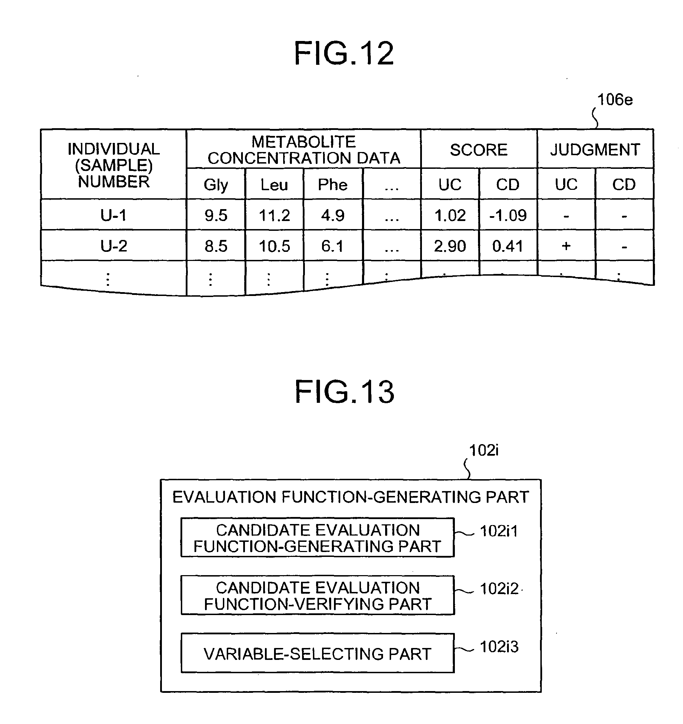

Back in FIG. 4, the evaluation result file 106e stores the evaluation results obtained in the biological state-evaluating part 102j described below. FIG. 12 is a chart showing an example of the information stored in the evaluation result file 106e. The information stored in the evaluation result file 106e includes subject (sample) number of individual to be evaluated, the previously acquired metabolite concentration data to be evaluated, score calculated by using the evaluation function, and evaluation results (judgment result, prediction result) that are correlated to each other.

In addition, the memory device 106 stores various Web data, CGI programs, and others for providing a web site to the client apparatuses 200. The Web data include various data for displaying on the Web page described below, and the data are generated as a HTML or XML text file or the like. Other temporary files such as files for the components for generation of Web data and for operation are also stored in the memory device 106. In addition, as needed, it may store sound files in the WAVE or AIFF Format for transmission to client apparatuses 200 and image files of still image or motion picture in the JPEG or MPEG2 format.

Back in FIG. 4, the communication interface 104 allows communication between the biological state-evaluating apparatus 100 and the network 300 (or communication apparatus such as router). Thus, the communication interface 104 has a function to transmit data via a communication line to and from other terminals.

The input/output interface 108 is connected to the input device 112 and the output device 114. A monitor (including home television), a speaker, or a printer may be used as the output device 114 (a monitor is described often as the output device 114 below). A keyboard, a mouse, a microphone, or a monitor functioning as a pointing device together with a mouse may be used as the input device 112.

The controlling device 102 has an internal memory storing control programs such as OS (Operating System), programs for various processing procedures, and other needed data, and performs information processing for execution of various processing according to these programs. As shown in the figure, the controlling device 102 includes grossly, the instruction-analyzing part 102a, the browsing processing part 102b, the authentication-processing part 102c, the electronic mail-generating part 102d, the Web page-generating part 102e, the sending part 102f, the biological state information-acquiring part 102g, the biological state information-designating part 102h, the evaluation function-generating part 102i, the biological state-evaluating part 102j, and the result outputting part 102k. The controlling device 102 performs data processing (data filtering and data editing) such as removal of data containing defective values or many outliers and of variables for the defective value-containing data in the biological state information obtained in the biological state information-acquiring part 102g described below.

The instruction analyzing part 102a analyzes the instruction from the client apparatus 200 or the database apparatus 400 and sends the instruction to other parts in the controlling device 102 according to the analytical result. Upon receiving browsing instruction for various screens from the client apparatus 200, the browsing processing part 102b generates and transmits the web data for these screens. Upon receiving authentication instruction from the client apparatus 200 or the database apparatus 400, the authentication-processing part 102c performs authentication. The electronic mail-generating part 102d generates an electronic mail containing various information. The Web page-generating part 102e generates a Web page for user browsing. The sending part 102f sends various information to the client apparatus 200 of the user and the evaluation function and the evaluation results to the client apparatus 200 to which biological state information has been sent.

The biological state information-acquiring part 102g acquires the biological state information including metabolite concentration data concerning metabolite concentration and also biological state indicator data concerning the indicator showing the biological state and the metabolite concentration data to be evaluated.

The biological state information-designating part 102h designates the biological state indicator data and metabolite concentration data to be processed in generating an evaluation function.

The evaluation function-generating part 102i generates an evaluation function having the metabolite concentration as the variable, based on the biological state information obtained in the biological state information-acquiring part 102g. Specifically, the evaluation function-generating part 102i generates an evaluation function from the biological state information designated in the biological state information-designating part 102h, by selecting a candidate evaluation function to be used as the evaluation function from the candidate evaluation functions prepared in the candidate evaluation function-generating part 102i1 described below, according to the verification results accumulated by repeating the processings in the candidate evaluation function-generating part 102i1, the candidate evaluation function-verifying part 102i2 and the variable-selecting part 102i3 described below.

Hereinafter, the configuration of the evaluation function-generating part 102i will be described with reference to FIG. 13. FIG. 13 is a block diagram showing the configuration of the evaluation function-generating part 102i, and only region in the configuration related to the present invention is shown conceptually. The evaluation function-generating part 102i has a candidate evaluation function-generating part 102i1, a candidate evaluation function-verifying part 102i2, and a variable-selecting part 102i3, additionally. The candidate evaluation function-generating part 102i1 generates a candidate evaluation function that is a candidate of the evaluation function from the biological state information according to a particular function-generating method. Specifically, the candidate evaluation function-generating part 102i1 generates the candidate evaluation functions from the biological state information, by using a plurality of different function-generating methods. The candidate evaluation function-verifying part 102i2 verifies the candidate evaluation functions prepared in the candidate evaluation function-generating part 102i1 according to a particular verification method. Specifically, the candidate evaluation function-verifying part 102i2 verifies at least one of the discrimination rate, sensitivity, specificity, and information criterion of the candidate evaluation functions according to at least one of bootstrap method, holdout method, and leave-one-out method. The variable-selecting part 102i3 selects the combination of the metabolite concentration data contained in the biological state information to be used in preparing the candidate evaluation function, by selecting a variable of the candidate evaluation function from the verification results in the candidate evaluation function-verifying part 102i2 according to a particular variable selection method. Specifically, the variable-selecting part 102i3 selects the variable of the candidate evaluation function from the verification results according to at least one of stepwise method, best path method, local search method, and genetic algorithm.

If a previously generated evaluation function is stored in a particular region of the memory device 106, the evaluation function-generating part 102i may generate an evaluation function by selecting a desired evaluation function out of the memory device 106.

Alternatively, the evaluation function-generating part 102i may generate the evaluation function by selecting a desired evaluation function from the evaluation functions previously stored in the memory device of another computer apparatus (e.g., database apparatus 400) and downloading it via the network 300.

Back in FIG. 4, the biological state-evaluating part 102j evaluates (predicts) the biological state to be evaluated, based on the evaluation function generated in the evaluation function-generating part 102i and the previously acquired metabolite concentration data to be evaluated. Specifically, the biological state to be evaluated is evaluated (predicted) by substituting metabolite concentration data to be evaluated into the evaluation function generated.

The result outputting part 102k outputs, for example, the results (including the evaluation results in the biological state-evaluating part 102j) of processing in each part of the controlling device 102 to the output device 114 or the like.

[2-2. System configuration of client apparatus 200]

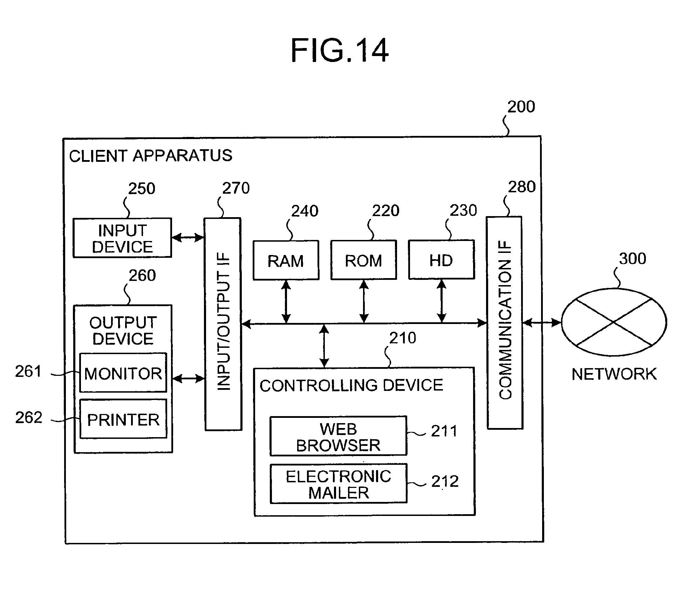

FIG. 14 is a block diagram showing an example of the configuration of the client apparatus 200 in the present system, and only region in the configuration related to the present invention is shown conceptually.

As shown in FIG. 14, the client apparatus 200 has a controlling device 210, a ROM 220, a HD 230, a RAM 240, an input device 250, an output device 260, input/output IF 270, and a communication IF 280, and the parts are connected communicatively with each other via an optional communication channel. The controlling device 210 has a Web browser 211 and an electronic mailer 212. The Web browser 211 performs browsing processing of interpreting the Web data and displaying the interpreted Web data on a monitor 261 described below. The Web browser 211 may contain various plug-in software, such as stream player, having functions, for example, to receive, display or feedback streaming screen image. The electronic mailer 212 sends and receives electronic mails using a particular protocol (e.g., SMTP (Simple Mail Transfer Protocol) or POP3 (Post Office Protocol version 3)). The input device 250 is, for example, a keyboard, mouse, or microphone. The monitor 261 described below also functions as a pointing device together with a mouse. The output device 260 is an output means that outputs the information received via the communication IF 280 and includes the monitor (including home television) 261 and the printer 262. In addition, the output device 260 may have a speaker or the like additionally. The communication IF 280 connects the client apparatus 200 with the network 300 (or communication apparatus such as router) communicatively. In other words, the client apparatuses 200 are connected to the network 300 via a communication apparatus such as modem, TA, or router or a private line. In this way, the client apparatuses 200 can access to the biological state-evaluating apparatus 100 and the database apparatus 400 by using a particular protocol.

The client apparatus 200 may be realized with an information processing apparatus of an information processing terminal such as known personal computer, workstation, family computer, Internet TV, PHS terminal, mobile phone terminal, mobile part communication terminal or PDA, as it is connected to peripheral parts such as printer, monitor, and image scanner as needed, and also as software (including programs and data) giving Web data-browsing function and electronic mail function is installed in the information processing apparatus. All or part of the controlling device 210 in client apparatus 200 may be performed by a CPU and programs, and read and executed by the CPU. Thus, computer programs for giving instructions to the CPU and executing various processing together with the OS (Operating System) are recorded in the ROM or HD. The computer programs, which are executed as they are loaded in RAM, constitute the controlling device 210 with the CPU. The computer programs may be stored in an application program server connected via any network to the client apparatus 200, and the client apparatus 200 may download all or part of them as needed. All or any part of the controlling device 210 may be realized as hardware such as wired-logic.

[2-3. System configuration of network 300]

The network 300 has a function to connect the biological state-evaluating apparatus 100, the client apparatuses 200, and the database apparatus 400 mutually, communicatively to each other, and is, for example, the Internet, intranet, or LAN (both wired/wireless). The network 300 may be VAN, personal computer communication network, public telephone network (including both analog and digital), leased line network (including both analog and digital), CATV network, portable switched network or portable packet-switched network (including IMT2000 system, GSM system, PDC/PDC-P system, and the like), wireless calling network, local wireless network such as Bluetooth, PHS network, satellite communication network (including CS, BS, ISDB and the like), or the like.

[2-4. System configuration of database apparatus 400]

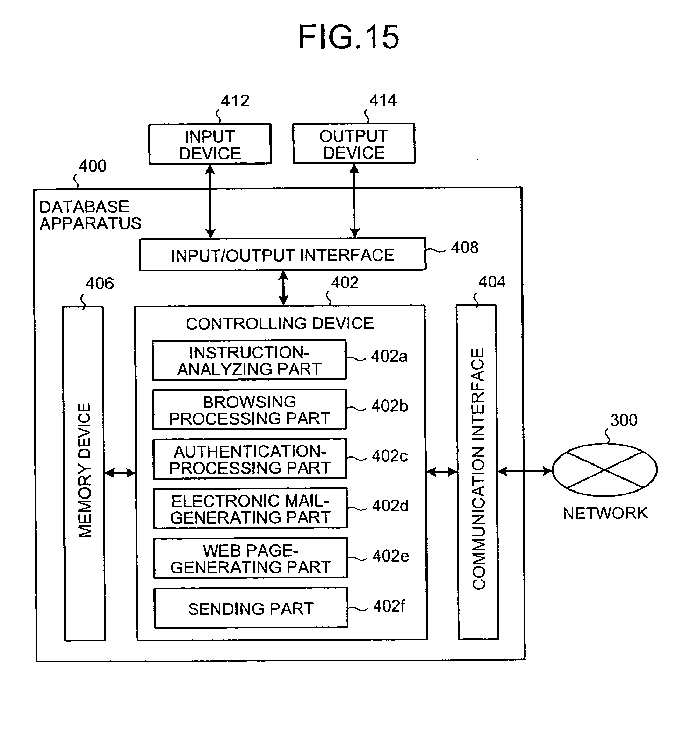

FIG. 15 is a block diagram showing an example of the configuration of the database apparatus 400 in the present system, and only region in the configuration related to the present invention is shown conceptually.

The database apparatus 400 has a function to store the biological state information to be used in preparing an evaluation function in the biological state-evaluating apparatus 100, the evaluation function prepared in the biological state-evaluating apparatus 100, and others. As shown in FIG. 15, the database apparatus 400 has mainly, a controlling device 402, such as CPU, which controls the entire database apparatus 400 integrally, a communication interface 404 connected to a communication apparatus such as router (not shown in the figure) connected, for example, to a communication line, a memory device 406 storing various data, tables or the like, and an input/output interface 408 connected to an input device 412 and an output device 414, and the parts are connected communicatively to each other via any communication channel. The database apparatus 400 is connected to the network 300 communicatively via a communication apparatus such as router and a wired or wireless communication line such as private line.

The memory device 406 is a storage means, and may be, for example, memory apparatus such as RAM or ROM, hard disk drive such as hard disk, flexible disk, optical disk, or the like. Various programs, tables, files, web-page files, and others used in various processings are stored in the memory device 406. The communication interface 404 allows communication between the database apparatus 400 and the network 300 (or communication apparatus such as router). Thus, the communication interface 404 has a function to communicate data with other terminal via a communication line. The input/output interface 408 is connected to an input device 412 and an output device 414. A monitor (including home television), speaker, or printer may be used as the output device 414 (hereinafter, a monitor may be described as the output device 414). A keyboard, a mouse, a microphone, or a monitor functioning as a pointing device together with a mouse may be used as the input device 412.

The controlling device 402 contains an internal memory storing control programs such as OS (Operating System), programs for various processing procedures, and other needed data, and performs information processing for execution of various processing according to these programs. As shown in the figure, the controlling device 402 includes grossly an instruction-analyzing part 402a, a browsing processing part 402b, an authentication-processing part 402 c, an electronic mail-generating part 402d, a Web page-generating part 402e, and a sending part 402f.

The instruction analyzing part 402a analyzes the instruction from the biological state-evaluating apparatus 100 and client apparatus 200 and sends the instruction to other parts in the controlling device 402 according to the analytical result. Upon receiving various screen-browsing instructions from the biological state-evaluating apparatus 100 and the client apparatus 200, the browsing processing part 402b generates and transmits the web data for these screens. The authentication-processing part 402c upon receipt of authentication instruction from the biological state-evaluating apparatus 100 or the client apparatus 200, performs authentication. The electronic mail-generating part 402d generates an electronic mail containing various information. The Web page-generating part 402e generates a Web page for user browsing. The sending part 402f sends various information to the user's biological state-evaluating apparatus 100 or the client apparatus 200, and the evaluation function and the evaluation results to the biological state-evaluating apparatus 100 or the client apparatus 200 to which biological state information is sent.

[3. Processing in system]

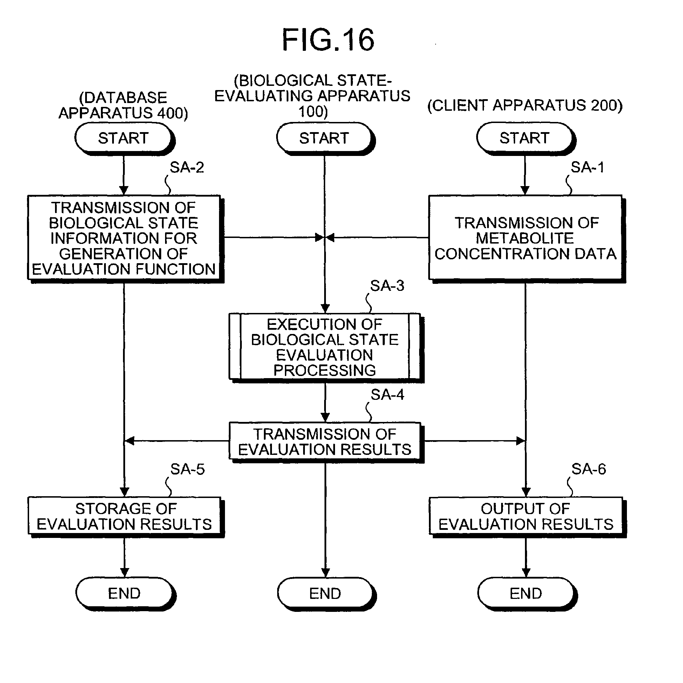

Hereinafter, an example of the processing in the present system in the configuration above will be described with reference to FIGS. 16 to 18.

[3-1. Biological state evaluation service processing]

Here, an example of the biological state evaluation service processing performed by using the present system will be described with reference to FIG. 16. FIG. 16 is a flowchart showing an example of the biological state evaluation service processing performed by using the present system.

First, the client apparatus 200 connects to the biological state-evaluating apparatus 100 via the network 300, when the user specifies the Web site address (such as URL) provided from the biological state-evaluating apparatus 100 via the input device 250 on the screen displaying Web browser 211. Specifically, when the user instructs update of the Web browser 211 screen on the client apparatus 200, the Web browser 211 sends the Web site URL using a particular protocol via the communication IF 280, transmits an instruction to the biological state-evaluating apparatus 100 to transmit the Web page corresponding to the biological state information transmission screen.

Then, the instruction-analyzing part 102a in the biological state-evaluating apparatus 100, upon receipt of the instruction from the client apparatus 200, analyzes the transmitted instruction, and sends the instruction to other parts in the controlling device 102 according to the analytical result. When the transmitted instruction is an instruction to send the Web page corresponding to the biological state information transmission screen, mainly the browsing processing part 102b obtains Web data for display from the Web page stored in a particular region of the memory device 106 and sends the Web data to the client apparatus 200 via the communication interface 104. The client apparatus 200 is identified with the IP address transmitted from the client apparatus 200 with the transmission instruction. When there is transmission instruction for Web page by the user, the biological state-evaluating apparatus 100 demands input of user ID or user password from the user. If the user ID and password are input, the authentication-processing part 102c examines the input user ID and password by comparing them with the user ID and user password stored in the user information file 106a for authentication, and the browsing processing part 102b sends the Web data only when the user is authenticated.

Then, the client apparatus 200 receives the Web data transmitted from the biological state-evaluating apparatus 100 via the communication IF 280, examines the Web data with the Web browser 211, and displays the biological state information transmission screen on the monitor 261.

The instruction for screen transmission from the client apparatus 200 to the biological state-evaluating apparatus 100, the transmission of the Web data from the biological state-evaluating apparatus 100 to the client apparatus 200 and the display of the Web page in the client apparatus 200 are performed almost similarly, and thus, detailed description will not be provided below.

When the user inputs and selects the metabolite concentration data to be evaluated via the input device 250 of client apparatus 200, the client apparatus 200 sends an identifier for identifying input information and selected item to the biological state-evaluating apparatus 100 (step SA-1). Thus, the user can send the metabolite concentration data to be evaluated to the biological state-evaluating apparatus 100. In step SA-1, transmission of the metabolite concentration data to the biological state-evaluating apparatus 100 may be performed, for example, by using an existing file transfer technology such as FTP.

Then, the instruction-analyzing part 102a of the biological state-evaluating apparatus 100 examines the identifier transmitted and analyzes the instruction from the client apparatus 200, and sends instruction to transmit the biological state information for use in generation of evaluation function to the database apparatus 400.

The instruction-analyzing part 402a of database apparatus 400 then analyzes the transmission instruction from the biological state-evaluating apparatus 100 and transmits the biological state information stored in a particular region of the memory device 406 via the communication interface 404 to the biological state-evaluating apparatus 100 (step SA-2). The database apparatus 400 may send the updated newest biological state information to the biological state-evaluating apparatus 100.

The controlling device 102 (or biological state information-acquiring part 102g) of the biological state-evaluating apparatus 100 then receives and acquires the biological state information sent from the database apparatus 400 via the communication interface 104 and executes [3-2.biological state evaluation processing] described below (step SA-3).

The sending part 102f of the biological state-evaluating apparatus 100 then sends the biological state evaluation results obtained in step SA-3 to the client apparatus 200 that have sent the metabolite concentration data to be evaluated to the biological state-evaluating apparatus 100 and the database apparatus 400 (step SA-4). Specifically, the Web page-generating part 102e of the biological state-evaluating apparatus 100 first generates the Web page for display of the evaluation result data of the user-transmitted biological state information and stores it in a particular memory region of the memory device 106. Then, the user is authenticated as described above by inputting a predetermined URL into the Web browser 211 of client apparatus 200 via the input device 250, and sends a Web page-browsing instruction for display of the evaluation result data stored in the memory device 106, in the client apparatus 200, to the biological state-evaluating apparatus 100. The browsing processing part 102b of the biological state-evaluating apparatus 100 then examines the browsing instruction transmitted from the client apparatus 200, reads out the Web page for display of the evaluation result data stored in the memory device 106, and sends the Web data corresponding to the Web page read out in the sending part 102f to the client apparatus 200. Only the evaluation result data may be sent to the database apparatus 400, or alternatively, the data identical with the Web data sent to the client apparatus 200 may be transmitted.

Here in step SA-4, the biological state-evaluating apparatus 100 may notify the user with the evaluation results by electronic mail. Specifically, the electronic mail-generating part 102d of the biological state-evaluating apparatus 100 acquires the user electronic mail address with reference to the user information stored in the user information file 106a at the transmission timing for example based on the user ID. The electronic mail-generating part 102d then generates electronic mail data including user name and evaluation result data, with the electronic mail address obtained as its destination address. The sending part 102f sends the data concerning the electronic mail generated. Alternatively in step SA-4, the evaluation result data may be transmitted to the client apparatus 200 by using for example an existing file transfer technology such as FTP.

Then, the controlling device 402 of the database apparatus 400 receives the evaluation result data or the Web data transmitted from the biological state-evaluating apparatus 100 via the communication interface 404, and stores (accumulates) the evaluation result data or the Web data in a particular memory region of the memory device 406 (step SA-5).

The client apparatus 200 receives the Web data transmitted from the biological state-evaluating apparatus 100 via the communication IF 280, analyzes the Web data with the Web browser 211, and outputs the Web page screen displaying the evaluation result data on the monitor 261 (step SA-6). The user browses the Web page displayed on the monitor 261 of client apparatus 200 and confirms the evaluation results concerning the biological state of evaluation subject. The user can print out the content of the Web page displayed on the monitor 261 on a printer 262. When the evaluation result data are transmitted by electronic mail from the biological state-evaluating apparatus 100, the user receives the transmitted electronic mail with the electronic mailer 212 of client apparatus 200 at any time, and read the received electronic mail displayed on the monitor 261 by the known function of the electronic mailer 212. The user can print out the content of the electronic mail displayed on the monitor 261 in the printer 262.

These are description of the biological state evaluation service processing.

[3-2. Biological state evaluation processing]

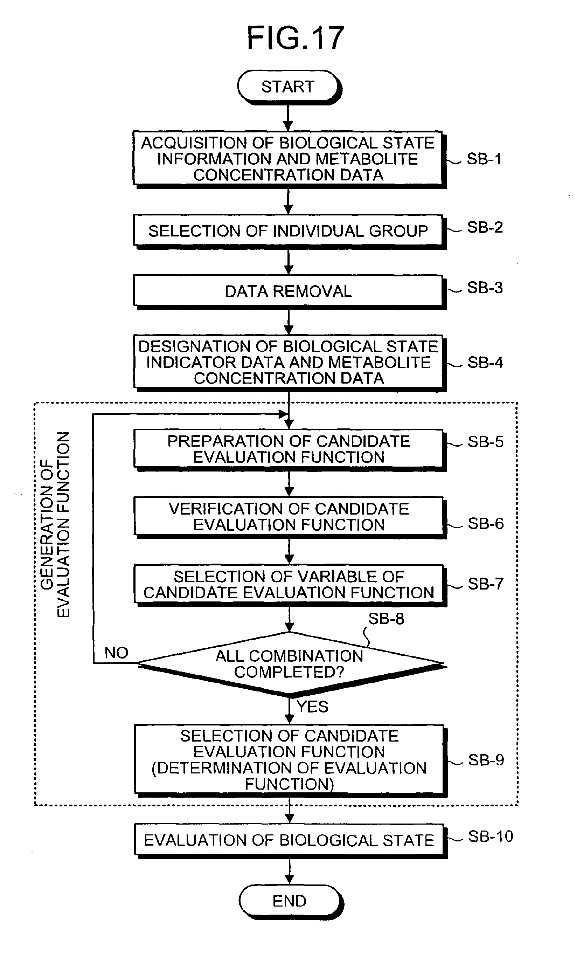

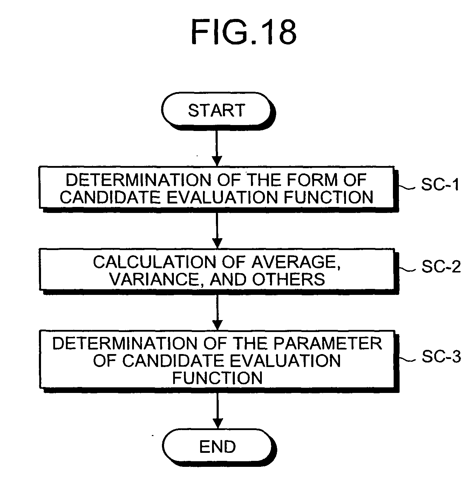

Here, an example of the biological state evaluation processing performed in the biological state-evaluating apparatus 100 will be described in detail with reference to FIGS. 17 and 18. FIG. 17 is a flowchart showing an example of the biological state evaluation processing performed in the biological state-evaluating apparatus 100.

First, the biological state information-acquiring part 102g acquires the biological state information transmitted from the database apparatus 400 via the communication interface 104 and the metabolite concentration data to be evaluated transmitted from the client apparatus 200, stores the obtained biological state information in a particular region of the biological state information file 106b, and stores the obtained metabolite concentration data in a particular region of the evaluation result file 106e (step SB-1). In step SB-1, the biological state information may not be acquired from the database apparatus 400, but, for example, may be stored previously in the biological state information file 106b of the memory device 106.

Then, the controlling device 102 selects individuals (samples) for use in preparation of the evaluation function in the biological state information obtained in step SB-1 (step SB-2).

The controlling device 102 then removes data unfavorable in preparation of the evaluation function (data containing defective values, outliers or the like) in the biological state information (step SB-3).

The biological state information-designating part 102h then designates biological state indicator data and metabolite concentration data in the biological state information, and stores the biological state information including the designated biological state indicator data and the metabolite concentration data in a particular memory region of the designated biological state information file 106c (step SB-4).

The evaluation function-generating part 102i then generates an evaluation function having the metabolite concentration as the variable, based on the biological state information including the biological state indicator data and the metabolite concentration data designated in step SB-4. Specifically, the candidate evaluation function-generating part 102i1 first generates a candidate evaluation function that is a candidate of evaluation function based on the biological state information including the biological state indicator data and the metabolite concentration data designated in step SB-4 according to a particular function-generating method, and stores the prepared candidate evaluation function in a particular memory region of the candidate evaluation function file 106d1 (step SB-5: candidate evaluation function-generating processing).

An example of the candidate evaluation function-generating processing performed in the candidate evaluation function-generating part 102i1 will be described with reference to FIG. 18. FIG. 18 is a flowchart showing an example of the candidate evaluation function-generating processing performed in the candidate evaluation function-generating part 102i1.

The candidate evaluation function-generating part 102i1 first selects a desired method out of a plurality of different function-generating methods (including multivariate analysis methods such as principal component analysis, discriminant analysis, support vector machine, multi regression analysis, logistic regression analysis, k-means method, cluster analysis, and decision tree and the like) and determines the form of the candidate evaluation function to be generated based on the selected function-generating method (step SC-1).

The candidate evaluation function-generating part 102i1 then performs various calculation corresponding to the function-selecting method selected in step SC-1 (e.g., average or variance), based on the biological state information including the biological state indicator data and the metabolite concentration data designated in step SC-1 (step SC-2).

The candidate evaluation function-generating part 102i1 then determines the parameters for the calculation result in step SC-2 and the candidate evaluation function of which the form is determined in step SC-1 (step SC-3). In this way, a candidate evaluation function is generated, based on the selected function-generating method.

When candidate evaluation functions are generated simultaneously, concurrently (in parallel) by using a plurality of different function-generating methods in combination, the processings in steps SC-1 to SC-3 are to be executed concurrently for each selected function-generating method. Alternatively when candidate evaluation functions are to be generated in series by using a plurality of different function-generating methods in combination, for example, candidate evaluation functions may be generated by converting biological state information with a candidate evaluation function prepared by performing principal component analysis and performing discriminant analysis of the converted biological state information.

These are description of the candidate evaluation function-generating processing.

Back in FIG. 17 again, the candidate evaluation function-verifying part 102i2 verifies (mutually verifies) the candidate evaluation function prepared in step SB-5 according to a particular verification method and stores the verification result in a particular memory region of verification result file 106d2 (step SB-6). Specifically, the candidate evaluation function-verifying part 102i2 first generates the verification data to be used in verification of the candidate evaluation function, based on the designated biological state information, and verifies the candidate evaluation function according to the verification data.

Here in step SB-6, at least one of the discrimination rate, sensitivity, specificity, information criterion, and the like of the candidate evaluation function may be verified, based on at least one method of the bootstrap, holdout, leave-one-out, and other methods. Thus, it is possible to select a candidate evaluation function higher in predictability or reliability, based on the biological state information and the diagnostic condition.

If the candidate evaluation functions are generated by using a plurality of different function-generating methods in step SB-5, the candidate evaluation function-verifying part 102i2 verifies each candidate evaluation function corresponding to each function-generating method according to a particular verification method.

Then, the variable-selecting part 102i3 selects the combination of metabolite concentration data contained in the biological state information to be used in preparing the candidate evaluation function by selecting a variable of the candidate evaluation function from the verification results in step SB-6 according to a particular variable selection method, and stores the biological state information including the selected combination of metabolite concentration data in a particular memory region of the selected biological state information file 106d3 (step SB-7). The variable-selecting part 102i3 may select the combination of the metabolite concentration data, based on the designated biological state information.

Here in step SB-7, the variable of the candidate evaluation function may be selected from the verification results according to at least one of stepwise method, best path method, local search method, and genetic algorithm. The best path method is a method of selecting a variable by optimizing the evaluation index of the candidate evaluation function while eliminating the variables contained in the candidate evaluation function one by one.

When the candidate evaluation functions are generated by using a plurality of different function-generating methods in step SB-5 and each candidate evaluation function corresponding to each function-generating method is verified according to a particular verification method in step SB-6, the variable-selecting part 102i3 selects the variable of the candidate evaluation function for each candidate evaluation function corresponding to the verification result obtained in step SB-6, according to a particular variable selection method.

The evaluation function-generating part 102i then judges whether all combinations of the metabolite concentration data contained in the biological state information designated in step SB-4 are processed, and, if the judgment result is "End" (Yes in step SB-8), the processing advances to the next step (step SB-9), and if the judgment result is not "End" (No in step SB-8), it returns to step SB-5.

The evaluation function-generating part 102i judges whether the processing is preformed a predetermined number of times, and if the judgment result is "End" (Yes in step SB-8), the processing may advance to the next step (step SB-9), and if the judgment result is not "End" (No in step SB-8), it returns to step SB-5.

The evaluation function-generating part 102i may judge whether the combination of the metabolite concentration data selected in step SB-7 is the same as the combination of the metabolite concentration data designated in step SB-4 or the combination of the metabolite concentrations selected in the previous step SB-7, and if the judgment result is "the same" (Yes in step SB-8), the processing may advance to the next step (step SB-9) and if the judgment result is not "the same" (No in step SB-8), it returns to step SB-5.

If the verification result is specifically the evaluation value for each candidate evaluation function, the evaluation function-generating part 102i may advance to step SB-9 or return to SB-5, based on the comparison of the evaluation value with a particular threshold corresponding to each function-generating method.

The evaluation function-generating part 102i then generates (determines) an evaluation function based on the verification results by selecting a candidate evaluation function to be used as the evaluation function among the candidate evaluation functions, and stores the generated evaluation function (selected candidate evaluation function) in a particular memory region of the evaluation function file 106d4 (step SB-9). Here in step SB-9, for example, the optimal candidate evaluation function may be selected from the candidate evaluation functions prepared by the same function-generating method or from all candidate evaluation functions.

The biological state-evaluating part 102j then evaluates the biological state to be evaluated, based on the evaluation function generated in step SB-9 and the metabolite concentration data to be evaluated received and obtained from the client apparatus 200 in step SB-1, and stores the evaluation results in a particular memory region of the evaluation result file 106e (step SB-10). Specifically, an indicator of the biological state of an evaluation subject is calculated, by applying the metabolite concentration data of the evaluation subject to the generated evaluation function.

These are description of the biological state evaluation processing.

As described above, the biological state-evaluating apparatus 100 generates an evaluation function, based on the biological state information including metabolite concentration data and biological state indicator data, and evaluates the biological state to be evaluated, based on the generated evaluation function and the metabolite concentration data to be evaluated. Specifically, the biological state-evaluating apparatus 100 generates an evaluation function, based on the biological state indicator data (e.g., health state, disease name, and severity) obtained from healthy people and patients and the metabolite concentration data (e.g., blood amino acid concentration and blood biochemical concentration), and evaluates the biological state of the evaluation subject, based on the generated evaluation function from the metabolite concentration data of evaluation subjects. The candidate evaluation function-generating part 102i1 generates a candidate evaluation function that is a candidate of evaluation function from the biological state information according to a particular function-generating method; the candidate evaluation function-verifying part 102i2 verifies the prepared candidate evaluation function according to a particular verification method; the variable-selecting part 102i3 selects the combination of metabolite concentration data contained in the biological state information to be used in preparing the candidate evaluation function by selecting a variable of the candidate evaluation function from the verification results according to a particular variable selection method; and the evaluation function-generating part 102i generates an evaluation function by selecting a candidate evaluation function to be used as the evaluation function among the candidate evaluation functions, based on the verification results accumulated by repeated processing in the candidate evaluation function-generating part 102il, the candidate evaluation function-verifying part 102i2 and the variable-selecting part 102i3. In this way, it is possible to evaluate the biological state to be evaluated accurately by using a verified evaluation function. It is thus possible to generate an evaluation function for evaluation of the biological state to be evaluated as the optimal index and evaluate (predict) various phenomena concerning the biological state to be evaluated.

It is also possible to use the biological state-evaluating apparatus 100 for quantitative evaluation (monitoring) of the degree (severity) of chronic diseases (in particular, life-style diseases and others), among many diseases. It is thus possible to diagnose various diseases promptly by introducing the biological state-evaluating apparatus 100.

The biological state-evaluating apparatus 100 may also be used for quantitative evaluation (monitoring) of the effect and adverse effect of medicine. It is thus possible to conduct new drug development efficiently and consequently to reduce the development cost, by introducing the biological state-evaluating apparatus 100.

The biological state-evaluating apparatus 100 also allows diagnosis of acute diseases (e.g., viral diseases, cancer) and of whether a person is healthy or sick. Thus it is possible to perform qualitative evaluation of a particular disease.

The metabolite concentration data suitably used in the biological state-evaluating apparatus 100 are, for example, the "concentration of an amino acid, an amino acid analogue, an amino or imino group-containing compound in biological sample", the "concentration of a peptide, a protein, a sugar, a lipid, a vitamin, a mineral or the metabolite thereof in biological sample", and the like.

The biological state-evaluating apparatus 100 is used suitably in evaluation of the disease state of a patient with ulcerative colitis or Crohn's disease. The biological state-evaluating apparatus 100 is also applicable, in addition to ulcerative colitis and Crohn's disease, to the patients with metabolic diseases in which the concentration of a metabolite (in particular, amino acid) fluctuates according to the change in biological state. The present invention is also applicable to diseases such as malignant tumors (lung cancer, esophageal cancer, stomach cancer, colon cancer, hepatoma cancer, pancreatic cancer, gallbladder-cholangiocarcinoma, prostate cancer, breast cancer, uterine cancer, ovarian cancer, hematopoietic tumor, hypophysis cancer, and thyroid cancer), Basedow's disease, hyperlipemia, diabetes, collagen diseases (rheumatoid arthritis, nettle rash, and systemic erythematosus), osteoporosis, arteriosclerosis obliterans (ASO), angina pectoris, myocardial infarction, cardiomyopathy, heart failure, arrhythmia, cerebrovascular diseases (cerebral infarction and cerebral artery cancer), chronic liver diseases (chronic hepatitis B or C, and liver cirrhosis), acute hepatitis, fatty liver, cholelithiasis, cholecystitis, jaundice, edema, hypertension, glomerulonephritis, pyelonephritis, tubular diseases (Fanconi's syndrome and renal tubular acidosis), renal amyloidosis, toxic renal damage, gestosis, nephrosclerosis, renal failure, porphyria, methemoglobinemia, leukemia, pituitary gland diseases (anterior hypopituitarism, posterior hypopituitarism, and syndrome of inappropriate antidiuretic hormone secretion), thyroidal disease (hypothyroidism, hyperthyroidism, diffuse goiter, thyroiditis), hyperinsulinism, hyperglucagonemia, adrenocortical diseases (hyperadrenocorticalism, hypoadrenocorticism, hyperaldosteronism, and adrenogenital syndrome), hysteromyoma, endometriosis, urinary calculus, nephrotic syndrome, anaphylactic syndromes (drug eruption), keloid, atopic dermatitis, pollinosis, asthma, tuberculosis, interstitial pneumonia-pulmonary fibrosis, plumonary emphysema (COPD), cataract, glaucoma, febrile convulsion, epilepsy, periodontal disease, amyotrophic lateral sclerosis (ALS), inflammatory bowel diseases (ulcerative colitis and Crohn's disease), immunodeficiency diseases, acquired immunodeficiency syndromes (AIDS), infectious disease, gout, hyperuricemia, phenylketonuria, tyrosinuria, alcaptonuria, homocystinuria, maple syrup urine disease, renal amino acid urine, Niemann-Pick disease, Gaucher's disease, Tay-Sachs disease, mitochondrial encephalomyopathy, glycogenosis, galactosemia, Lesch-Nyhan syndrome, Wilson's disease, muscular dystrophy, hemophilia, duodenal ulcer, gastric ulcer, gastrisis, gastric polyp, gastric adenoma, Alzheimer's disease, Parkinson's disease, polio, and the like.

In addition to the embodiments above, the present invention may be modified in various different embodiments in the technological scope of the claims.

For example if an evaluation function generated is stored in the memory device 106 of the biological state-evaluating apparatus 100 or the memory device 406 of the database apparatus 400, the biological state to be evaluated may be evaluated in the biological state-evaluating part 102j, based on the metabolite concentration data to be evaluated and the evaluation function stored.

All or part of the processing performed automatically, among the processings described in the embodiments above, may be performed manually, and all or part of those performed manually may be performed automatically. In addition, the processing procedures, control procedure, typical name, information including various registered data and parameters such as retrieve condition, example of screen, and database configuration described above or shown in drawing may be modified arbitrarily, unless specified otherwise. For example, the components of the biological state-evaluating apparatus 100 shown in the figures are conceptual functionally and may not be the same physically as those shown in the figure. In addition, all or part of the operational function of each component and each device in the biological state-evaluating apparatus 100 (in particular, processings in the controlling device 102) may be executed by the CPU (Central Processing Part) or the programs executed by the CPU, and thus realized as wired-logic hardware.

The "program" is a data processing method written in any language or by any description method and may be in any format such as source code or binary code. The "program" may not be an independent program, and may be operated together with a plurality of modules and libraries or with a different program such as OS (Operating System). The program is stored on a recording medium and read mechanically as needed by the biological state-evaluating apparatus 100. Any well-known configuration or procedure may be used for reading the programs recorded on the recording medium in each apparatus and for retrieval of the procedure and installation of the procedure after reading.

The "recording media" include any "portable physical media", "fixed physical media", and "communication media". Examples of the "portable physical media" include flexible disk, magnetic optical disk, ROM, EPROM, EEPROM, CD-ROM, MO, DVD, and the like. Examples of the "fixed physical media" include various media installed in a computer system such as ROM, RAM, and HD. The "communication media" are, for example, media storing the program for a short period of time such as communication line and carrier wave when the program is transmitted via a network such as LAN, WAN, or the Internet.

Example 1

In Example 1, the results of evaluation of ulcerative colitis by using a biological state-evaluating system in the embodiment above and those by a doctor will be described. The metabolite described in Example 1 is amino acid, but the metabolite is not limited thereto, and the results are applicable to any metabolite.

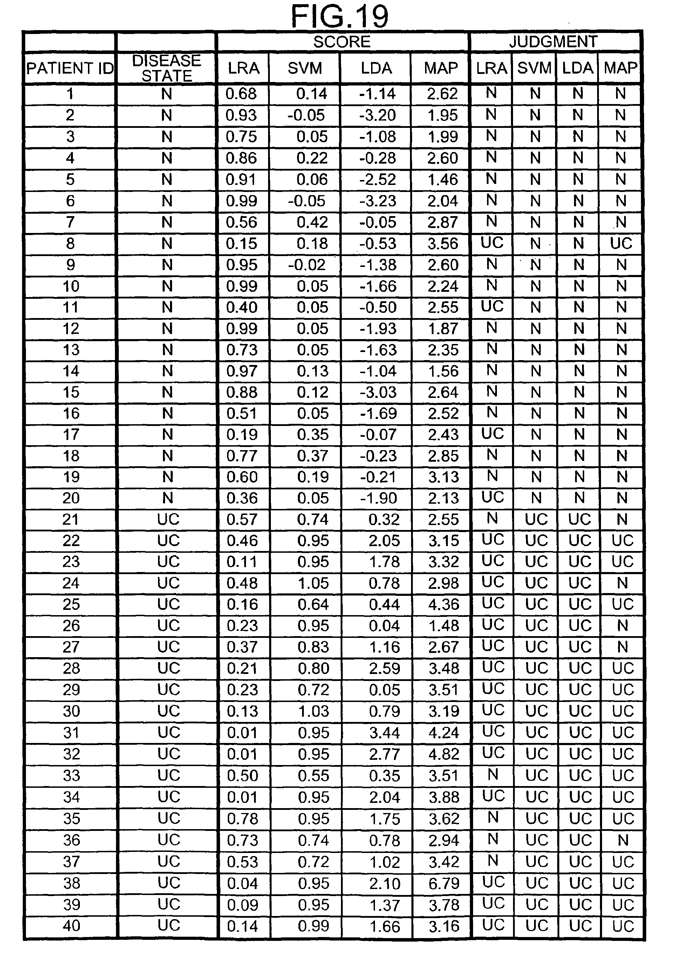

First by using the biological state-evaluating system in the embodiment above, a model (evaluation function in the embodiment above) for discriminating (evaluating) Slovakian healthy peoples (N: 20) from Slovakian ulcerative colitis patients (UC: 20) was generated. A model for discriminating healthy people from ulcerative colitis patients will be described in Example 1, but the subject of the present invention is not limited to the ulcerative colitis patients. FIG. 19 depicts the relationship among the disease states of healthy people and also ulcerative colitis patients, the scores obtained by logistic regression analysis (LRA), support vector machine (SVM), discriminant analysis (LDA) and the method described in WO 2004/052191 (hereinafter, referred to as MAP method), and the disease states predicted from the scores. In the logistic regression analysis (LRA), subjects having a score of 0.5 or more were regarded healthy, and those having a score of less than 0.5, patients with ulcerative colitis. In the support vector machine (SVM), subjects having a score of less than 0.5 were regarded healthy and those having a score of 0.5 or more, patients with ulcerative colitis. Alternatively in the discriminant analysis (LDA), subjects having a score of less than 0 was regarded healthy and those having a score of 0 or more, patients with ulcerative colitis. Further in the MAP method, subjects having a score of less than 3.14 were regarded healthy and those having a score of 3.14 or more, patients with ulcerative colitis.

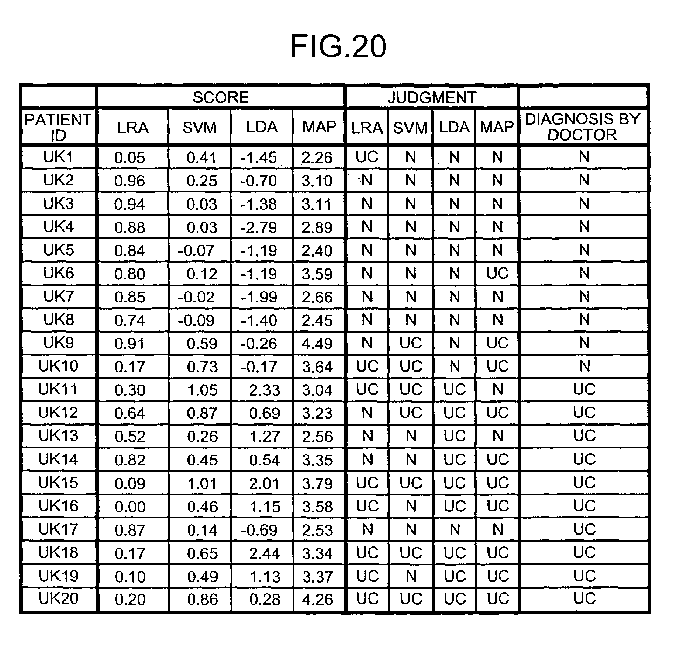

An additional group of subjects were studied (evaluated) by the models prepared and also by a doctor about their ulcerative colitis. FIG. 20 depicts the relationship among the scores obtained by the models prepared, the disease states predicted from the score, and the diagnosis result (disease state) by a doctor of the newly added subjects. Here in FIG. 20, the criteria of determining the disease state from the score is the same as that in FIG. 19.

As shown in FIG. 19, by the logistic regression analysis (LRA) and the MAP method, it was not possible to determine the disease state of part of the healthy people and ulcerative colitis patients whose disease states were already known, although the analysis was optimized. On the other hand, by the support vector machine (SVM) and discriminant analysis (LDA), it was possible to correctly determine the disease state of the all of the healthy people and ulcerative colitis patients whose disease states were already known. In addition, newly added subjects were examined by using the prepared models, and the number of the newly added subjects whose result was different from the actual diagnosis by a doctor was 7 by support vector machine (SVM), while 1 by discriminant analysis (LDA), as shown in FIG. 20. It was 5 by the MAP method, and 6 by the logistic regression analysis (LRA). The difference above in evaluation results is caused by the difference in discrimination standard allocated to each analytical method. However, in the case of ulcerative colitis, it is most desirable to evaluate by using the model prepared by discriminant analysis (LDA), and it was possible to evaluate the disease state of newly added subjects at a high probability of 95% by using the biological state-evaluating system in the embodiment above. In this way, it would be possible to perform more accurate and precise quantitative evaluation by using a plurality of analytical methods in combination than by using the MAP method alone.

Example 2

In Example 2, the results of evaluation of Crohn's disease by using a biological state-evaluating system in the embodiment above and those by a doctor will be described. The metabolite described in Example 2 was amino acid, but the metabolite is not limited thereto, and the results are applicable to any metabolite.

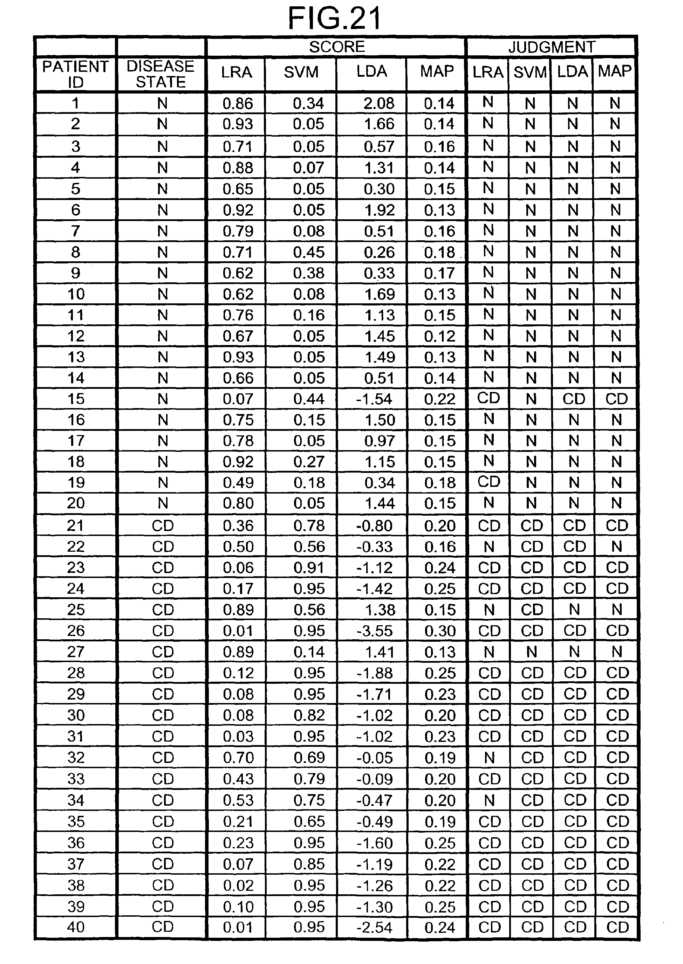

First by using the biological state-evaluating system in the embodiment above, a model (evaluation function in the embodiment above) for discriminating (evaluating) Slovakian healthy peoples (N: 20) from Slovakian ulcerative colitis patients (CD: 20) was generated. A model for discriminating healthy people from Crohn's disease patients will be described in Example 2, but the subject of the present invention is not limited to the Crohn's disease patients. FIG. 21 depicts the relationship among the disease states respectively of healthy people and Crohn's disease patients, the scores obtained in logistic regression analysis (LRA), support vector machine (SVM), discriminant analysis (LDA) and MAP method, and the disease states predicted from the scores. In the logistic regression analysis (LRA), subjects having a score of 0.5 or more were regarded healthy, and those having a score of less than 0.5, patients with Crohn's disease. In the support vector machine (SVM), subjects having a score of less than 0.5 were regarded healthy and those having a score of 0.5 or more, patients with Crohn's disease. In the discriminant analysis (LDA), subjects having a score of less than 0 were regarded healthy and those having a score of 0 or more, patients with Crohn's disease. Further in the MAP method, subjects having a score of less than 0.18 were regarded healthy and those having a score of 0.18 or more, patients with Crohn's disease.

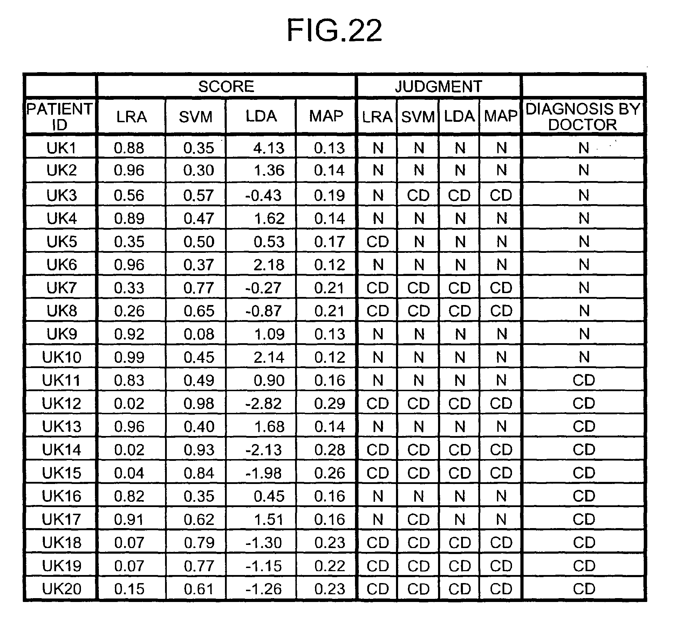

An additional group of subjects were studied (evaluated) by the model prepared and also by a doctor, about their Crohn's disease. FIG. 22 depicts the relationship among the scores obtained by the models prepared, the disease states predicted from the score, and the diagnosis result (disease state) by a doctor of the newly added subjects. Here in FIG. 22, the criteria of determining the disease state from the score is the same as that in FIG. 21.

As shown in FIG. 21, it was not possible by any analytical method to determine the disease state of part of the healthy people and Crohn's disease patients whose disease states were already known, although the analysis was optimized. One reason for the results above seems to be that the change in amino acid concentration by Crohn's disease is smaller. In addition, newly added subjects were examined by using the prepared models, and the number of the newly added subjects whose prediction was different from the actual diagnosis by a doctor was 7 by the logistic regression analysis (LRA), 6 by the support vector machine (SVM), 7 by the MAP method, and 7 by the discriminant analysis (LDA) as shown in FIG. 22. The results indicate that, although the evaluation ability is not so high at about 70% by any analytical method, it is probably because the change in amino acid concentration caused by the Crohn's disease is smaller, as described above. However, considering the fact that the number of the subjects used in constructing the model was 40 and the number of the data evaluated was 20, an evaluation ability of approximately 70% may be considered relatively high. It is because increase in the number of the subjects used in constructing a model likely leads to increase in the evaluation ability. Thus, it would be possible to perform more accurate and precise quantitative evaluation by using a plurality of analytical methods in combination than by using the MAP method alone.

Example 3

In Example 3, the results of evaluation of diabetic rats and healthy rats by using the biological state-evaluating system in the embodiment above will be described. The metabolite described in Example 3 was amino acid, but the metabolite is not limited thereto, and the results are applicable to any metabolite.

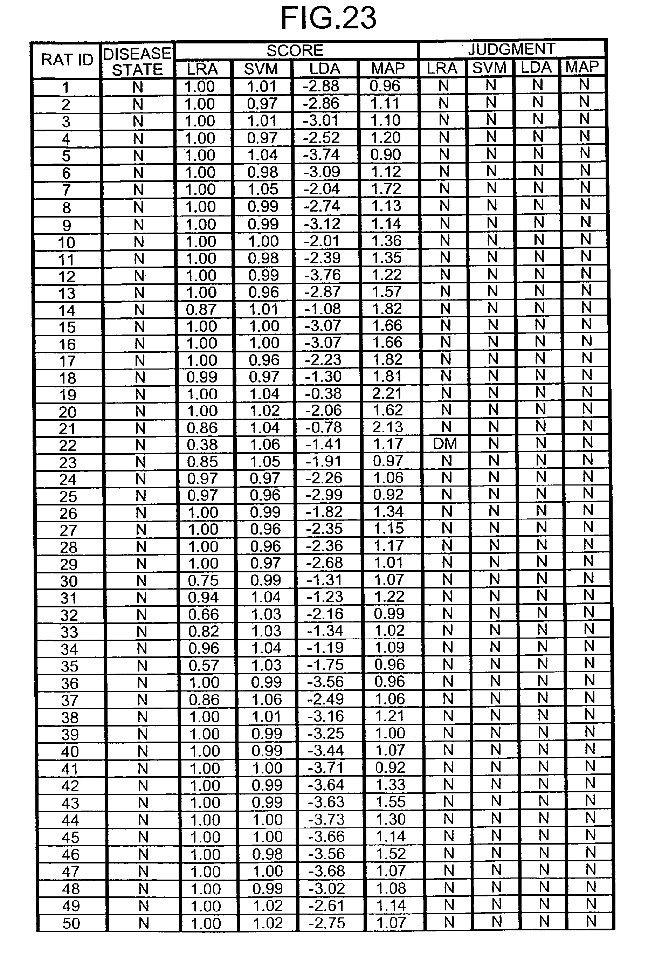

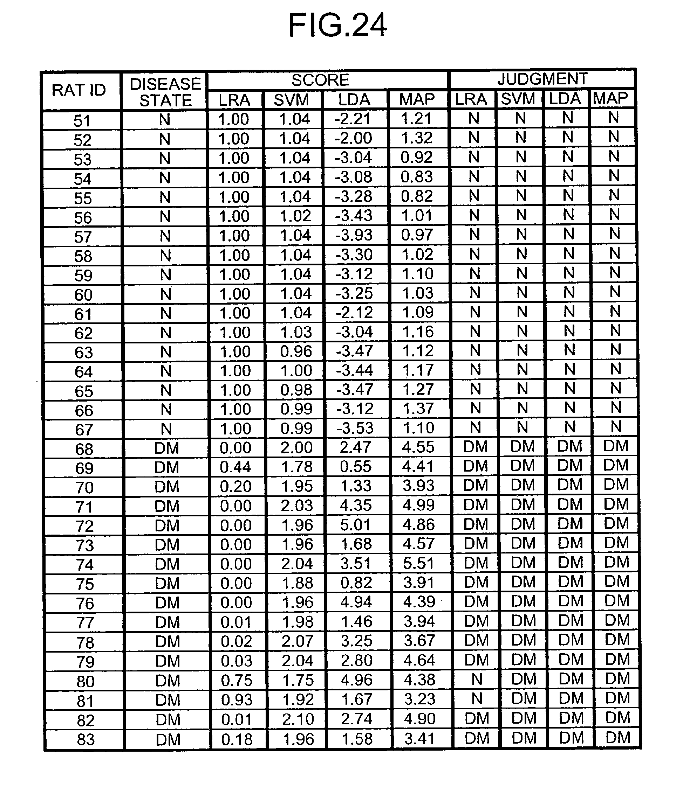

First, a model (evaluation function in the embodiment above) for discriminating (evaluating) healthy rat (N: 67) from diabetic rat (DM: 16) was generated by using the biological state-evaluating system in the embodiment above. A model for discrimination of healthy rats from diabetic rats will be described in Example 3, but the subject of the present invention is not limited to the diabetic rats. FIGS. 23 and 24 depict the relationship among the disease states respectively of healthy rats and diabetic rats, the scores obtained in logistic regression analysis (LRA), support vector machine (SVM), discriminant analysis (LDA) and MAP method, and the disease states predicted from the scores. In the logistic regression analysis (LRA), rats having a score of 0.5 or more were regarded healthy, and those having a score of less than 0.5, rats with diabetes. In the support vector machine (SVM), rats having a score of 1.5 or more were regarded healthy and those having a score of less than 1.5, rats with diabetes. In the discriminant analysis (LDA), rats having a score of 0 or more were regarded healthy and those having a score of less than 0, rats with diabetes. Further in the MAP method, rats having a score of less than 2.25 were regarded healthy and those having a score of 2.25 or more, rats with diabetes.

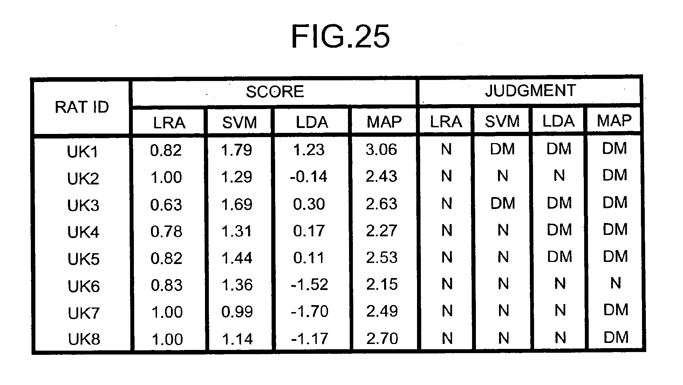

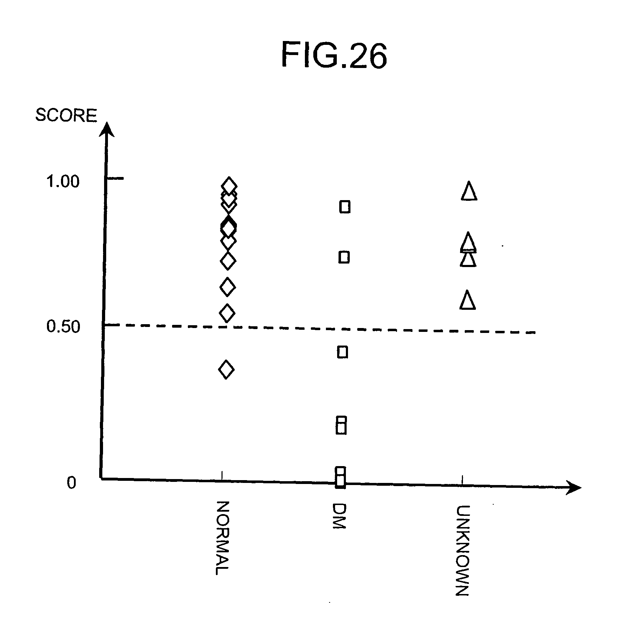

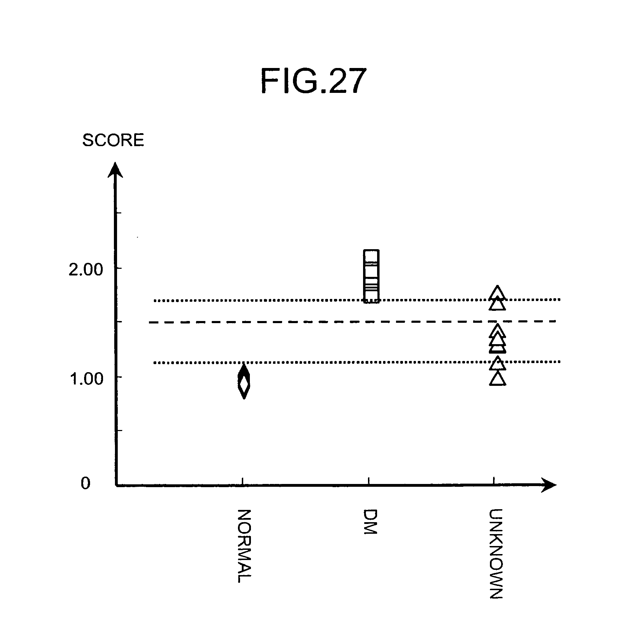

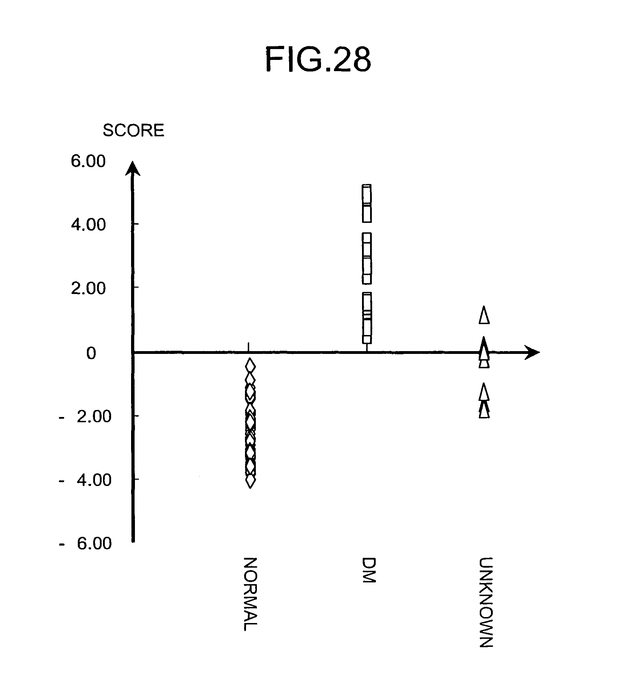

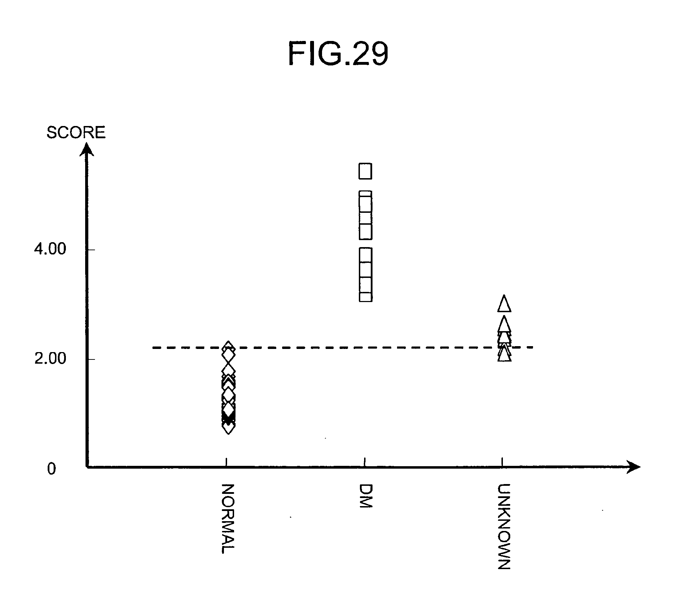

Then, the health state of insulin-administered and treated diabetic rats were examined (evaluated) by using the prepared model. FIG. 25 depicts the relationship in diabetic rats after insulin administration between the scores calculated by using respective models prepared and the disease states predicted from the scores. Here in FIG. 25, the criteria of determining the disease state from the score is the same as that in FIGS. 23 and 24. In FIG. 26, the scores evaluated by the logistic regression analysis (LRA) are plotted for respective groups of healthy rats (Normal), diabetic rats (DM), insulin-administered and treated diabetic rats (Unknown). Also in FIG. 27, the scores evaluated by the support vector machine (SVM) are plotted for respective groups of healthy rats (Normal), diabetic rats (DM), insulin-administered and treated diabetic rats (Unknown). Also in FIG. 28, the scores evaluated by the discriminant analysis (LDA) are plotted for respective groups of healthy rats (Normal), diabetic rats (DM), insulin-administered and treated diabetic rats (Unknown). Also in FIG. 29, the scores evaluated by the MAP method are plotted for respective groups of healthy rats (Normal), diabetic rats (DM), insulin-administered and treated diabetic rats (Unknown).

As shown in FIGS. 23 and 24, the logistic regression analysis (LRA), although optimized, could not determine the disease state of part of the healthy rats and diabetic rats whose disease states were already known. Thus, the results by the logistic regression analysis in Example 3 were seemingly lower in validity. On the other hand, it was possible to determine the disease state of all of the healthy rats and the diabetic rats with known disease state correctly by the support vector machine (SVM), discriminant analysis (LDA) or MAP method. Then as shown in FIG. 25, the diabetic rats after insulin administration were evaluated by using the prepared models, and the support vector machine (SVM) diagnosed that rats UK1 and UK3 were diabetic, while the discriminant analysis (LDA) diagnosed that rats UK1, UK3, UK4, and UK5 were diabetic. Alternatively, the MAP method judged that all rats except UK6 were diabetic. The difference above in evaluation results seems to come from the difference in discrimination criteria allocated to each analytical method.

As for the scores of diabetic rat by support vector machine (SVM) and discriminant analysis (LDA) after insulin administration, for example, rat UK1 was diagnosed as diabetic by both analytical methods, and the score of support vector machine (SVM) and the score of discriminant analysis (LDA) were also greater than the thresholds of discrimination criteria. On the other hand, the scores of support vector machine (SVM) for the rats UK4 and UK5, which were determined to be diabetic by discriminant analysis (LDA), are closer to the threshold of discrimination criteria than those of the other healthy rats, although it is in the healthy region. Thus, the results seem to indicate that the rats UK1 and UK3, although treated with insulin, were not recovered with lower treatment effect, compared to other rats. Judging from the scores of both analytical methods, it seems that the rats UK4 and UK5 are healthier than the rats UK1 and UK3, but are not well recovered by insulin treatment than the other rats. Thus, it would be possible to perform more accurate and precise quantitative evaluation by using a plurality of analytical methods in combination than by using the MAP method alone.

Example 4

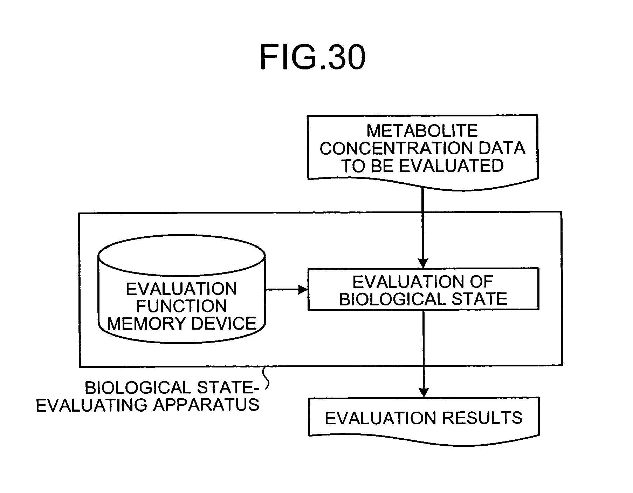

In Example 4, the results of evaluating ulcerative colitis (UC) by using a biological state-evaluating apparatus storing a previously obtained evaluation function in an evaluation function memory part and evaluating the biological state to be evaluated, based on the stored evaluation function and the previously acquired metabolite concentration data to be evaluated in the biological state-evaluating part (see FIG. 30), and also the results of diagnosis of ulcerative colitis by a doctor will be described. The metabolite described in Example 4 was amino acid, but the metabolite is not limited thereto, and the results are applicable to any metabolite.

First, the biological state of healthy people (N: 30) and ulcerative colitis patients (UC: 30) was evaluated by using the biological state-evaluating apparatus. The biological state-evaluating apparatus has the following evaluation functions 1 to 3 previously installed. The evaluation function used in evaluating the biological state of ulcerative colitis patients is not limited to those described below.

(Evaluation function 1)

$$-0.80 \times Asn - 0.89 \times Asp - 0.73 \times Orn - 1.01 \times Trp - 0.16 \times Ser - 0.05 \times Thr + 1.15 \times AAB + 0.04 \times Gly + 0.55 \times Cit - 0.55 \times Met + 0.21 \times Tyr + 0.02 \times Gln + 0.14 \times Ile + 0.71 \times Phe + 52.0$$

(Evaluation function 2)

$$1/[1 + \exp(-1169.5 + 17.18 \times Tau + 17.5 \times Asn - 10.15 \times Cit - 26.5 \times AAB - 5.16 \times Leu - 12.85 \times Phe + 15.37 \times Trp + 19.81 \times Orn)]$$

(Evaluation function 3)

[Formula 6]

$$[K|x_K = \min\{x_1, x_2\}](K = 1,2)$$

where,

$$x_1 = \left(\vec{x} - \vec{x_1}\right) X_1^{-1} \left(\vec{x} - \vec{x_1}\right)^t, \quad x_2 = \left(\vec{x} - \vec{x_2}\right) X_2^{-1} \left(\vec{x} - \vec{x_2}\right)^t$$

$$\vec{x} = \begin{pmatrix} Asn & Asp & Orn & Trp & Ser & AAB & Cit & His & Tyr & Arg \end{pmatrix}$$

$$\vec{x_1} = \begin{pmatrix} 41.0 & 24.2 & 50.8 & 47.4 & 119.0 & 13.9 & 27.0 & 74.4 & 60.0 & 75.9 \end{pmatrix}$$

$$\vec{x_2} = \begin{pmatrix} 30.7 & 18.4 & 40.5 & 41.2 & 100.9 & 16.6 & 24.6 & 71.9 & 56.1 & 75.4 \end{pmatrix}$$

[Formula 7]

$$X_1 = \begin{pmatrix} 120.0 & -11.7 & -61.8 & -16.6 & 145.3 & 7.3 & -25.5 & 26.1 & 137.5 & -40.3 \\ -11.7 & 112.8 & 37.6 & -28.0 & 205.7 & 9.9 & 60.2 & 23.8 & 112.2 & 89.4 \\ -61.8 & 37.6 & 304.2 & 63.6 & -84.9 & 9.6 & 157.3 & 62.9 & -63.3 & 212.2 \\ -16.6 & -28.0 & 63.6 & 93.3 & -129.6 & 24.0 & 13.1 & 20.1 & -81.2 & 51.3 \\ 145.3 & 205.7 & -84.9 & -129.6 & 1950.8 & 30.6 & -5.4 & 62.2 & 1246.1 & 108.3 \\ 7.3 & 9.9 & 9.6 & 24.0 & 30.6 & 49.3 & 2.0 & 11.1 & -41.0 & 69.0 \\ -25.5 & 60.2 & 157.3 & 13.1 & -5.4 & 2.0 & 153.8 & 53.5 & -15.6 & 116.9 \\ 26.1 & 23.8 & 62.9 & 20.1 & 62.2 & 11.1 & 53.5 & 109.84 & 84.6 & 51.6 \\ 137.5 & 112.2 & -63.3 & -81.2 & 1246.1 & -41.0 & -15.6 & 84.6 & 1450.0 & -144.1 \\ -40.3 & 89.4 & 212.2 & 51.3 & 108.3 & 69.0 & 116.9 & 51.6 & -144.1 & 484.9 \end{pmatrix}$$

$$X_t = \begin{pmatrix} 89.2 & 7.9 & 4.2 & -3.8 & 12.0 & 10.7 & -6.9 & 24.3 & -1.5 & 52.3 \\ 7.9 & 10.8 & -0.23 & 3.8 & 16.5 & 1.5 & -0.29 & 12.3 & 2.6 & 3.9 \\ 4.2 & -0.23 & 115.0 & -3.1 & -19.7 & -4.9 & 41.4 & 18.6 & 66.0 & 128.9 \\ -3.8 & 3.8 & -3.1 & 129.4 & 3.5 & 31.3 & 37.9 & 45.4 & 69.1 & 35.1 \\ 12.0 & 16.5 & -19.7 & 3.5 & 388.2 & -15.1 & 21.2 & 94.1 & 72.7 & 27.5 \\ 10.7 & 1.5 & -4.9 & 31.3 & -15.1 & 53.3 & -8.0 & 25.4 & 0.56 & -2.4 \\ -6.9 & -0.29 & 41.4 & 37.9 & 21.2 & -8.0 & 75.6 & 52.9 & 57.7 & 86.5 \\ 24.3 & 12.3 & 18.6 & 45.4 & 94.1 & 25.4 & 52.9 & 190.4 & 118.3 & 91.8 \\ -1.5 & 2.6 & 66.0 & 69.1 & 72.7 & 0.56 & 57.7 & 118.3 & 296.9 & 105.2 \\ 52.3 & 3.9 & 128.9 & 35.1 & 27.5 & -2.4 & 86.5 & 91.8 & 105.2 & 364.4 \end{pmatrix}$$

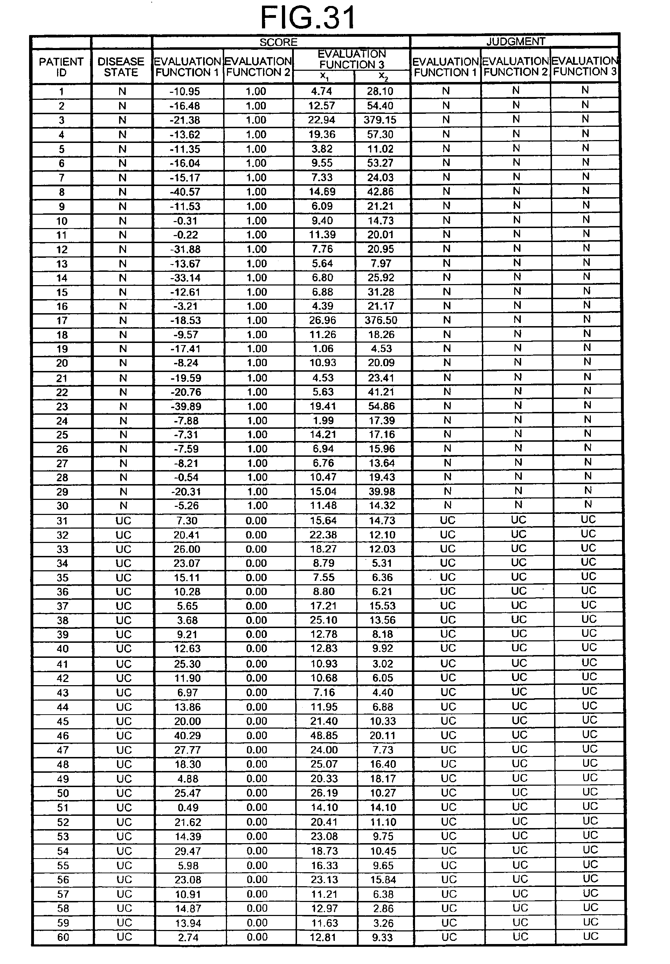

FIG. 31 is a table showing the relationship among the disease states of healthy people and ulcerative colitis patients, the scores as determined by the evaluation functions 1 to 3, and the disease states predicted from the scores. Of the evaluation function 1, subjects having a score of less than 0 were regarded healthy, while subjects having a score of 0 or more, with ulcerative colitis. Of the evaluation function 2, subjects having a score of 0.5 or more were regarded healthy, while subjects having a score of less than 0.5 with ulcerative colitis. Of the evaluation function 3, subjects satisfying "$X_1 < X_2$" were regarded healthy, while subjects satisfying "$X_1 > X_2$" with ulcerative colitis.

As shown in FIG. 31, the evaluation functions 1, 2, and 3 determined the disease state of all patients correctly. Thus, it is possible to obtain results similar to those diagnosed by a doctor by using an endoscope, by using the present biological state-evaluating system employing the evaluation functions 1 to 3. In other words, it was possible to evaluate whether a subject is with ulcerative colitis without use of an endoscope at an accuracy equivalent to the diagnosis of a doctor, only by inputting the amino acid concentration of a subject suspected to be with ulcerative colitis into the present biological state-evaluating apparatus.

Example 5

Results of evaluation of Crohn's disease (CD) by using the biological state-evaluating apparatus shown in FIG. 30 in Example 5, and also results of diagnosis of Crohn's disease by a doctor will be described. The metabolite described in Example 5 was amino acid, but the metabolite is not limited thereto, and the results are applicable to any metabolite.

First, the biological state of healthy people (N: 30) and Crohn's disease patients (CD: 30) was evaluated by using the biological state-evaluating apparatus. The biological state-evaluating apparatus has the following evaluation functions 1 to 3 previously installed. The evaluation function used in evaluating the biological state of Crohn's disease patients is not limited to those described below.

(Evaluation function 1)

$$-0.79 \times Phe + 0.49 \times Asn + 0.05 \times Ser + 0.12 \times Cit - 0.09 \times Thr - 0.02 \times Gln + 0.01 \times Leu + 0.23 \times Asp - 0.0002 \times Ala + 0.34 \times AAB + 0.23 \times Orn - 0.01 \times Tyr + 0.23 \times His + 0.30 \times Ile + 0.01 \times Glu + 0.32 \times Met + 0.03 \times Lys - 0.001 \times Gly + 0.10 \times Val$$

(Evaluation function 2)

$$1/[1 + \exp(-4.52 + 0.218 \times Asp + 0.151 \times Asn + 0.060 \times Glu - 0.010 \times Gln + 0.094 \times Cit + 0.161 \times AAB - 0.281 \times Phe + 0.157 \times Trp + 0.019 \times Lys + 0.059 \times His - 0.069 \times Arg)]$$

(Evaluation function 3)

[Formula 8]

$$\left[ K \middle| x_K = \min\{x_1, x_2\} \right] (K = 1,2)$$

where,

$$x_1 = \left( \vec{x} - \vec{x_1} \right) X_1^{-1} \left( \vec{x} - \vec{x_1} \right)^t, \quad x_2 = \left( \vec{x} - \vec{x_2} \right) X_2^{-1} \left( \vec{x} - \vec{x_2} \right)^t$$

$$\vec{x} = \left( Phe \quad Cit \quad Thr \quad Gln \quad Leu \quad Asp \quad AAB \quad Orn \quad Glu \quad Lys \right)$$

$$\vec{x_1} = \left( 52.5 \quad 27.0 \quad 144.5 \quad 650.2 \quad 105.6 \quad 24.2 \quad 13.9 \quad 50.8 \quad 49.7 \quad 166.4 \right)$$

$$\vec{x_2} = \left( 67.1 \quad 22.0 \quad 163.6 \quad 617.1 \quad 96.2 \quad 21.5 \quad 12.2 \quad 47.1 \quad 46.5 \quad 171.7 \right)$$

[Formula 9]

$$X_1 = \begin{pmatrix} 155.0 & 29.2 & 260.5 & -104.4 & 217.4 & 12.6 & 28.9 & 126.4 & 181.5 & 282.0 \\ 29.2 & 153.8 & 46.7 & 94.4 & 194.9 & 60.2 & 2.0 & 157.3 & 12.0 & 147.5 \\ 260.5 & 46.7 & 1851.4 & 371.1 & 586.0 & 71.4 & 122.0 & 174.2 & 516.4 & 610.8 \\ -104.4 & 94.4 & 371.1 & 8523.2 & 600.5 & 231.0 & 23.7 & 167.0 & -20.6 & 376.0 \\ 217.4 & 194.9 & 586.0 & 600.5 & 1072.4 & 83.3 & 123.8 & 263.0 & 328.6 & 781.1 \\ 12.6 & 60.2 & 71.4 & 231.0 & 83.3 & 112.8 & 9.9 & 37.6 & 29.5 & 66.1 \\ 28.9 & 2.0 & 122.0 & 23.7 & 123.8 & 9.9 & 49.3 & 9.6 & 33.0 & 86.2 \\ 126.4 & 157.3 & 174.2 & 167.0 & 263.0 & 37.6 & 9.6 & 304.2 & 171.6 & 316.1 \\ 181.5 & 12.0 & 516.4 & -20.6 & 328.6 & 29.5 & 33.0 & 171.6 & 656.7 & 536.8 \\ 282.0 & 147.5 & 610.8 & 376.0 & 781.1 & 696.1 & 86.2 & 316.1 & 536.8 & 1486.6 \end{pmatrix}$$

$$X_2 = \begin{pmatrix} 300.8 & 65.2 & 167.4 & -25.8 & 189.3 & 41.2 & 18.9 & -22.2 & 156.2 & 583.6 \\ 65.2 & 89.7 & 153.6 & 516.1 & 19.9 & 34.1 & 17.4 & 24.6 & 117.8 & 151.4 \\ 167.4 & 153.6 & 2517.3 & 2480.6 & 245.8 & 131.1 & 43.8 & 59.8 & 213.4 & 1801.9 \\ -25.8 & 516.1 & 2480.6 & 11836.8 & 993.9 & 282.9 & 198.1 & 385.8 & 535.7 & 2033.8 \\ 189.3 & 19.9 & 245.8 & 993.9 & 675.9 & 34.9 & 28.4 & 72.9 & 67.5 & 1130.6 \\ 41.2 & 34.1 & 131.1 & 282.9 & 34.9 & 39.8 & -0.95 & 30.7 & 58.9 & 117.9 \\ 18.9 & 17.4 & 43.8 & 198.1 & 28.4 & -0.95 & 33.9 & -20.8 & 33.0 & -8.9 \\ -22.2 & 24.6 & 59.8 & 385.8 & 72.9 & 30.7 & -20.8 & 214.8 & 65.4 & 128.3 \\ 156.2 & 117.8 & 213.4 & 535.7 & 67.5 & 58.9 & 33.0 & 65.4 & 447.7 & 592.4 \\ 583.6 & 151.4 & 1801.9 & 2033.8 & 1130.6 & 117.9 & -8.9 & 128.3 & 592.4 & 5791.8 \end{pmatrix}$$

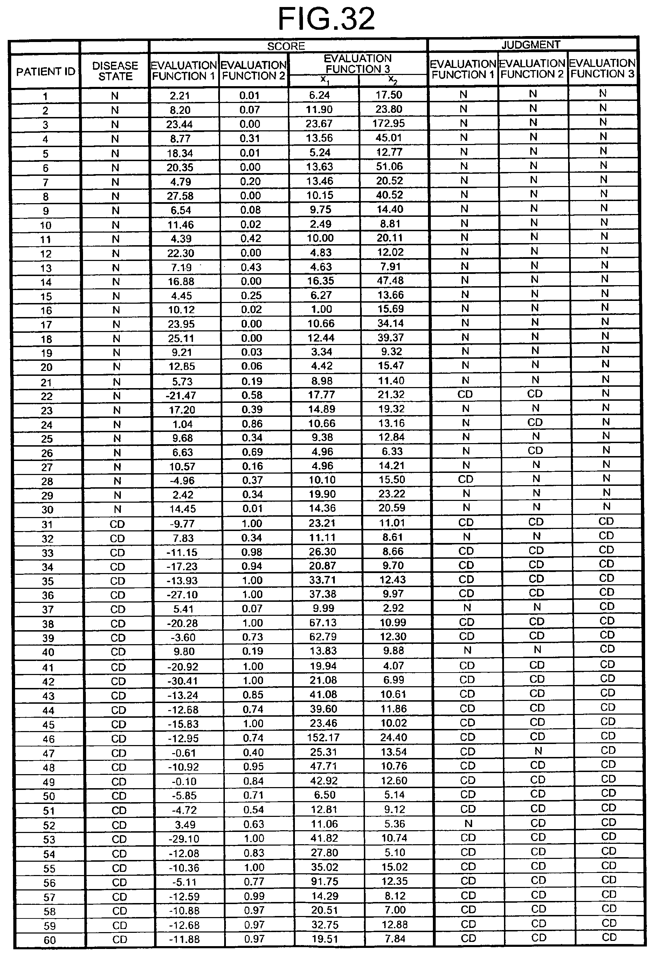

FIG. 32 is a table showing the relationship among the disease states of healthy people and Crohn's disease patients, the scores as determined by the evaluation functions 1 to 3, and the disease states predicted from the scores. Of the evaluation function 1, subjects having a score of 0 or more were regarded healthy, while subjects having a score of less than 0, with Crohn's disease. Of the evaluation function 2, subjects having a score of less than 0.5 were regarded healthy, while subjects having a score of 0.5 or more, with Crohn's disease. Of the evaluation function 3, subjects satisfying "$X_1 < X_2$" were regarded healthy, while subjects satisfying "$X_1 > X_2$" with Crohn's disease.

As shown in FIG. 32, the evaluation function 1 determined the patient disease state correctly at a rate of 90%, the evaluation function 2 at a rate of 88.3%, and the evaluation function 3 at a rate of 100%. It was possible to evaluate whether a subject is with Crohn's disease without use of an endoscope at higher accuracy, only by inputting the amino acid concentration of a subject suspected to be with Crohn's disease into the present biological state-evaluating apparatus similarly to the case of ulcerative colitis, although the prediction accuracy was slightly lower than that for ulcerative colitis.

Example 6

In Example 6, results of evaluation of asthma by using the biological state-evaluating apparatus shown in FIG. 30 will be described. The metabolite described in Example 6 was amino acid, but the metabolite is not limited thereto, and the results are applicable to any metabolite.

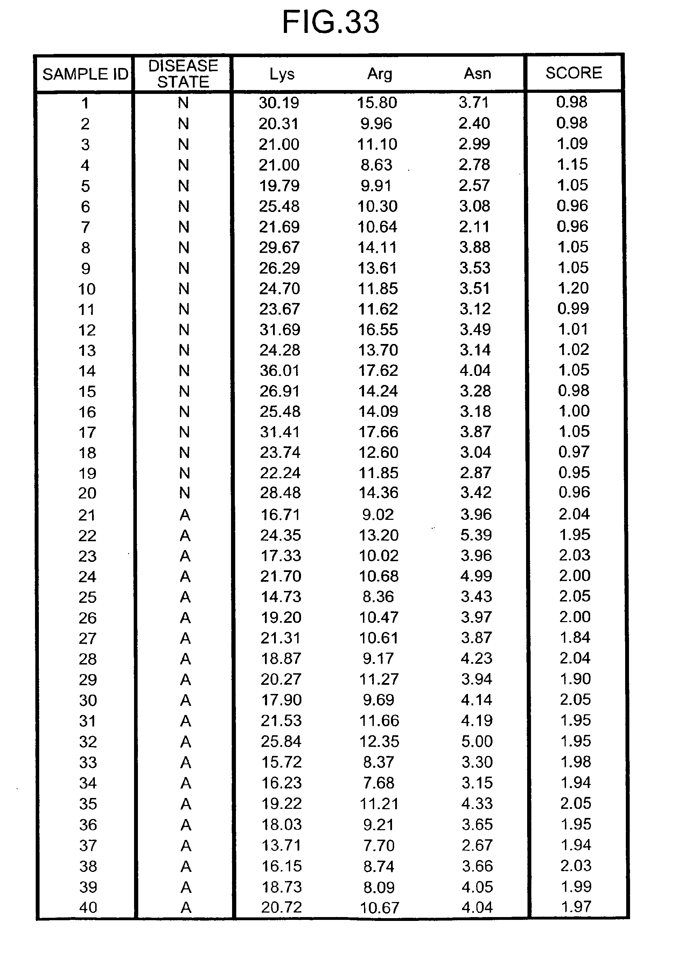

First, the biological state of healthy mice (N: 10) and asthma model mice (A: 10) was evaluated by using the biological state-evaluating apparatus. The biological state-evaluating system is assumed to have the training model (corresponding to an evaluation function) trained by the support vector machine, based on the data in FIG. 33. The kernel function used in training by the support vector machine was radial basis function. The "score" in FIG. 33 is a value obtained by evaluating the trained data by the training model prepared. The evaluation function for use in evaluating the biological state of asthma model mice and also of asthma patients is not limited to the training model.

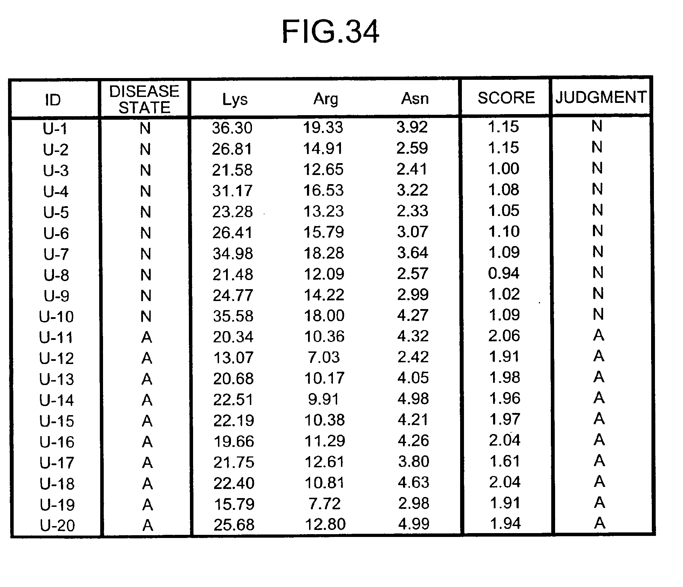

FIG. 34 is a table showing the relationship among the disease states of healthy mice and asthma model mice, blood amino acid (Lys, Arg and Asn) concentrations, the scores evaluated by the prepared training model, and the disease states predicted from the scores. Mice having a score of less than 1.5 were regarded healthy, while mice having a score of 1.5 or more, with asthma.

As shown in FIG. 34, the training model determined the disease state of all mice correctly. Asthma is diagnosed subjectively by a doctor in the current medical settings, but it is possible to evaluate asthma accurately with an objective indicator of body metabolite concentration, by inputting the amino acid concentrations of asthma-suspected patient to the present biological state-evaluating apparatus.

Example 7

In Example 7, results of rheumatism evaluation by using the biological state-evaluating apparatus shown in FIG. 30 will be described. The metabolite described in Example 7 was amino acid, but the metabolite is not limited thereto, and the results are applicable to any metabolite.

First, the biological state of healthy mice (N: 27) and rheumatoid mice (R: 27) was evaluated by using the biological state-evaluation. The biological state evaluation is assumed to have the following evaluation functions previously installed. The evaluation function used in evaluating the biological state of the rheumatoid mice and rheumatoid patients is not limited to those described below.

(Evaluation function 1)

$$-2.51 \times Asp + 0.866 \times Cys$$

(Evaluation function 2)

$$1/[1 + \exp(-23.3 - 16.3 \times Cys + 53.8 \times Asp)]$$

(Evaluation function 3)

[Formula 10]

$$[K \mid x_k = \min\{x_1, x_2\}](K = 1,2)$$

where,

$$x_1 = \left(\vec{x} - \vec{x_1}\right) X_1^{-1} \left(\vec{x} - \vec{x_1}\right)^t, \; x_2 = \left(\vec{x} - \vec{x_2}\right) X_2^{-1} \left(\vec{x} - \vec{x_2}\right)$$

$$\vec{x} = \left(Asp \; Gln\right)$$

$$\vec{x_1} = \left(2.31 \; 62.6\right)$$

$$\vec{x_2} = \left(0.817 \; 42.8\right)$$

$$X_1 = \begin{pmatrix} 0.262 & 1.72 \\ 1.72 & 67.8 \end{pmatrix}$$

$$X_2 = \begin{pmatrix} 0.058 & 0.916 \\ 0.916 & 39.4 \end{pmatrix}$$

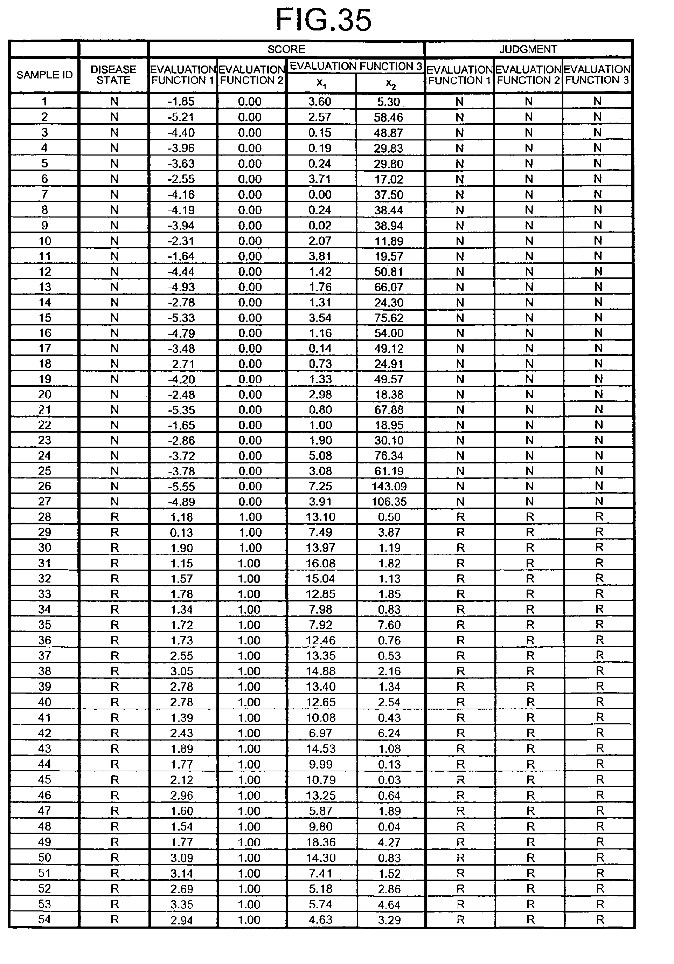

FIG. 35 is a table showing the relationship among the disease states of healthy mice and rheumatoid mice, the scores obtained by evaluation functions 1 to 3, and the disease states predicted from the scores. Of the evaluation function 1, mice having a score of less than 0 were regarded healthy, while mice having a score of 0 or more, with rheumatism. Of the evaluation function 2, mice having a score of less than 0.5 were regarded healthy, while mice having a score of 0.5 or more, with rheumatism. Of the evaluation function 3, mice satisfying "$X_1 < X_2$" were regarded healthy, while mice satisfying "$X_1 > X_2$" with rheumatism.

As shown in FIG. 35, the evaluation functions 1, 2, and 3 determined the disease state of all patients correctly. Rheumatism is diagnosed subjectively by a doctor in the current medical settings, but it is possible to evaluate rheumatism accurately with an objective indicator of body metabolite concentration by inputting the amino acid concentrations of asthma-suspected patient to the present biological state-evaluating apparatus.

INDUSTRIAL APPLICABILITY

As described above, the biological state-evaluating apparatus, the biological state-evaluating method, the biological state-evaluating system, the biological state-evaluating program, the evaluation function-generating apparatus, the evaluation function-generating method, the evaluation function-generating program and the recording medium according to the present invention may be practiced widely in many industrial fields, in particular in pharmaceutical, food, cosmetics, and medical fields, and are extremely useful in the field of bioinformatics, for example, performing disease state prediction, disease risk prediction, and proteome or metabolome analysis.

**Claims**

1. A biological state-evaluating apparatus, comprising:

    an evaluation function-generating unit that generates an evaluation function having the metabolite concentration as the variable, based on previously-obtained biological state information including metabolite concentration data concerning metabolite concentration and biological state indicator data concerning the indicator showing the biological state; and
    a biological state-evaluating unit that evaluates the biological state to be evaluated, based on the evaluation function generated by the evaluation function-generating unit and the previously acquired metabolite concentration data to be evaluated,
    the evaluation function-generating unit further including:

    a candidate evaluation function-generating unit that generates a candidate evaluation function that is a candidate of the evaluation function from the biological state information according to a particular function-generating method;
    a candidate evaluation function-verifying unit that verifies the candidate evaluation function prepared by the candidate evaluation function-generating unit according to a particular verification method; and
    a variable-selecting unit that selects the combination of the metabolite concentration data contained in the biological state information to be used in preparing the candidate evaluation function by selecting a variable of the candidate evaluation function from the verification results by the candidate evaluation function verification unit according to a particular variable selection method, wherein
    the evaluation function-generating unit generates the evaluation function by selecting a candidate evaluation function to be used as the evaluation function among the candidate evaluation functions based on the verification results accumulated by repeated execution of the candidate evaluation function-generating unit, the candidate evaluation function-verifying unit and the variable-selecting unit.

2. The biological state-evaluating apparatus according to claim 1, wherein
the candidate evaluation function-generating unit generates the candidate evaluation functions from the biological state information by using a plurality of different function-generating methods.

3. The biological state-evaluating apparatus according to claim 1 or 2, wherein
the function-generating method is multivariate analysis.

4. The biological state-evaluating apparatus according to any one of claims 1 to 3, wherein
the candidate evaluation function-verifying unit verifies at least one of the discrimination rate, sensitivity, specificity, and information criterion of the candidate evaluation functions according to at least one of bootstrap method, holdout method, and leave-one-out method.

5. The biological state-evaluating apparatus according to any one of claims 1 to 4, wherein
the variable-selecting unit selects the variable of the candidate evaluation function from the verification results according to at least one of stepwise method, best path method, local search method, and genetic algorithm.

6. The biological state-evaluating apparatus according to any one of claims 1 to 5, wherein
the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample.

7. The biological state-evaluating apparatus according to any one of claims 1 to 6, wherein
the metabolite concentration data to be evaluated are of a patient with ulcerative colitis or Crohn's disease.

8. A method of evaluating biological state, comprising:

an evaluation function-generating step of forming an evaluation function having the metabolite concentration as the variable, based on previously-obtained biological state information including metabolite concentration data concerning metabolite concentration and biological state indicator data concerning the indicator showing the biological state; and
a biological state-evaluating step of evaluating the biological state to be evaluated, based on the evaluation function generated in the evaluation function-generating step and the previously acquired metabolite concentration data to be evaluated,
the evaluation function-generating step further including:

a candidate evaluation function-generating step of generating a candidate evaluation function that is a candidate of the evaluation function from the biological state information according to a particular function-generating method;
a candidate evaluation function-verifying step of verifying the candidate evaluation function generated in the candidate evaluation function-generating step according to a particular verification method verification; and
a variable-selecting step of selecting the combination of the metabolite concentration data contained in the biological state information to be used in preparing the candidate evaluation function by selecting a variable of the candidate evaluation function from the verification results obtained in the candidate evaluation function-verifying step according to a particular variable selection method, wherein
in the evaluation function-generating step, the evaluation function is generated by selecting a candidate evaluation function to be used as the evaluation function among the candidate evaluation functions based on the verification results accumulated by repeated execution of the candidate evaluation function-generating step, the candidate evaluation function verification step and the variable-selecting step.

9. The biological state-evaluating method according to claim 8, wherein
the candidate evaluation functions are generated from the biological state information by using a plurality of different function-generating methods in the candidate evaluation function-generating step.

10. The biological state-evaluating method according to claim 8 or 9, wherein
the function-generating method is multivariate analysis.

11. The biological state-evaluating method according to any one of claims 8 to 10, wherein
verification is performed according to at least one of bootstrap method, holdout method, and leave-one-out method, on at least one of the discrimination rate, sensitivity, specificity, and information criterion of the candidate evaluation functions, in the candidate evaluation function-verifying step.

**12.** The biological state-evaluating method according to any one of claims 8 to 11, wherein
the variable of the candidate evaluation function is selected from the verification results according to at least one of stepwise method, best path method, local search method, and genetic algorithm in the variable-selecting step.

**13.** The biological state-evaluating method according to any one of claims 8 to 12, wherein
the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample.

**14.** The biological state-evaluating method according to any one of claims 8 to 13, wherein
the metabolite concentration data to be evaluated are of a patient with ulcerative colitis or Crohn's disease.

**15.** A biological state-evaluating system, comprising a biological state-evaluating apparatus that evaluates biological state and information communication terminal apparatuses that provide the metabolite concentration data to be evaluated communicatively connected thereto via a network,
the information communication terminal apparatus including:

a sending unit that sends the metabolite concentration data to the biological state-evaluating apparatus; and
a receiving unit that receives the evaluation results corresponding to the metabolite concentration data sent from the sending unit from the biological state-evaluating apparatus;
the biological state-evaluating apparatus including:

an evaluation function-generating unit that generates an evaluation function having the metabolite concentration as the variable, based on previously-obtained biological state information including metabolite concentration data concerning metabolite concentration and biological state indicator data concerning the indicator showing the biological state;
a biological state-evaluating unit that evaluates the biological state to be evaluated, based on the evaluation function generated by the evaluation function-generating unit and the previously acquired metabolite concentration data to be evaluated; and
an evaluation result-sending unit that sends the evaluation results obtained by the biological state-evaluating unit to the information communication terminal apparatus, wherein
the evaluation function-generating unit further includes:

a candidate evaluation function-generating unit that generates a candidate evaluation function that is a candidate of the evaluation function from the biological state information according to a particular function-generating method;
a candidate evaluation function-verifying unit that verifies the candidate evaluation function prepared by the candidate evaluation function-generating unit according to a particular verification method; and
a variable-selecting unit that selects the combination of the metabolite concentration data contained in the biological state information to be used in preparing the candidate evaluation function by selecting a variable of the candidate evaluation function from the verification results by the candidate evaluation function verification unit according to a particular variable selection method, and
the evaluation function-generating unit generates the evaluation function by selecting a candidate evaluation function to be used as the evaluation function among the candidate evaluation functions based on the verification results accumulated by repeated execution of the candidate evaluation function-generating unit, the candidate evaluation function-verifying unit and the variable-selecting unit.

**16.** The biological state-evaluating system according to claim 15, wherein
the candidate evaluation function-generating unit generates the candidate evaluation functions from the biological state information by using a plurality of different function-generating methods.

**17.** The biological state-evaluating system according to claim 15 or 16, wherein
the function-generating method is multivariate analysis.

**18.** The biological state-evaluating system according to any one of claims 15 or 17, wherein
the candidate evaluation function-verifying unit verifies at least one of the discrimination rate, sensitivity, specificity, and information criterion of the candidate evaluation functions according to at least one of bootstrap method, holdout method, and leave-one-out method.

**19.** The biological state-evaluating system according to any one of claims 15 or 18, wherein
the variable-selecting unit selects the variable of the candidate evaluation function from the verification results according to at least one of stepwise method, best path method, local search method, and genetic algorithm.

**20.** The biological state-evaluating system according to any one of claims 15 or 19, wherein
the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample.

**21.** The biological state-evaluating system according to any one of claims 15 or 20, wherein
the metabolite concentration data to be evaluated are of a patient with ulcerative colitis or Crohn's disease.

**22.** A biological state-evaluating program making computer execute a biological state-evaluating method comprising:

an evaluation function-generating step of generating an evaluation function having the metabolite concentration as the variable based on previously-obtained biological state information including metabolite concentration data concerning metabolite concentration and biological state indicator data concerning the indicator showing the biological state; and
a biological state-evaluating step of evaluating the biological state to be evaluated, based on the evaluation function generated in the evaluation function-generating step and the previously acquired metabolite concentration data to be evaluated,
the evaluation function-generating step further including:

a candidate evaluation function-generating step of generating a candidate evaluation function that is a candidate of the evaluation function from the biological state information according to a particular function-generating method;
a candidate evaluation function-verifying step of verifying the candidate evaluation function generated in the candidate evaluation function-generating step according to a particular verification method verification; and
a variable-selecting step of selecting the combination of the metabolite concentration data contained in the biological state information to be used in preparing the candidate evaluation function by selecting a variable of the candidate evaluation function from the verification results obtained in the candidate evaluation function-verifying step according to a particular variable selection method, wherein
in the evaluation function-generating step, the evaluation function is generated by selecting a candidate evaluation function to be used as the evaluation function among the candidate evaluation functions based on the verification results accumulated by repeated execution of the candidate evaluation function-generating step, the candidate evaluation function verification step and the variable-selecting step.

**23.** A computer-readable recording medium, comprising the biological state-evaluating program according to claim 22.

**24.** A biological state-evaluating apparatus, comprising an evaluation function-storing unit that stores an evaluation function having the metabolite concentration as the variable and a biological state-evaluating unit that evaluates the biological state to be evaluated, based on the evaluation function stored in the evaluation function-storing unit and the previously acquired metabolite concentration data to be evaluated.

**25.** The biological state-evaluating apparatus according to claim 24, wherein
the evaluation function-storing unit stores at least one of the evaluation functions represented by the following Formulae 1 to 4:

[Formula 1]

$$a_1x_1 + a_2x_2 + \cdots + a_nx_n \quad \cdots (formula1)$$

[Formula 2]

$$\frac{1}{1 + \exp(b_{n+1} + b_1 x_1 + b_2 x_2 + \cdots + b_n x_n)}$$

$$\cdots (\text{formula2})$$

[Formula 3]

$$c_1 x_1 + c_2 x_2 + \cdots + c_n x_n + \Theta(\vec{x}) \qquad \cdots (\text{formula3})$$

[Formula 4]

$$\left[ K \mid \left(\vec{x} - \overrightarrow{x_K}\right) X_K \left(\vec{x} - \overrightarrow{x_K}\right)^t \right.$$
$$\left. = \min\left\{ \left(\vec{x} - \overrightarrow{x_1}\right) X_1 \left(\vec{x} - \overrightarrow{x_1}\right)^t, \left(\vec{x} - \overrightarrow{x_2}\right) X_2 \left(\vec{x} - \overrightarrow{x_2}\right)^t, \cdots, \left(\vec{x} - \overrightarrow{x_j}\right) X_j \left(\vec{x} - \overrightarrow{x_j}\right)^t \right\} \right]$$

$$\cdots (\text{formula4})$$

[Formula 5]

$$\Theta = \left[ \left\{ \gamma(\vec{c} \cdot \vec{x}) + c_0 \right\}^p, \quad \exp\left(-\gamma \times |\vec{c} - \vec{x}|^2\right), \quad \tanh\left\{ \gamma(\vec{c} \cdot \vec{x}) + c_0 \right\} \right]$$
$$\vec{x} = (x_1, x_2, \cdots, x_n) \qquad \vec{c} = (c_1, c_2, \cdots, c_n) \qquad \gamma, c_0 : \text{cons} \tan t$$

$$\cdots (\text{formula5})$$

(in Formula 1, each of $a_1$ to $a_n$ is a real number, satisfying the formula: "$a_1 + a_2 + \ldots + a_n = 1$"; in Formula 2, each of $b_1$ to $b_{n+1}$ is a real number, satisfying the formula "$|b_i| < 1$" (i=1 to n) ; in Formula 3, each of $c_1$ to $c_n$ is a real number; $\Theta$ is defined by Formula 5; and in Formula 4, j is an integer), and the biological state-evaluating unit evaluates the biological state, based on at least one of the stored evaluation functions and the metabolite concentration data.

26. The biological state-evaluating apparatus according to claim 24 or 25, wherein
the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample.

27. The biological state-evaluating apparatus according to any one of claims 24 to 26, wherein
the metabolite concentration data to be evaluated are of a patient with any of ulcerative colitis, Crohn's disease, asthma, or rheumatism.

28. An evaluation function-generating apparatus that generates an evaluation function having the metabolite concentration as the variable, based on previously-obtained biological state information including metabolite concentration data concerning metabolite concentration and biological state indicator data concerning the indicator showing the biological state, comprising:

a candidate evaluation function-generating unit that generates a candidate evaluation function that is a candidate of the evaluation function from the biological state information according to a particular function-generating method;

a candidate evaluation function-verifying unit that verifies the candidate evaluation function generated by the candidate evaluation function-generating unit according to a particular verification method; and

a variable-selecting unit that selects the combination of the metabolite concentration data contained in the biological state information to be used in preparing the candidate evaluation function by selecting a variable of the candidate evaluation function from the verification results by the candidate evaluation function verification unit according to a particular variable selection method, wherein

the evaluation function is generated by selecting a candidate evaluation function to be used as the evaluation function among the candidate evaluation functions based on the verification results accumulated by repeated execution of the candidate evaluation function-generating unit, the candidate evaluation function-verifying unit and the variable-selecting unit.

29. The evaluation function-generating apparatus according to claim 28, wherein
the candidate evaluation function-generating unit generates the candidate evaluation functions from the biological state information by using a plurality of different function-generating methods.

30. The evaluation function-generating apparatus according to claim 28 or 29, wherein
the function-generating method is multivariate analysis.

31. The evaluation function-generating apparatus according to any one of claims 28 to 30, wherein
the candidate evaluation function-verifying unit verifies at least one of the discrimination rate, sensitivity, specificity, and information criterion of the candidate evaluation functions according to at least one of bootstrap method, holdout method, and leave-one-out method.

32. The evaluation function-generating apparatus according to any one of claims 28 to 31, wherein
the variable-selecting unit selects the variable of the candidate evaluation function from the verification results according to at least one of stepwise method, best path method, local search method, and genetic algorithm.

33. The evaluation function-generating apparatus according to any one of claims 28 to 32, wherein
the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample.

34. An evaluation function-generating method of generating an evaluation function having the metabolite concentration as the variable, based on previously-obtained biological state information including metabolite concentration data concerning metabolite concentration and biological state indicator data concerning the indicator showing the biological state, comprising:

a candidate function-generating step of generating a candidate evaluation function that is a candidate of the evaluation function from the biological state information according to a particular function-generating method;

a candidate evaluation function-verifying step of verifying the candidate evaluation function generated in the candidate evaluation function-generating step according to a particular verification method; and

a variable-selecting step of selecting the combination of the metabolite concentration data contained in the biological state information to be used in preparing the candidate evaluation function by selecting a variable of the candidate evaluation function from the verification results obtained in the candidate evaluation function-verifying step according to a particular variable selection method, wherein

the evaluation function is generated by selecting a candidate evaluation function to be used as the evaluation function among the candidate evaluation functions based on the verification results accumulated by repeated execution of the candidate evaluation function-generating step, the candidate evaluation function verification step and the variable-selecting step.

35. The evaluation function-generating method according to claim 34, wherein
the candidate evaluation functions are generated by using a plurality of different function-generating methods in the candidate evaluation function-generating step from the biological state information.

36. The evaluation function-generating method according to claim 34 or 35, wherein
the function-generating method is multivariate analysis.

37. The evaluation function-generating method according to any one of claims 34 to 36, wherein

the candidate evaluation function is verified according to at least one of bootstrap method, holdout method, and leave-one-out method, on at least one of the discrimination rate, sensitivity, specificity, and information criterion of the candidate evaluation functions, in the candidate evaluation function-verifying step.

**38.** The evaluation function-generating method according to any one of claims 34 to 37, wherein the variable of the candidate evaluation function is selected from the verification results according to at least one of stepwise method, best path method, local search method, and genetic algorithm in the variable-selecting step.

**39.** The evaluation function-generating method according to any one of claims 34 to 38, wherein the metabolite concentration data are data concerning the concentration of an amino acid, an amino acid analogue, or an amino or imino group-containing compound in a biological sample, or data concerning the concentration of a peptide, a protein, a sugar, a lipid, a vitamin, a mineral or the metabolite thereof in a biological sample.

**40.** An evaluation function-generating program that makes computer execute an evaluation function-generating method of generating an evaluation function having the metabolite concentration as the variable, based on previously-obtained biological state information including metabolite concentration data concerning metabolite concentration and biological state indicator data concerning the indicator showing the biological state, the method comprising:

a candidate evaluation function-generating step of generating a candidate evaluation function that is a candidate of the evaluation function from the biological state information according to a particular function-generating method;
a candidate evaluation function-verifying step of verifying the candidate evaluation function generated in the candidate evaluation function-generating step according to a particular verification method; and
a variable-selecting step of selecting the combination of the metabolite concentration data contained in the biological state information to be used in preparing the candidate evaluation function by selecting a variable of the candidate evaluation function from the verification results obtained in the candidate evaluation function-verifying step according to a particular variable selection method, wherein
the evaluation function is generated by selecting a candidate evaluation function to be used as the evaluation function among the candidate evaluation functions based on the verification results accumulated by repeated execution of the candidate evaluation function-generating step, the candidate evaluation function verification step and the variable-selecting step.

**41.** A computer-readable recording medium, comprising the recorded evaluation function-generating program according to claim 40.

EP 1 862 797 A1

# FIG.1

# FIG.2

100

BIOLOGICAL STATE-
EVALUATING APPARATUS

300

NETWORK

200

CLIENT APPARATUS
(INFORMATION
COMMUNICATION
TERMINAL APPARATUS)

200

CLIENT APPARATUS
(INFORMATION
COMMUNICATION
TERMINAL APPARATUS)

200

CLIENT APPARATUS
(INFORMATION
COMMUNICATION
TERMINAL APPARATUS)

200

CLIENT APPARATUS
(INFORMATION
COMMUNICATION
TERMINAL APPARATUS)

EP 1 862 797 A1

# FIG.3

CLIENT APPARATUS
(INFORMATION
COMMUNICATION
TERMINAL APPARATUS)

CLIENT APPARATUS
(INFORMATION
COMMUNICATION
TERMINAL APPARATUS)

CLIENT APPARATUS
(INFORMATION
COMMUNICATION
TERMINAL APPARATUS)

CLIENT APPARATUS
(INFORMATION
COMMUNICATION
TERMINAL APPARATUS)

200

BIOLOGICAL STATE-
EVALUATING APPARATUS

100

300

NETWORK

400

DATABASE APPARATUS

EP 1 862 797 A1

FIG.4

EP 1 862 797 A1

INPUT DEVICE ~112

OUTPUT DEVICE ~114

100

BIOLOGICAL STATE-EVALUATING APPARATUS

INPUT/OUTPUT INTERFACE ~108

106

MEMORY DEVICE

USER INFORMATION FILE ~106a

BIOLOGICAL STATE INFORMATION FILE ~106b

DESIGNATED BIOLOGICAL STATE INFORMATION FILE ~106c

EVALUATION FUNCTION-RELATED INFORMATION DATABASE ~106d

CANDIDATE EVALUATION FUNCTION FILE 106d1
VERIFICATION RESULT FILE 106d2
SELECTED BIOLOGICAL STATE INFORMATION FILE 106d3
EVALUATION FUNCTION FILE 106d4

EVALUATION RESULT FILE ~106e

102

CONTROLLING DEVICE

INSTRUCTION-ANALYZING PART ~102a

BROWSING PROCESSING PART ~102b

AUTHENTICATION-PROCESSING PART ~102c

ELECTRONIC MAIL-GENERATING PART ~102d

WEB PAGE-GENERATING PART ~102e

SENDING PART ~102f

BIOLOGICAL STATE INFORMATION-ACQUIRING PART ~102g

BIOLOGICAL STATE INFORMATION-DESIGNATING PART ~102h

EVALUATION FUNCTION-GENERATING PART ~102i

BIOLOGICAL STATE-EVALUATING PART ~102j

RESULT OUTPUTTING PART ~102k

104

COMMUNICATION INTERFACE

300

NETWORK

# FIG.5

| USER ID | USER PASSWORD | NAME | ORGANIZATION ID | DEPARTMENT ID | DEPARTMENT NAME | ELECTRONIC MAIL ADDRESS | · · · |
|---|---|---|---|---|---|---|---|
| ⋮ | ⋮ | ⋮ | ⋮ | ⋮ | ⋮ | ⋮ | ⋮ |

106a

EP 1 862 797 A1

# FIG.6

| INDIVIDUAL (SAMPLE) NUMBER | BIOLOGICAL STATE INDICATOR DATA (T) | | | | METABOLITE CONCENTRATION DATA | | | | | |
|---|---|---|---|---|---|---|---|---|---|---|
| | $T_1$ | $T_2$ | $T_3$ | ... | Gly | Leu | Val | Ile | Phe | ... |
| A-1 | 23.4 | 62.5 | 37.1 | ... | 9.5 | 11.2 | 2.7 | 8.5 | 4.9 | ... |
| A-2 | 27.5 | 66.1 | 39.5 | ... | 8.5 | 10.5 | 3.9 | 9.8 | 6.1 | ... |
| ⋮ | ⋮ | ⋮ | ⋮ | ⋮ | ⋮ | | | | | |

106b

# FIG.7

106c

| INDIVIDUAL (SAMPLE) NUMBER | BIOLOGICAL STATE INDICATOR DATA (T) | METABOLITE CONCENTRATION DATA | | | |
|---|---|---|---|---|---|
| | $T_2$ | Gly | Leu | Phe | ... |
| A-1 | 62.5 | 9.5 | 11.2 | 4.9 | ... |
| A-2 | 66.1 | 8.5 | 10.5 | 6.1 | ... |
| ⋮ | ⋮ | . | | . | . |

# FIG.8

106d1

| RANK | CANDIDATE EVALUATION FUNCTION |
|---|---|
| 1 | $F_1(Gly, Leu, Phe, ...)$ |
| 2 | $F_2(Gly, Leu, Phe, ...)$ |
| 3 | $F_3(Gly, Leu, Phe, ...)$ |
| ⋮ | ⋮ |

# FIG.9

106d2

| RANK | CANDIDATE EVALUATION FUNCTION | VERIFICATION RESULT |
|------|-------------------------------|---------------------|
| 1 | $F_k$(Gly,Leu,Phe,...) | 1.22 |
| 2 | $F_m$(Gly,Leu,Phe,...) | 2.28 |
| 3 | $F_l$(Gly,Leu,Phe,...) | 2.95 |
| ⋮ | ⋮ | ⋮ |

# FIG.10

106d3

| INDIVIDUAL (SAMPLE) NUMBER | BIOLOGICAL STATE INDICATOR DATA (T) | METABOLITE CONCENTRATION DATA | | |
|----------------------------|-------------------------------------|--------|--------|--------|
| | $T_2$ | Leu | Phe | ... |
| A-1 | 62.5 | 11.2 | 4.9 | ... |
| A-2 | 66.1 | 10.5 | 6.1 | ... |
| ⋮ | ⋮ | ⋮ | ⋮ | ⋮ |

# FIG.11

106d4

| RANK | EVALUATION FUNCTION | THRESHOLD | VERIFICATION RESULT |
|------|---------------------|-----------|---------------------|
| 1 | $F_p(Phe,...)$ | 0.23 | 0.62 |
| 2 | $F_p(Gly,Leu,Phe)$ | -2.12 | 1.02 |
| 3 | $F_k(Gly,Leu,Phe,...)$ | 1.23 | 1.22 |
| ⋮ | ⋮ | ⋮ | ⋮ |

# FIG.12

106e

| INDIVIDUAL (SAMPLE) NUMBER | METABOLITE CONCENTRATION DATA | | | | SCORE | | JUDGMENT | |
|---|---|---|---|---|---|---|---|---|
| | Gly | Leu | Phe | ... | UC | CD | UC | CD |
| U-1 | 9.5 | 11.2 | 4.9 | ... | 1.02 | -1.09 | - | - |
| U-2 | 8.5 | 10.5 | 6.1 | ... | 2.90 | 0.41 | + | - |
| ⋮ | ⋮ | ⋮ | ⋮ | ⋮ | ⋮ | | ⋮ | ⋮ |

# FIG.13

102i

EVALUATION FUNCTION-GENERATING PART

| CANDIDATE EVALUATION FUNCTION-GENERATING PART | ~ 102i1 |

| CANDIDATE EVALUATION FUNCTION-VERIFYING PART | ~ 102i2 |

| VARIABLE-SELECTING PART | ~ 102i3 |

# FIG.14

CLIENT APPARATUS — 200

INPUT DEVICE — 250

OUTPUT DEVICE — 260

MONITOR — 261

PRINTER — 262

INPUT/OUTPUT IF — 270

RAM — 240

ROM — 220

HD — 230

COMMUNICATION IF — 280

CONTROLLING DEVICE — 210

WEB BROWSER — 211

ELECTRONIC MAILER — 212

NETWORK — 300

# FIG.15

| | | | | |
|---|---|---|---|---|
| 412 | | 414 | | |
| INPUT DEVICE | | OUTPUT DEVICE | | |

400

DATABASE APPARATUS

INPUT/OUTPUT INTERFACE — 408

406

402

404

CONTROLLING DEVICE

INSTRUCTION-ANALYZING PART — 402a

BROWSING PROCESSING PART — 402b

AUTHENTICATION-PROCESSING PART — 402c

ELECTRONIC MAIL-GENERATING PART — 402d

WEB PAGE-GENERATING PART — 402e

SENDING PART — 402f

MEMORY DEVICE

COMMUNICATION INTERFACE

300

NETWORK

# FIG.16

(DATABASE APPARATUS 400)

(BIOLOGICAL STATE-EVALUATING APPARATUS 100)

(CLIENT APPARATUS 200)

START

START

START

SA-2

TRANSMISSION OF BIOLOGICAL STATE INFORMATION FOR GENERATION OF EVALUATION FUNCTION

SA-1

TRANSMISSION OF METABOLITE CONCENTRATION DATA

SA-3

EXECUTION OF BIOLOGICAL STATE EVALUATION PROCESSING

SA-4

TRANSMISSION OF EVALUATION RESULTS

SA-5

STORAGE OF EVALUATION RESULTS

SA-6

OUTPUT OF EVALUATION RESULTS

END

END

END

# FIG.17

START

ACQUISITION OF BIOLOGICAL STATE INFORMATION AND METABOLITE CONCENTRATION DATA — SB-1

SELECTION OF INDIVIDUAL GROUP — SB-2

DATA REMOVAL — SB-3

DESIGNATION OF BIOLOGICAL STATE INDICATOR DATA AND METABOLITE CONCENTRATION DATA — SB-4

GENERATION OF EVALUATION FUNCTION

PREPARATION OF CANDIDATE EVALUATION FUNCTION — SB-5

VERIFICATION OF CANDIDATE EVALUATION FUNCTION — SB-6

SELECTION OF VARIABLE OF CANDIDATE EVALUATION FUNCTION — SB-7

SB-8

ALL COMBINATION COMPLETED?

NO

YES

SELECTION OF CANDIDATE EVALUATION FUNCTION (DETERMINATION OF EVALUATION FUNCTION) — SB-9

EVALUATION OF BIOLOGICAL STATE — SB-10

END

# FIG.18

```
        ┌─────────────┐
        │    START    │
        └─────────────┘
               │
               ▼
┌───────────────────────────────┐
│  DETERMINATION OF THE FORM OF  │──  SC-1
│  CANDIDATE EVALUATION FUNCTION │
└───────────────────────────────┘
               │
               ▼
┌───────────────────────────────┐
│     CALCULATION OF AVERAGE,    │──  SC-2
│      VARIANCE, AND OTHERS      │
└───────────────────────────────┘
               │
               ▼
┌───────────────────────────────┐
│  DETERMINATION OF THE PARAMETER│
│   OF CANDIDATE EVALUATION      │──  SC-3
│           FUNCTION             │
└───────────────────────────────┘
               │
               ▼
        ┌─────────────┐
        │     END     │
        └─────────────┘
```

## FIG.19

| PATIENT ID | DISEASE STATE | SCORE | | | | JUDGMENT | | | |
|---|---|---|---|---|---|---|---|---|---|
| | | LRA | SVM | LDA | MAP | LRA | SVM | LDA | MAP |
| 1 | N | 0.68 | 0.14 | -1.14 | 2.62 | N | N | N | N |
| 2 | N | 0.93 | -0.05 | -3.20 | 1.95 | N | N | N | N |
| 3 | N | 0.75 | 0.05 | -1.08 | 1.99 | N | N | N | N |
| 4 | N | 0.86 | 0.22 | -0.28 | 2.60 | N | N | N | N |
| 5 | N | 0.91 | 0.06 | -2.52 | 1.46 | N | N | N | N |
| 6 | N | 0.99 | -0.05 | -3.23 | 2.04 | N | N | N | N |
| 7 | N | 0.56 | 0.42 | -0.05 | 2.87 | N | N | N | N |
| 8 | N | 0.15 | 0.18 | -0.53 | 3.56 | UC | N | N | UC |
| 9 | N | 0.95 | -0.02 | -1.38 | 2.60 | N | N | N | N |
| 10 | N | 0.99 | 0.05 | -1.66 | 2.24 | N | N | N | N |
| 11 | N | 0.40 | 0.05 | -0.50 | 2.55 | UC | N | N | N |
| 12 | N | 0.99 | 0.05 | -1.93 | 1.87 | N | N | N | N |
| 13 | N | 0.73 | 0.05 | -1.63 | 2.35 | N | N | N | N |
| 14 | N | 0.97 | 0.13 | -1.04 | 1.56 | N | N | N | N |
| 15 | N | 0.88 | 0.12 | -3.03 | 2.64 | N | N | N | N |
| 16 | N | 0.51 | 0.05 | -1.69 | 2.52 | N | N | N | N |
| 17 | N | 0.19 | 0.35 | -0.07 | 2.43 | UC | N | N | N |
| 18 | N | 0.77 | 0.37 | -0.23 | 2.85 | N | N | N | N |
| 19 | N | 0.60 | 0.19 | -0.21 | 3.13 | N | N | N | N |
| 20 | N | 0.36 | 0.05 | -1.90 | 2.13 | UC | N | N | N |
| 21 | UC | 0.57 | 0.74 | 0.32 | 2.55 | N | UC | UC | N |
| 22 | UC | 0.46 | 0.95 | 2.05 | 3.15 | UC | UC | UC | UC |
| 23 | UC | 0.11 | 0.95 | 1.78 | 3.32 | UC | UC | UC | UC |
| 24 | UC | 0.48 | 1.05 | 0.78 | 2.98 | UC | UC | UC | N |
| 25 | UC | 0.16 | 0.64 | 0.44 | 4.36 | UC | UC | UC | UC |
| 26 | UC | 0.23 | 0.95 | 0.04 | 1.48 | UC | UC | UC | N |
| 27 | UC | 0.37 | 0.83 | 1.16 | 2.67 | UC | UC | UC | N |
| 28 | UC | 0.21 | 0.80 | 2.59 | 3.48 | UC | UC | UC | UC |
| 29 | UC | 0.23 | 0.72 | 0.05 | 3.51 | UC | UC | UC | UC |
| 30 | UC | 0.13 | 1.03 | 0.79 | 3.19 | UC | UC | UC | UC |
| 31 | UC | 0.01 | 0.95 | 3.44 | 4.24 | UC | UC | UC | UC |
| 32 | UC | 0.01 | 0.95 | 2.77 | 4.82 | UC | UC | UC | UC |
| 33 | UC | 0.50 | 0.55 | 0.35 | 3.51 | N | UC | UC | UC |
| 34 | UC | 0.01 | 0.95 | 2.04 | 3.88 | UC | UC | UC | UC |
| 35 | UC | 0.78 | 0.95 | 1.75 | 3.62 | N | UC | UC | UC |
| 36 | UC | 0.73 | 0.74 | 0.78 | 2.94 | N | UC | UC | N |
| 37 | UC | 0.53 | 0.72 | 1.02 | 3.42 | N | UC | UC | UC |
| 38 | UC | 0.04 | 0.95 | 2.10 | 6.79 | UC | UC | UC | UC |
| 39 | UC | 0.09 | 0.95 | 1.37 | 3.78 | UC | UC | UC | UC |
| 40 | UC | 0.14 | 0.99 | 1.66 | 3.16 | UC | UC | UC | UC |

## FIG.20

| PATIENT ID | SCORE | | | | JUDGMENT | | | | DIAGNOSIS BY DOCTOR |
| --- | --- | --- | --- | --- | --- | --- | --- | --- | --- |
| | LRA | SVM | LDA | MAP | LRA | SVM | LDA | MAP | |
| UK1 | 0.05 | 0.41 | -1.45 | 2.26 | UC | N | N | N | N |
| UK2 | 0.96 | 0.25 | -0.70 | 3.10 | N | N | N | N | N |
| UK3 | 0.94 | 0.03 | -1.38 | 3.11 | N | N | N | N | N |
| UK4 | 0.88 | 0.03 | -2.79 | 2.89 | N | N | N | N | N |
| UK5 | 0.84 | -0.07 | -1.19 | 2.40 | N | N | N | N | N |
| UK6 | 0.80 | 0.12 | -1.19 | 3.59 | N | N | N | UC | N |
| UK7 | 0.85 | -0.02 | -1.99 | 2.66 | N | N | N | N | N |
| UK8 | 0.74 | -0.09 | -1.40 | 2.45 | N | N | N | N | N |
| UK9 | 0.91 | 0.59 | -0.26 | 4.49 | N | UC | N | UC | N |
| UK10 | 0.17 | 0.73 | -0.17 | 3.64 | UC | UC | N | UC | N |
| UK11 | 0.30 | 1.05 | 2.33 | 3.04 | UC | UC | UC | N | UC |
| UK12 | 0.64 | 0.87 | 0.69 | 3.23 | N | UC | UC | UC | UC |
| UK13 | 0.52 | 0.26 | 1.27 | 2.56 | N | N | UC | N | UC |
| UK14 | 0.82 | 0.45 | 0.54 | 3.35 | N | N | UC | UC | UC |
| UK15 | 0.09 | 1.01 | 2.01 | 3.79 | UC | UC | UC | UC | UC |
| UK16 | 0.00 | 0.46 | 1.15 | 3.58 | UC | N | UC | UC | UC |
| UK17 | 0.87 | 0.14 | -0.69 | 2.53 | N | N | N | N | UC |
| UK18 | 0.17 | 0.65 | 2.44 | 3.34 | UC | UC | UC | UC | UC |
| UK19 | 0.10 | 0.49 | 1.13 | 3.37 | UC | N | UC | UC | UC |
| UK20 | 0.20 | 0.86 | 0.28 | 4.26 | UC | UC | UC | UC | UC |

# FIG.21

| PATIENT ID | DISEASE STATE | SCORE | | | | JUDGMENT | | | |
|---|---|---|---|---|---|---|---|---|---|
| | | LRA | SVM | LDA | MAP | LRA | SVM | LDA | MAP |
| 1 | N | 0.86 | 0.34 | 2.08 | 0.14 | N | N | N | N |
| 2 | N | 0.93 | 0.05 | 1.66 | 0.14 | N | N | N | N |
| 3 | N | 0.71 | 0.05 | 0.57 | 0.16 | N | N | N | N |
| 4 | N | 0.88 | 0.07 | 1.31 | 0.14 | N | N | N | N |
| 5 | N | 0.65 | 0.05 | 0.30 | 0.15 | N | N | N | N |
| 6 | N | 0.92 | 0.05 | 1.92 | 0.13 | N | N | N | N |
| 7 | N | 0.79 | 0.08 | 0.51 | 0.16 | N | N | N | N |
| 8 | N | 0.71 | 0.45 | 0.26 | 0.18 | N | N | N | N |
| 9 | N | 0.62 | 0.38 | 0.33 | 0.17 | N | N | N | N |
| 10 | N | 0.62 | 0.08 | 1.69 | 0.13 | N | N | N | N |
| 11 | N | 0.76 | 0.16 | 1.13 | 0.15 | N | N | N | N |
| 12 | N | 0.67 | 0.05 | 1.45 | 0.12 | N | N | N | N |
| 13 | N | 0.93 | 0.05 | 1.49 | 0.13 | N | N | N | N |
| 14 | N | 0.66 | 0.05 | 0.51 | 0.14 | N | N | N | N |
| 15 | N | 0.07 | 0.44 | -1.54 | 0.22 | CD | N | CD | CD |
| 16 | N | 0.75 | 0.15 | 1.50 | 0.15 | N | N | N | N |
| 17 | N | 0.78 | 0.05 | 0.97 | 0.15 | N | N | N | N |
| 18 | N | 0.92 | 0.27 | 1.15 | 0.15 | N | N | N | N |
| 19 | N | 0.49 | 0.18 | 0.34 | 0.18 | CD | N | N | N |
| 20 | N | 0.80 | 0.05 | 1.44 | 0.15 | N | N | N | N |
| 21 | CD | 0.36 | 0.78 | -0.80 | 0.20 | CD | CD | CD | CD |
| 22 | CD | 0.50 | 0.56 | -0.33 | 0.16 | N | CD | CD | N |
| 23 | CD | 0.06 | 0.91 | -1.12 | 0.24 | CD | CD | CD | CD |
| 24 | CD | 0.17 | 0.95 | -1.42 | 0.25 | CD | CD | CD | CD |
| 25 | CD | 0.89 | 0.56 | 1.38 | 0.15 | N | CD | N | N |
| 26 | CD | 0.01 | 0.95 | -3.55 | 0.30 | CD | CD | CD | CD |
| 27 | CD | 0.89 | 0.14 | 1.41 | 0.13 | N | N | N | N |
| 28 | CD | 0.12 | 0.95 | -1.88 | 0.25 | CD | CD | CD | CD |
| 29 | CD | 0.08 | 0.95 | -1.71 | 0.23 | CD | CD | CD | CD |
| 30 | CD | 0.08 | 0.82 | -1.02 | 0.20 | CD | CD | CD | CD |
| 31 | CD | 0.03 | 0.95 | -1.02 | 0.23 | CD | CD | CD | CD |
| 32 | CD | 0.70 | 0.69 | -0.05 | 0.19 | N | CD | CD | CD |
| 33 | CD | 0.43 | 0.79 | -0.09 | 0.20 | CD | CD | CD | CD |
| 34 | CD | 0.53 | 0.75 | -0.47 | 0.20 | N | CD | CD | CD |
| 35 | CD | 0.21 | 0.65 | -0.49 | 0.19 | CD | CD | CD | CD |
| 36 | CD | 0.23 | 0.95 | -1.60 | 0.25 | CD | CD | CD | CD |
| 37 | CD | 0.07 | 0.85 | -1.19 | 0.22 | CD | CD | CD | CD |
| 38 | CD | 0.02 | 0.95 | -1.26 | 0.22 | CD | CD | CD | CD |
| 39 | CD | 0.10 | 0.95 | -1.30 | 0.25 | CD | CD | CD | CD |
| 40 | CD | 0.01 | 0.95 | -2.54 | 0.24 | CD | CD | CD | CD |

# FIG.22

| PATIENT ID | SCORE | | | | JUDGMENT | | | | DIAGNOSIS BY DOCTOR |
|---|---|---|---|---|---|---|---|---|---|
| | LRA | SVM | LDA | MAP | LRA | SVM | LDA | MAP | |
| UK1 | 0.88 | 0.35 | 4.13 | 0.13 | N | N | N | N | N |
| UK2 | 0.96 | 0.30 | 1.36 | 0.14 | N | N | N | N | N |
| UK3 | 0.56 | 0.57 | -0.43 | 0.19 | N | CD | CD | CD | N |
| UK4 | 0.89 | 0.47 | 1.62 | 0.14 | N | N | N | N | N |
| UK5 | 0.35 | 0.50 | 0.53 | 0.17 | CD | N | N | N | N |
| UK6 | 0.96 | 0.37 | 2.18 | 0.12 | N | N | N | N | N |
| UK7 | 0.33 | 0.77 | -0.27 | 0.21 | CD | CD | CD | CD | N |
| UK8 | 0.26 | 0.65 | -0.87 | 0.21 | CD | CD | CD | CD | N |
| UK9 | 0.92 | 0.08 | 1.09 | 0.13 | N | N | N | N | N |
| UK10 | 0.99 | 0.45 | 2.14 | 0.12 | N | N | N | N | N |
| UK11 | 0.83 | 0.49 | 0.90 | 0.16 | N | N | N | N | CD |
| UK12 | 0.02 | 0.98 | -2.82 | 0.29 | CD | CD | CD | CD | CD |
| UK13 | 0.96 | 0.40 | 1.68 | 0.14 | N | N | N | N | CD |
| UK14 | 0.02 | 0.93 | -2.13 | 0.28 | CD | CD | CD | CD | CD |
| UK15 | 0.04 | 0.84 | -1.98 | 0.26 | CD | CD | CD | CD | CD |
| UK16 | 0.82 | 0.35 | 0.45 | 0.16 | N | N | N | N | CD |
| UK17 | 0.91 | 0.62 | 1.51 | 0.16 | N | CD | N | N | CD |
| UK18 | 0.07 | 0.79 | -1.30 | 0.23 | CD | CD | CD | CD | CD |
| UK19 | 0.07 | 0.77 | -1.15 | 0.22 | CD | CD | CD | CD | CD |
| UK20 | 0.15 | 0.61 | -1.26 | 0.23 | CD | CD | CD | CD | CD |

# FIG.23

| RAT ID | DISEASE STATE | SCORE | | | | JUDGMENT | | | |
|---|---|---|---|---|---|---|---|---|---|
| | | LRA | SVM | LDA | MAP | LRA | SVM | LDA | MAP |
| 1 | N | 1.00 | 1.01 | -2.88 | 0.96 | N | N | N | N |
| 2 | N | 1.00 | 0.97 | -2.86 | 1.11 | N | N | N | N |
| 3 | N | 1.00 | 1.01 | -3.01 | 1.10 | N | N | N | N |
| 4 | N | 1.00 | 0.97 | -2.52 | 1.20 | N | N | N | N |
| 5 | N | 1.00 | 1.04 | -3.74 | 0.90 | N | N | N | N |
| 6 | N | 1.00 | 0.98 | -3.09 | 1.12 | N | N | N | N |
| 7 | N | 1.00 | 1.05 | -2.04 | 1.72 | N | N | N | N |
| 8 | N | 1.00 | 0.99 | -2.74 | 1.13 | N | N | N | N |
| 9 | N | 1.00 | 0.99 | -3.12 | 1.14 | N | N | N | N |
| 10 | N | 1.00 | 1.00 | -2.01 | 1.36 | N | N | N | N |
| 11 | N | 1.00 | 0.98 | -2.39 | 1.35 | N | N | N | N |
| 12 | N | 1.00 | 0.99 | -3.76 | 1.22 | N | N | N | N |
| 13 | N | 1.00 | 0.96 | -2.87 | 1.57 | N | N | N | N |
| 14 | N | 0.87 | 1.01 | -1.08 | 1.82 | N | N | N | N |
| 15 | N | 1.00 | 1.00 | -3.07 | 1.66 | N | N | N | N |
| 16 | N | 1.00 | 1.00 | -3.07 | 1.66 | N | N | N | N |
| 17 | N | 1.00 | 0.96 | -2.23 | 1.82 | N | N | N | N |
| 18 | N | 0.99 | 0.97 | -1.30 | 1.81 | N | N | N | N |
| 19 | N | 1.00 | 1.04 | -0.38 | 2.21 | N | N | N | N |
| 20 | N | 1.00 | 1.02 | -2.06 | 1.62 | N | N | N | N |
| 21 | N | 0.86 | 1.04 | -0.78 | 2.13 | N | N | N | N |
| 22 | N | 0.38 | 1.06 | -1.41 | 1.17 | DM | N | N | N |
| 23 | N | 0.85 | 1.05 | -1.91 | 0.97 | N | N | N | N |
| 24 | N | 0.97 | 0.97 | -2.26 | 1.06 | N | N | N | N |
| 25 | N | 0.97 | 0.96 | -2.99 | 0.92 | N | N | N | N |
| 26 | N | 1.00 | 0.99 | -1.82 | 1.34 | N | N | N | N |
| 27 | N | 1.00 | 0.96 | -2.35 | 1.15 | N | N | N | N |
| 28 | N | 1.00 | 0.96 | -2.36 | 1.17 | N | N | N | N |
| 29 | N | 1.00 | 0.97 | -2.68 | 1.01 | N | N | N | N |
| 30 | N | 0.75 | 0.99 | -1.31 | 1.07 | N | N | N | N |
| 31 | N | 0.94 | 1.04 | -1.23 | 1.22 | N | N | N | N |
| 32 | N | 0.66 | 1.03 | -2.16 | 0.99 | N | N | N | N |
| 33 | N | 0.82 | 1.03 | -1.34 | 1.02 | N | N | N | N |
| 34 | N | 0.96 | 1.04 | -1.19 | 1.09 | N | N | N | N |
| 35 | N | 0.57 | 1.03 | -1.75 | 0.96 | N | N | N | N |
| 36 | N | 1.00 | 0.99 | -3.56 | 0.96 | N | N | N | N |
| 37 | N | 0.86 | 1.06 | -2.49 | 1.06 | N | N | N | N |
| 38 | N | 1.00 | 1.01 | -3.16 | 1.21 | N | N | N | N |
| 39 | N | 1.00 | 0.99 | -3.25 | 1.00 | N | N | N | N |
| 40 | N | 1.00 | 0.99 | -3.44 | 1.07 | N | N | N | N |
| 41 | N | 1.00 | 1.00 | -3.71 | 0.92 | N | N | N | N |
| 42 | N | 1.00 | 0.99 | -3.64 | 1.33 | N | N | N | N |
| 43 | N | 1.00 | 0.99 | -3.63 | 1.55 | N | N | N | N |
| 44 | N | 1.00 | 1.00 | -3.73 | 1.30 | N | N | N | N |
| 45 | N | 1.00 | 1.00 | -3.66 | 1.14 | N | N | N | N |
| 46 | N | 1.00 | 0.98 | -3.56 | 1.52 | N | N | N | N |
| 47 | N | 1.00 | 1.00 | -3.68 | 1.07 | N | N | N | N |
| 48 | N | 1.00 | 0.99 | -3.02 | 1.08 | N | N | N | N |
| 49 | N | 1.00 | 1.02 | -2.61 | 1.14 | N | N | N | N |
| 50 | N | 1.00 | 1.02 | -2.75 | 1.07 | N | N | N | N |

# FIG.24

| RAT ID | DISEASE STATE | SCORE | | | | JUDGMENT | | | |
|---|---|---|---|---|---|---|---|---|---|
| | | LRA | SVM | LDA | MAP | LRA | SVM | LDA | MAP |
| 51 | N | 1.00 | 1.04 | -2.21 | 1.21 | N | N | N | N |
| 52 | N | 1.00 | 1.04 | -2.00 | 1.32 | N | N | N | N |
| 53 | N | 1.00 | 1.04 | -3.04 | 0.92 | N | N | N | N |
| 54 | N | 1.00 | 1.04 | -3.08 | 0.83 | N | N | N | N |
| 55 | N | 1.00 | 1.04 | -3.28 | 0.82 | N | N | N | N |
| 56 | N | 1.00 | 1.02 | -3.43 | 1.01 | N | N | N | N |
| 57 | N | 1.00 | 1.04 | -3.93 | 0.97 | N | N | N | N |
| 58 | N | 1.00 | 1.04 | -3.30 | 1.02 | N | N | N | N |
| 59 | N | 1.00 | 1.04 | -3.12 | 1.10 | N | N | N | N |
| 60 | N | 1.00 | 1.04 | -3.25 | 1.03 | N | N | N | N |
| 61 | N | 1.00 | 1.04 | -2.12 | 1.09 | N | N | N | N |
| 62 | N | 1.00 | 1.03 | -3.04 | 1.16 | N | N | N | N |
| 63 | N | 1.00 | 0.96 | -3.47 | 1.12 | N | N | N | N |
| 64 | N | 1.00 | 1.00 | -3.44 | 1.17 | N | N | N | N |
| 65 | N | 1.00 | 0.98 | -3.47 | 1.27 | N | N | N | N |
| 66 | N | 1.00 | 0.99 | -3.12 | 1.37 | N | N | N | N |
| 67 | N | 1.00 | 0.99 | -3.53 | 1.10 | N | N | N | N |
| 68 | DM | 0.00 | 2.00 | 2.47 | 4.55 | DM | DM | DM | DM |
| 69 | DM | 0.44 | 1.78 | 0.55 | 4.41 | DM | DM | DM | DM |
| 70 | DM | 0.20 | 1.95 | 1.33 | 3.93 | DM | DM | DM | DM |
| 71 | DM | 0.00 | 2.03 | 4.35 | 4.99 | DM | DM | DM | DM |
| 72 | DM | 0.00 | 1.96 | 5.01 | 4.86 | DM | DM | DM | DM |
| 73 | DM | 0.00 | 1.96 | 1.68 | 4.57 | DM | DM | DM | DM |
| 74 | DM | 0.00 | 2.04 | 3.51 | 5.51 | DM | DM | DM | DM |
| 75 | DM | 0.00 | 1.88 | 0.82 | 3.91 | DM | DM | DM | DM |
| 76 | DM | 0.00 | 1.96 | 4.94 | 4.39 | DM | DM | DM | DM |
| 77 | DM | 0.01 | 1.98 | 1.46 | 3.94 | DM | DM | DM | DM |
| 78 | DM | 0.02 | 2.07 | 3.25 | 3.67 | DM | DM | DM | DM |
| 79 | DM | 0.03 | 2.04 | 2.80 | 4.64 | DM | DM | DM | DM |
| 80 | DM | 0.75 | 1.75 | 4.96 | 4.38 | N | DM | DM | DM |
| 81 | DM | 0.93 | 1.92 | 1.67 | 3.23 | N | DM | DM | DM |
| 82 | DM | 0.01 | 2.10 | 2.74 | 4.90 | DM | DM | DM | DM |
| 83 | DM | 0.18 | 1.96 | 1.58 | 3.41 | DM | DM | DM | DM |

# FIG.25

| RAT ID | SCORE | | | | JUDGMENT | | | |
|--------|-----|-----|-----|-----|-----|-----|-----|-----|
| | LRA | SVM | LDA | MAP | LRA | SVM | LDA | MAP |
| UK1 | 0.82 | 1.79 | 1.23 | 3.06 | N | DM | DM | DM |
| UK2 | 1.00 | 1.29 | -0.14 | 2.43 | N | N | N | DM |
| UK3 | 0.63 | 1.69 | 0.30 | 2.63 | N | DM | DM | DM |
| UK4 | 0.78 | 1.31 | 0.17 | 2.27 | N | N | DM | DM |
| UK5 | 0.82 | 1.44 | 0.11 | 2.53 | N | N | DM | DM |
| UK6 | 0.83 | 1.36 | -1.52 | 2.15 | N | N | N | N |
| UK7 | 1.00 | 0.99 | -1.70 | 2.49 | N | N | N | DM |
| UK8 | 1.00 | 1.14 | -1.17 | 2.70 | N | N | N | DM |

FIG.26

# FIG.27

# FIG.28

# FIG.29

# FIG.30

METABOLITE
CONCENTRATION DATA
TO BE EVALUATED

EVALUATION
FUNCTION
MEMORY DEVICE

EVALUATION OF
BIOLOGICAL STATE

BIOLOGICAL STATE-
EVALUATING APPARATUS

EVALUATION RESULTS

# FIG.31

| PATIENT ID | DISEASE STATE | SCORE | | | | JUDGMENT | | |
|---|---|---|---|---|---|---|---|---|
| | | EVALUATION FUNCTION 1 | EVALUATION FUNCTION 2 | EVALUATION FUNCTION 3 $x_1$ | $x_2$ | EVALUATION FUNCTION 1 | EVALUATION FUNCTION 2 | EVALUATION FUNCTION 3 |
| 1 | N | -10.95 | 1.00 | 4.74 | 28.10 | N | N | N |
| 2 | N | -16.48 | 1.00 | 12.57 | 54.40 | N | N | N |
| 3 | N | -21.38 | 1.00 | 22.94 | 379.15 | N | N | N |
| 4 | N | -13.62 | 1.00 | 19.36 | 57.30 | N | N | N |
| 5 | N | -11.35 | 1.00 | 3.82 | 11.02 | N | N | N |
| 6 | N | -16.04 | 1.00 | 9.55 | 53.27 | N | N | N |
| 7 | N | -15.17 | 1.00 | 7.33 | 24.03 | N | N | N |
| 8 | N | -40.57 | 1.00 | 14.69 | 42.86 | N | N | N |
| 9 | N | -11.53 | 1.00 | 6.09 | 21.21 | N | N | N |
| 10 | N | -0.31 | 1.00 | 9.40 | 14.73 | N | N | N |
| 11 | N | -0.22 | 1.00 | 11.39 | 20.01 | N | N | N |
| 12 | N | -31.88 | 1.00 | 7.76 | 20.95 | N | N | N |
| 13 | N | -13.67 | 1.00 | 5.64 | 7.97 | N | N | N |
| 14 | N | -33.14 | 1.00 | 6.80 | 25.92 | N | N | N |
| 15 | N | -12.61 | 1.00 | 6.88 | 31.28 | N | N | N |
| 16 | N | -3.21 | 1.00 | 4.39 | 21.17 | N | N | N |
| 17 | N | -18.53 | 1.00 | 26.96 | 376.50 | N | N | N |
| 18 | N | -9.57 | 1.00 | 11.26 | 18.26 | N | N | N |
| 19 | N | -17.41 | 1.00 | 1.06 | 4.53 | N | N | N |
| 20 | N | -8.24 | 1.00 | 10.93 | 20.09 | N | N | N |
| 21 | N | -19.59 | 1.00 | 4.53 | 23.41 | N | N | N |
| 22 | N | -20.76 | 1.00 | 5.63 | 41.21 | N | N | N |
| 23 | N | -39.89 | 1.00 | 19.41 | 54.86 | N | N | N |
| 24 | N | -7.88 | 1.00 | 1.99 | 17.39 | N | N | N |
| 25 | N | -7.31 | 1.00 | 14.21 | 17.16 | N | N | N |
| 26 | N | -7.59 | 1.00 | 6.94 | 15.96 | N | N | N |
| 27 | N | -8.21 | 1.00 | 6.76 | 13.64 | N | N | N |
| 28 | N | -0.54 | 1.00 | 10.47 | 19.43 | N | N | N |
| 29 | N | -20.31 | 1.00 | 15.04 | 39.98 | N | N | N |
| 30 | N | -5.26 | 1.00 | 11.48 | 14.32 | N | N | N |
| 31 | UC | 7.30 | 0.00 | 15.64 | 14.73 | UC | UC | UC |
| 32 | UC | 20.41 | 0.00 | 22.38 | 12.10 | UC | UC | UC |
| 33 | UC | 26.00 | 0.00 | 18.27 | 12.03 | UC | UC | UC |
| 34 | UC | 23.07 | 0.00 | 8.79 | 5.31 | UC | UC | UC |
| 35 | UC | 15.11 | 0.00 | 7.55 | 6.36 | UC | UC | UC |
| 36 | UC | 10.28 | 0.00 | 8.80 | 6.21 | UC | UC | UC |
| 37 | UC | 5.65 | 0.00 | 17.21 | 15.53 | UC | UC | UC |
| 38 | UC | 3.68 | 0.00 | 25.10 | 13.56 | UC | UC | UC |
| 39 | UC | 9.21 | 0.00 | 12.78 | 8.18 | UC | UC | UC |
| 40 | UC | 12.63 | 0.00 | 12.83 | 9.92 | UC | UC | UC |
| 41 | UC | 25.30 | 0.00 | 10.93 | 3.02 | UC | UC | UC |
| 42 | UC | 11.90 | 0.00 | 10.68 | 6.05 | UC | UC | UC |
| 43 | UC | 6.97 | 0.00 | 7.16 | 4.40 | UC | UC | UC |
| 44 | UC | 13.86 | 0.00 | 11.95 | 6.88 | UC | UC | UC |
| 45 | UC | 20.00 | 0.00 | 21.40 | 10.33 | UC | UC | UC |
| 46 | UC | 40.29 | 0.00 | 48.85 | 20.11 | UC | UC | UC |
| 47 | UC | 27.77 | 0.00 | 24.00 | 7.73 | UC | UC | UC |
| 48 | UC | 18.30 | 0.00 | 25.07 | 16.40 | UC | UC | UC |
| 49 | UC | 4.88 | 0.00 | 20.33 | 18.17 | UC | UC | UC |
| 50 | UC | 25.47 | 0.00 | 26.19 | 10.27 | UC | UC | UC |
| 51 | UC | 0.49 | 0.00 | 14.10 | 14.10 | UC | UC | UC |
| 52 | UC | 21.62 | 0.00 | 20.41 | 11.10 | UC | UC | UC |
| 53 | UC | 14.39 | 0.00 | 23.08 | 9.75 | UC | UC | UC |
| 54 | UC | 29.47 | 0.00 | 18.73 | 10.45 | UC | UC | UC |
| 55 | UC | 5.98 | 0.00 | 16.33 | 9.65 | UC | UC | UC |
| 56 | UC | 23.08 | 0.00 | 23.13 | 15.84 | UC | UC | UC |
| 57 | UC | 10.91 | 0.00 | 11.21 | 6.38 | UC | UC | UC |
| 58 | UC | 14.87 | 0.00 | 12.97 | 2.86 | UC | UC | UC |
| 59 | UC | 13.94 | 0.00 | 11.63 | 3.26 | UC | UC | UC |
| 60 | UC | 2.74 | 0.00 | 12.81 | 9.33 | UC | UC | UC |

# EP 1 862 797 A1

# FIG.32

| PATIENT ID | DISEASE STATE | SCORE | | | | JUDGMENT | | |
|---|---|---|---|---|---|---|---|---|
| | | EVALUATION FUNCTION 1 | EVALUATION FUNCTION 2 | EVALUATION FUNCTION 3 $x_1$ | $x_2$ | EVALUATION FUNCTION 1 | EVALUATION FUNCTION 2 | EVALUATION FUNCTION 3 |
| 1 | N | 2.21 | 0.01 | 6.24 | 17.50 | N | N | N |
| 2 | N | 8.20 | 0.07 | 11.90 | 23.80 | N | N | N |
| 3 | N | 23.44 | 0.00 | 23.67 | 172.95 | N | N | N |
| 4 | N | 8.77 | 0.31 | 13.56 | 45.01 | N | N | N |
| 5 | N | 18.34 | 0.01 | 5.24 | 12.77 | N | N | N |
| 6 | N | 20.35 | 0.00 | 13.63 | 51.06 | N | N | N |
| 7 | N | 4.79 | 0.20 | 13.46 | 20.52 | N | N | N |
| 8 | N | 27.58 | 0.00 | 10.15 | 40.52 | N | N | N |
| 9 | N | 6.54 | 0.08 | 9.75 | 14.40 | N | N | N |
| 10 | N | 11.46 | 0.02 | 2.49 | 8.81 | N | N | N |
| 11 | N | 4.39 | 0.42 | 10.00 | 20.11 | N | N | N |
| 12 | N | 22.30 | 0.00 | 4.83 | 12.02 | N | N | N |
| 13 | N | 7.19 | 0.43 | 4.63 | 7.91 | N | N | N |
| 14 | N | 16.88 | 0.00 | 16.35 | 47.48 | N | N | N |
| 15 | N | 4.45 | 0.25 | 6.27 | 13.66 | N | N | N |
| 16 | N | 10.12 | 0.02 | 1.00 | 15.69 | N | N | N |
| 17 | N | 23.95 | 0.00 | 10.66 | 34.14 | N | N | N |
| 18 | N | 25.11 | 0.00 | 12.44 | 39.37 | N | N | N |
| 19 | N | 9.21 | 0.03 | 3.34 | 9.32 | N | N | N |
| 20 | N | 12.85 | 0.06 | 4.42 | 15.47 | N | N | N |
| 21 | N | 5.73 | 0.19 | 8.98 | 11.40 | N | N | N |
| 22 | N | -21.47 | 0.58 | 17.77 | 21.32 | CD | CD | N |
| 23 | N | 17.20 | 0.39 | 14.89 | 19.32 | N | N | N |
| 24 | N | 1.04 | 0.86 | 10.66 | 13.16 | N | CD | N |
| 25 | N | 9.68 | 0.34 | 9.38 | 12.84 | N | N | N |
| 26 | N | 6.63 | 0.69 | 4.96 | 6.33 | N | CD | N |
| 27 | N | 10.57 | 0.16 | 4.96 | 14.21 | N | N | N |
| 28 | N | -4.96 | 0.37 | 10.10 | 15.50 | CD | N | N |
| 29 | N | 2.42 | 0.34 | 19.90 | 23.22 | N | N | N |
| 30 | N | 14.45 | 0.01 | 14.36 | 20.59 | N | N | N |
| 31 | CD | -9.77 | 1.00 | 23.21 | 11.01 | CD | CD | CD |
| 32 | CD | 7.83 | 0.34 | 11.11 | 8.61 | N | N | CD |
| 33 | CD | -11.15 | 0.98 | 26.30 | 8.66 | CD | CD | CD |
| 34 | CD | -17.23 | 0.94 | 20.87 | 9.70 | CD | CD | CD |
| 35 | CD | -13.93 | 1.00 | 33.71 | 12.43 | CD | CD | CD |
| 36 | CD | -27.10 | 1.00 | 37.38 | 9.97 | CD | CD | CD |
| 37 | CD | 5.41 | 0.07 | 9.99 | 2.92 | N | N | CD |
| 38 | CD | -20.28 | 1.00 | 67.13 | 10.99 | CD | CD | CD |
| 39 | CD | -3.60 | 0.73 | 62.79 | 12.30 | CD | CD | CD |
| 40 | CD | 9.80 | 0.19 | 13.83 | 9.88 | N | N | CD |
| 41 | CD | -20.92 | 1.00 | 19.94 | 4.07 | CD | CD | CD |
| 42 | CD | -30.41 | 1.00 | 21.08 | 6.99 | CD | CD | CD |
| 43 | CD | -13.24 | 0.85 | 41.08 | 10.61 | CD | CD | CD |
| 44 | CD | -12.68 | 0.74 | 39.60 | 11.86 | CD | CD | CD |
| 45 | CD | -15.83 | 1.00 | 23.46 | 10.02 | CD | CD | CD |
| 46 | CD | -12.95 | 0.74 | 152.17 | 24.40 | CD | CD | CD |
| 47 | CD | -0.61 | 0.40 | 25.31 | 13.54 | CD | N | CD |
| 48 | CD | -10.92 | 0.95 | 47.71 | 10.76 | CD | CD | CD |
| 49 | CD | -0.10 | 0.84 | 42.92 | 12.60 | CD | CD | CD |
| 50 | CD | -5.85 | 0.71 | 6.50 | 5.14 | CD | CD | CD |
| 51 | CD | -4.72 | 0.54 | 12.81 | 9.12 | CD | CD | CD |
| 52 | CD | 3.49 | 0.63 | 11.06 | 5.36 | N | CD | CD |
| 53 | CD | -29.10 | 1.00 | 41.82 | 10.74 | CD | CD | CD |
| 54 | CD | -12.08 | 0.83 | 27.80 | 5.10 | CD | CD | CD |
| 55 | CD | -10.36 | 1.00 | 35.02 | 15.02 | CD | CD | CD |
| 56 | CD | -5.11 | 0.77 | 91.75 | 12.35 | CD | CD | CD |
| 57 | CD | -12.59 | 0.99 | 14.29 | 8.12 | CD | CD | CD |
| 58 | CD | -10.88 | 0.97 | 20.51 | 7.00 | CD | CD | CD |
| 59 | CD | -12.68 | 0.97 | 32.75 | 12.88 | CD | CD | CD |
| 60 | CD | -11.88 | 0.97 | 19.51 | 7.84 | CD | CD | CD |

# FIG.33

| SAMPLE ID | DISEASE STATE | Lys | Arg | Asn | SCORE |
|---|---|---|---|---|---|
| 1 | N | 30.19 | 15.80 | 3.71 | 0.98 |
| 2 | N | 20.31 | 9.96 | 2.40 | 0.98 |
| 3 | N | 21.00 | 11.10 | 2.99 | 1.09 |
| 4 | N | 21.00 | 8.63 | 2.78 | 1.15 |
| 5 | N | 19.79 | 9.91 | 2.57 | 1.05 |
| 6 | N | 25.48 | 10.30 | 3.08 | 0.96 |
| 7 | N | 21.69 | 10.64 | 2.11 | 0.96 |
| 8 | N | 29.67 | 14.11 | 3.88 | 1.05 |
| 9 | N | 26.29 | 13.61 | 3.53 | 1.05 |
| 10 | N | 24.70 | 11.85 | 3.51 | 1.20 |
| 11 | N | 23.67 | 11.62 | 3.12 | 0.99 |
| 12 | N | 31.69 | 16.55 | 3.49 | 1.01 |
| 13 | N | 24.28 | 13.70 | 3.14 | 1.02 |
| 14 | N | 36.01 | 17.62 | 4.04 | 1.05 |
| 15 | N | 26.91 | 14.24 | 3.28 | 0.98 |
| 16 | N | 25.48 | 14.09 | 3.18 | 1.00 |
| 17 | N | 31.41 | 17.66 | 3.87 | 1.05 |
| 18 | N | 23.74 | 12.60 | 3.04 | 0.97 |
| 19 | N | 22.24 | 11.85 | 2.87 | 0.95 |
| 20 | N | 28.48 | 14.36 | 3.42 | 0.96 |
| 21 | A | 16.71 | 9.02 | 3.96 | 2.04 |
| 22 | A | 24.35 | 13.20 | 5.39 | 1.95 |
| 23 | A | 17.33 | 10.02 | 3.96 | 2.03 |
| 24 | A | 21.70 | 10.68 | 4.99 | 2.00 |
| 25 | A | 14.73 | 8.36 | 3.43 | 2.05 |
| 26 | A | 19.20 | 10.47 | 3.97 | 2.00 |
| 27 | A | 21.31 | 10.61 | 3.87 | 1.84 |
| 28 | A | 18.87 | 9.17 | 4.23 | 2.04 |
| 29 | A | 20.27 | 11.27 | 3.94 | 1.90 |
| 30 | A | 17.90 | 9.69 | 4.14 | 2.05 |
| 31 | A | 21.53 | 11.66 | 4.19 | 1.95 |
| 32 | A | 25.84 | 12.35 | 5.00 | 1.95 |
| 33 | A | 15.72 | 8.37 | 3.30 | 1.98 |
| 34 | A | 16.23 | 7.68 | 3.15 | 1.94 |
| 35 | A | 19.22 | 11.21 | 4.33 | 2.05 |
| 36 | A | 18.03 | 9.21 | 3.65 | 1.95 |
| 37 | A | 13.71 | 7.70 | 2.67 | 1.94 |
| 38 | A | 16.15 | 8.74 | 3.66 | 2.03 |
| 39 | A | 18.73 | 8.09 | 4.05 | 1.99 |
| 40 | A | 20.72 | 10.67 | 4.04 | 1.97 |

# FIG.34

| ID | DISEASE STATE | Lys | Arg | Asn | SCORE | JUDGMENT |
|---|---|---|---|---|---|---|
| U-1 | N | 36.30 | 19.33 | 3.92 | 1.15 | N |
| U-2 | N | 26.81 | 14.91 | 2.59 | 1.15 | N |
| U-3 | N | 21.58 | 12.65 | 2.41 | 1.00 | N |
| U-4 | N | 31.17 | 16.53 | 3.22 | 1.08 | N |
| U-5 | N | 23.28 | 13.23 | 2.33 | 1.05 | N |
| U-6 | N | 26.41 | 15.79 | 3.07 | 1.10 | N |
| U-7 | N | 34.98 | 18.28 | 3.64 | 1.09 | N |
| U-8 | N | 21.48 | 12.09 | 2.57 | 0.94 | N |
| U-9 | N | 24.77 | 14.22 | 2.99 | 1.02 | N |
| U-10 | N | 35.58 | 18.00 | 4.27 | 1.09 | N |
| U-11 | A | 20.34 | 10.36 | 4.32 | 2.06 | A |
| U-12 | A | 13.07 | 7.03 | 2.42 | 1.91 | A |
| U-13 | A | 20.68 | 10.17 | 4.05 | 1.98 | A |
| U-14 | A | 22.51 | 9.91 | 4.98 | 1.96 | A |
| U-15 | A | 22.19 | 10.38 | 4.21 | 1.97 | A |
| U-16 | A | 19.66 | 11.29 | 4.26 | 2.04 | A |
| U-17 | A | 21.75 | 12.61 | 3.80 | 1.61 | A |
| U-18 | A | 22.40 | 10.81 | 4.63 | 2.04 | A |
| U-19 | A | 15.79 | 7.72 | 2.98 | 1.91 | A |
| U-20 | A | 25.68 | 12.80 | 4.99 | 1.94 | A |

# FIG.35

| SAMPLE ID | DISEASE STATE | SCORE | | | | JUDGMENT | | |
|---|---|---|---|---|---|---|---|---|
| | | EVALUATION FUNCTION 1 | EVALUATION FUNCTION 2 | EVALUATION FUNCTION 3 $x_1$ | $x_2$ | EVALUATION FUNCTION 1 | EVALUATION FUNCTION 2 | EVALUATION FUNCTION 3 |
| 1 | N | -1.85 | 0.00 | 3.60 | 5.30 | N | N | N |
| 2 | N | -5.21 | 0.00 | 2.57 | 58.46 | N | N | N |
| 3 | N | -4.40 | 0.00 | 0.15 | 48.87 | N | N | N |
| 4 | N | -3.96 | 0.00 | 0.19 | 29.83 | N | N | N |
| 5 | N | -3.63 | 0.00 | 0.24 | 29.80 | N | N | N |
| 6 | N | -2.55 | 0.00 | 3.71 | 17.02 | N | N | N |
| 7 | N | -4.16 | 0.00 | 0.00 | 37.50 | N | N | N |
| 8 | N | -4.19 | 0.00 | 0.24 | 38.44 | N | N | N |
| 9 | N | -3.94 | 0.00 | 0.02 | 38.94 | N | N | N |
| 10 | N | -2.31 | 0.00 | 2.07 | 11.89 | N | N | N |
| 11 | N | -1.64 | 0.00 | 3.81 | 19.57 | N | N | N |
| 12 | N | -4.44 | 0.00 | 1.42 | 50.81 | N | N | N |
| 13 | N | -4.93 | 0.00 | 1.76 | 66.07 | N | N | N |
| 14 | N | -2.78 | 0.00 | 1.31 | 24.30 | N | N | N |
| 15 | N | -5.33 | 0.00 | 3.54 | 75.62 | N | N | N |
| 16 | N | -4.79 | 0.00 | 1.16 | 54.00 | N | N | N |
| 17 | N | -3.48 | 0.00 | 0.14 | 49.12 | N | N | N |
| 18 | N | -2.71 | 0.00 | 0.73 | 24.91 | N | N | N |
| 19 | N | -4.20 | 0.00 | 1.33 | 49.57 | N | N | N |
| 20 | N | -2.48 | 0.00 | 2.98 | 18.38 | N | N | N |
| 21 | N | -5.35 | 0.00 | 0.80 | 67.88 | N | N | N |
| 22 | N | -1.65 | 0.00 | 1.00 | 18.95 | N | N | N |
| 23 | N | -2.86 | 0.00 | 1.90 | 30.10 | N | N | N |
| 24 | N | -3.72 | 0.00 | 5.08 | 76.34 | N | N | N |
| 25 | N | -3.78 | 0.00 | 3.08 | 61.19 | N | N | N |
| 26 | N | -5.55 | 0.00 | 7.25 | 143.09 | N | N | N |
| 27 | N | -4.89 | 0.00 | 3.91 | 106.35 | N | N | N |
| 28 | R | 1.18 | 1.00 | 13.10 | 0.50 | R | R | R |
| 29 | R | 0.13 | 1.00 | 7.49 | 3.87 | R | R | R |
| 30 | R | 1.90 | 1.00 | 13.97 | 1.19 | R | R | R |
| 31 | R | 1.15 | 1.00 | 16.08 | 1.82 | R | R | R |
| 32 | R | 1.57 | 1.00 | 15.04 | 1.13 | R | R | R |
| 33 | R | 1.78 | 1.00 | 12.85 | 1.85 | R | R | R |
| 34 | R | 1.34 | 1.00 | 7.98 | 0.83 | R | R | R |
| 35 | R | 1.72 | 1.00 | 7.92 | 7.60 | R | R | R |
| 36 | R | 1.73 | 1.00 | 12.46 | 0.76 | R | R | R |
| 37 | R | 2.55 | 1.00 | 13.35 | 0.53 | R | R | R |
| 38 | R | 3.05 | 1.00 | 14.88 | 2.16 | R | R | R |
| 39 | R | 2.78 | 1.00 | 13.40 | 1.34 | R | R | R |
| 40 | R | 2.78 | 1.00 | 12.65 | 2.54 | R | R | R |
| 41 | R | 1.39 | 1.00 | 10.08 | 0.43 | R | R | R |
| 42 | R | 2.43 | 1.00 | 6.97 | 6.24 | R | R | R |
| 43 | R | 1.89 | 1.00 | 14.53 | 1.08 | R | R | R |
| 44 | R | 1.77 | 1.00 | 9.99 | 0.13 | R | R | R |
| 45 | R | 2.12 | 1.00 | 10.79 | 0.03 | R | R | R |
| 46 | R | 2.96 | 1.00 | 13.25 | 0.64 | R | R | R |
| 47 | R | 1.60 | 1.00 | 5.87 | 1.89 | R | R | R |
| 48 | R | 1.54 | 1.00 | 9.80 | 0.04 | R | R | R |
| 49 | R | 1.77 | 1.00 | 18.36 | 4.27 | R | R | R |
| 50 | R | 3.09 | 1.00 | 14.30 | 0.83 | R | R | R |
| 51 | R | 3.14 | 1.00 | 7.41 | 1.52 | R | R | R |
| 52 | R | 2.69 | 1.00 | 5.18 | 2.86 | R | R | R |
| 53 | R | 3.35 | 1.00 | 5.74 | 4.64 | R | R | R |
| 54 | R | 2.94 | 1.00 | 4.63 | 3.29 | R | R | R |

## INTERNATIONAL SEARCH REPORT

| | International application No. |
|---|---|
| | PCT/JP2006/304398 |

A. CLASSIFICATION OF SUBJECT MATTER
*G01N33/48*(2006.01), *A61B10/00*(2006.01), *G06F19/00*(2006.01), *G06Q50/00* (2006.01)

According to International Patent Classification (IPC) or to both national classification and IPC

B. FIELDS SEARCHED

Minimum documentation searched (classification system followed by classification symbols)
*G01N33/48*(2006.01), *A61B10/00*(2006.01), *G06F19/00*(2006.01), *G06Q50/00* (2006.01)

Documentation searched other than minimum documentation to the extent that such documents are included in the fields searched
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2004-135546 A (Sumitomo Pharmaceuticals Co., Ltd.), 13 May, 2004 (13.05.04), Claims (Family: none) | 1-41 |
| Y | JP 2004-321179 A (Nitto Boseki Co., Ltd.), 18 November, 2004 (18.11.04), Claims (Family: none) | 1-41 |
| Y | JP 11-190734 A (Wakamoto Pharmaceutical Co., Ltd.), 13 July, 1999 (13.07.99), Claims & US 6777197 B & EP 1052511 A & WO 99/34218 A | 1-41 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 27 March, 2006 (27.03.06) | 04 April, 2006 (04.04.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**EP 1 862 797 A1**

**INTERNATIONAL SEARCH REPORT**

International application No.

PCT/JP2006/304398

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-159095 A (Takeda Chemical Industries, Ltd.), 03 June, 2003 (03.06.03), Claims & WO 2003/7187 A | 1-41 |

Form PCT/ISA/210 (continuation of second sheet) (April 2005)

72

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5687716 A

- WO 2004052191 A